# EUROPEAN PATENT APPLICATION

(11) **EP 3 964 512 A1**
(43) Date of publication of application: **09.03.2022**
(21) Application number: 20830639.9
(22) Date of filing: 23.06.2020
(51) Int. Cl.: C07D 487/04, A61K 31/517, A61K 31/519, A61P 35/00, A61P 37/00, A61P 11/00

(54) **CASEIN KINASE 1epsilon INHIBITOR, PHARMACEUTICAL COMPOSITION AND APPLICATION THEREOF**

(30) Priority: 27.06.2019 CN 201910565300
(71) Applicant: Hangzhou Healzen Therapeutics Co., Ltd., Hangzhou, Zhejiang 310018 (CN)
(72) Inventor: ZHOU, Xinglu, Hangzhou, Zhejiang 310018 (CN); LIU, Xingguo, Hangzhou, Zhejiang 310018 (CN); HU, Miao, Hangzhou, Zhejiang 310018 (CN)
(74) Representative: Gong, Jinping
(86) International application number: PCT/CN2020/097577
(87) International publication number: WO 2020/259463

(57) **Abstract**

Disclosed in the present invention is a novel substituted pyrazolopyrimidine compound that inhibits the activity of casein kinase 1ε (CK1ε) and a stereoisomer or stereoisomer mixture thereof, or a pharmaceutically acceptable salt or solvate thereof, and an application thereof in preparation of drugs for treating diseases, disorders or conditions that benefit from the inhibition of the activity of the casein kinase 1ε (CK1ε). The compound of the present invention has an inhibitory activity on CK1ε kinase, OCI-LY10 cells and Karpas299 cells, shows good anti-tumor activity in an OCI-LY10 subcutaneous xenogeneic model and shows excellent synergistic anti-tumor activity in combination with BTK inhibitors. The compound of the present invention has good pharmacokinetics properties and may be used alone or in combination with other drugs to treat diseases, disorders or conditions that benefit from the inhibition of the activity of the casein kinase 1ε, the diseases, disorders or conditions comprising tumors, autoimmune diseases, etc.

## Description

### FIELD OF TECHNOLOGY

The present invention belongs to the field of medicines, more specifically relating to a substituted pyrazolopyrimidine casein kinase 1ε inhibitor, a pharmaceutical composition and an application thereof.

### BACKGROUND

Protein kinases play an important role in signal transduction, mediating intracellular signal transduction by phosphorylation of substrate proteins, thus being an important type of drug targets. Casein kinase 1 (CK1) belongs to the serine-threonine kinase family with seven isoforms in mammals: α, β, γ1, γ2, γ3, δ and ε. These isoforms are involved in Wnt signal transduction, circadian rhythms, cell signal transduction, membrane transport, DNA replication, DNA damage and RNA metabolism and can regulate organismal functions.

Phosphorylated substrates of CK1 are involved in a variety of intracellular regulations. For example, the tumor suppressor p53 and oncogene MDM2 are both important proteins that control abnormal cell growth, and substrates for CK1 as well. The isoforms of CK1 kinase (primarily CK1ε and CK1δ) have a significant correlation with p53 phosphorylation, p53 stabilization and activation, p53-MDM2 interaction, etc. They are also associated with the formation of centrosome and spindle apparatus during cell division followed by regulations of protein synthesis, or with apoptosis of TRAIL (TNF-related apoptosis-inducing ligand) and FAS.

Casein kinase 1ε (CK1ε), an important isoform of the CK1 family, is a key regulator of various cell growth and survival processes, which not only plays an important role in the regulation of circadian rhythms, but is equally important in the development and progression of cancer. For example, pharmacological inhibition of CK1ε or shRNA-mediated excision of CK1ε can hinder the growth and survival of a variety of cancers, including pancreatic cancer, sarcoma, breast cancer, rectal cancer, ovarian caner, leukemia, etc. Studies have demonstrated that the mechanism underlying CK1ε-regulated tumor growth and survival is yet unclear due to the lack of substrate specificity of the CK1 gene, which may be associated with Akt, MYC, and β -catenin (Varghese et al., Scientific Reports, 2018, 8: 13621).

The Wnt signaling pathway regulates the cell proliferation process, and incidence of mutations in this pathway can lead to various cancers, including skin cancer, liver cancer, brain tumor, and colon cancer. CK1 can promote the degradation of β-catenin by phosphorylation thereof. CK1ε also regulates the Wnt signaling pathway by phosphorylation of Tcf3 as the phosphorylated Tcf3 binds to β-catenin to promote the degradation thereof (Knippschild et al., Cellular Signalling, 2005, 17: 675-689).

Structures of several types of CK1 kinase inhibitors have been reported, such as 1) **PF-4800567,** a substituted pyrazolopyrimidine, which is a selective CK1ε inhibitor (IC₅₀ = 32nM; Walton et al., J Pharmacol Exp Ther. 2009, 330(2):430-9); 2) **PF-670462,** a substituted imidazol-5-yl pyrimidine compound, which is a selective CKIε/CKIδ inhibitor (IC₅₀ of 7.7nM and 14nM, respectively; Badura et al., J Pharmacol Exp Ther. 2007, 322(2):730-8); 3) **CK1-IN-1,** an imidazol-5-yl pyridine compound, which is a multitarget inhibitor with IC₅₀ of 15nM, 16nM and 73nM for CKIδ, CKIε and p38σ MAPK, respectively (WO2015119579A1).

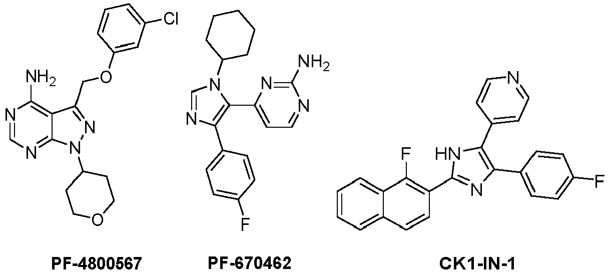

In addition, CK1ε, as an important component for the regulation of Wnt signaling pathway, can drive the development and progression of chronic lymphocytic leukemia (CLL). CK1ε is significantly upregulated in CLL patients. The development of CLL can be blocked through inhibition of CK1ε without affecting the BCR-related pathways; in addition, CK1ε can enhance the *in-vitro* and *in-vivo* anti-tumor activity of ibrutinib, a BTK inhibitor of the BCR pathway. Therefore, CK1ε is also an important anti-tumor target (Janovska et al., Blood, 2019, 11: 1206~1218).

Therefore, the development of drugs with the capability to inhibit CK1ε will play an important role in treating a variety of diseases such as cancer and autoimmune diseases.

### SUMMARY

It is an object of the present invention to provide a novel substituted pyrazolopyrimidine compound that inhibits the activity of casein kinase 1ε (CK1ε), and a stereoisomer or stereoisomer mixture thereof, or a pharmaceutically acceptable salt or solvate thereof.

According to the present invention, there is further provided a pharmaceutical composition comprising the above compound and the stereoisomer or stereoisomer mixture thereof, or the pharmaceutically acceptable salt or solvate thereof.

According to the present invention, there is also provided an application of the above compound and the stereoisomer or stereoisomer mixture thereof or the pharmaceutically acceptable salt or solvate thereof in preparation of medicines for treating diseases, disorders or conditions that benefit from the inhibition of the activity of the casein kinase 1ε (CK1ε).

The following technical scheme is adopted in the present invention:

The CK1ε inhibitor provided in the present invention has a structure of general formula I: 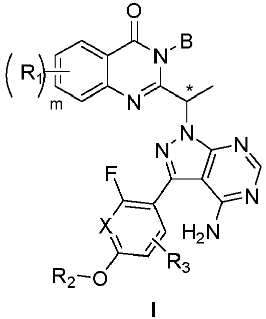 or a stereoisomer thereof, or a stereoisomer mixture thereof, or a pharmaceutically acceptable salt or solvate thereof;
wherein:
R₁ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, substituted or unsubstituted C₂-C₁₀ alkenyl, substituted or unsubstituted C₂-C₁₀ alkinyl, halogen, cyano, amino, nitro or hydroxy;
m is an integer between 1 and 4; and when m is greater than 1, the plurality of R₁ may be the same or different, independently of each other;
R₂ is H, substituted or unsubstituted C₁-C₆ alkyl, substituted or unsubstituted C₃-C₁₀ cycloalkyl, or substituted or unsubstituted C₅-C₁₂ aryl;
R₃ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, halogen, cyano, amino, nitro or hydroxy;
X is CR₄ or N;
R₄ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, halogen, cyano, amino or hydroxy;
B is selected from substituted or unsubstituted C₁-C₆ alkyl, substituted or unsubstituted C₃-C₁₀ cycloalkyl, substituted or unsubstituted 3- to 10-membered heterocycloalkyl, substituted or unsubstituted C₅-C₁₂ aryl, and substituted or unsubstituted 5- to 12-membered heteroaryl.

As a preferred scheme, the R₃ and R₄ are not H simultaneously.

Further, a preferable compound of the present invention has a structure of general formula II-A, II-B or II-C: 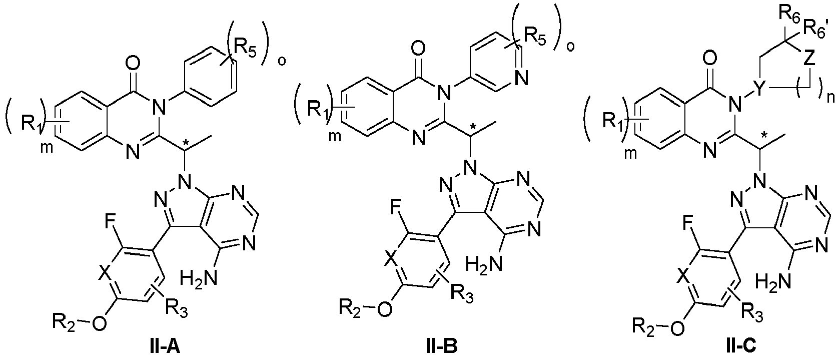 or a stereoisomer thereof, or a stereoisomer mixture thereof, or a pharmaceutically acceptable salt or solvate thereof;
wherein: the definitions of R₁, m, R₂, R₃, X, R₄ and B are the same as defined in general formula I;
R₅ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, substituted or unsubstituted C₁-C₆ hydroxyalkyl, substituted or unsubstituted C₁-C₆ halohydroxyalkyl, halogen, cyano, amino, nitro or hydroxy;
o is an integer between 1 and 5;
R₆ and R₆' are independently selected from H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, halogen, cyano, amino or hydroxy, or, R₅ and R₅' are joined to form a 3- to 6-membered cycloalkyl or heterocycloalkyl;
n is absent, or is 0, 1 or 2; wherein:
when n is absent, i.e., when Y and Z are not joined to form a ring, Y is CR₇R₇' or NR₈; and Z is CHR₇R₇', OH or NHR₈; R₇ in Y and Z may be the same or different, independently of each other; similarly, R₇' in Y and Z may be the same or different, independently of each other; and
when n is 0, 1 or 2, Y is CH or N; and Z is CR₇R₇', O or NR₈;
R₇ and R₇' are independently selected from H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, substituted or unsubstituted C₃-C₁₀cycloalkyl, substituted or unsubstituted 3- to 10-membered heterocycloalkyl, halogen, cyano, amino or hydroxy; or, R₇ and R₇' are joined to form a 3- to 6-membered cycloalkyl or heterocycloalkyl;
R₈ is H, alkyl (preferably, substituted or unsubstituted C₁-C₆ alkyl or substituted or unsubstituted C₁-C₆ haloalkyl), substituted or unsubstituted C₃-C₁₀cycloalkyl, substituted or unsubstituted 3- to 10-membered heterocycloalkyl, -C(O)-R₉ or -S(O)₂-R₁₀;
R₉ is substituted or unsubstituted C₁-C₆ alkyl, substituted or unsubstituted C₁-C₆ cycloalkyl; and
R₁₀ is substituted or unsubstituted C₁-C₆ alkyl, or substituted or unsubstituted C₁-C₆ cycloalkyl.

Even further, a preferable compound of the present invention has a structure of general formula III-A, III-B or III-C: 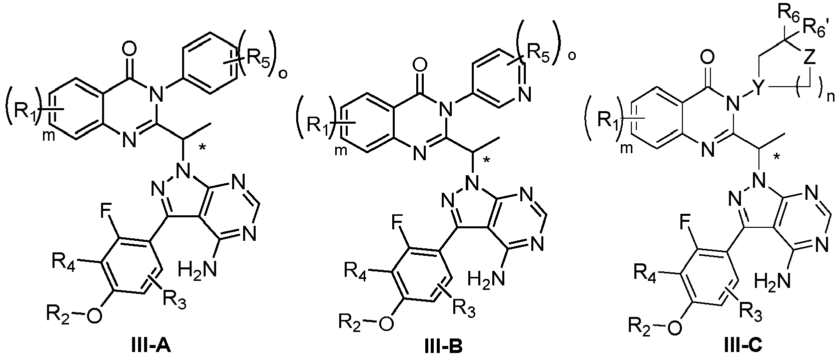 or a stereoisomer thereof, or a stereoisomer mixture thereof, or a pharmaceutically acceptable salt or solvate thereof;
wherein, the definitions of R₁, R₂, R₃, R₄, R₅, R₆, R₆', Y, Z and n are the same as defined in general formulas II-A, II-B and II-C.

Even further, a preferable compound of the present invention has a structure of general formula IV-A, IV-B or IV-C: 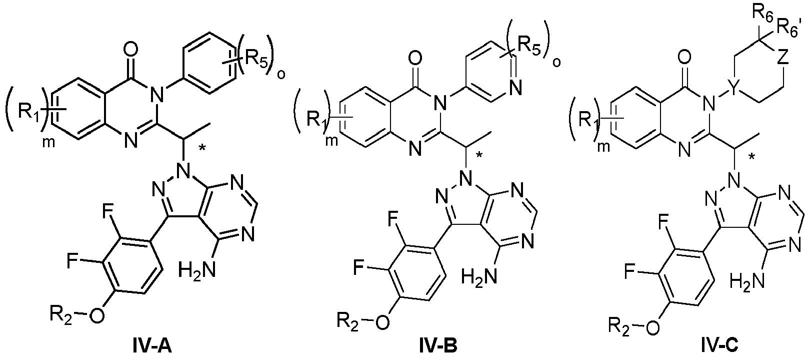 or a stereoisomer thereof, or a stereoisomer mixture thereof, or a pharmaceutically acceptable salt or solvate thereof;
wherein:
R₁is selected from H, substituted or unsubstituted C₁-C₆ alkyl, and halogen;
m is selected from integers of 1 to 4;
R₂ is selected from methyl, ethyl, isopropyl and cyclopropyl;
R₅ is selected from H, substituted or unsubstituted C₁-C₆ alkyl, and halogen;
o is selected from integers of 1 to 4;
R₆ and R₆' are selected from H, substituted or unsubstituted C₁-C₆ alkyl, and halogen;
Y is selected from N or CH;
Z is selected from CH₂, O and NR₈;
R₈ is selected from H, substituted or unsubstituted C₁-C₆ alkyl, and -C(O)-R₉; and
R₉ is selected from substituted or unsubstituted C₁-C₆ alkyl.

Even further, a preferable compound of the present invention has a structure of general formula V-A, V-B or V-C: 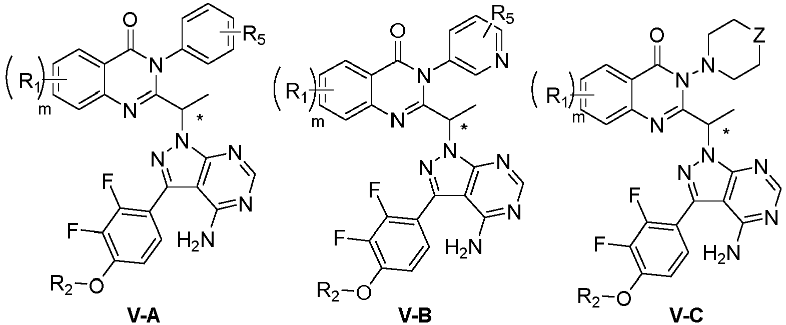 or a stereoisomer thereof, or a stereoisomer mixture thereof, or a pharmaceutically acceptable salt or solvate thereof;
wherein: the definitions of R₁, m, R₅ and Z are the same as defined in general formulas IV-A, IV-B and IV-C; and R₂ is methyl, ethyl or isopropyl.

Preferably, a preferable compound of the present invention has a structure of general formula V-A, V-B or V-C, wherein:
R₁is selected from H and halogen (preferably Cl and F);
R₂ is selected from methyl, ethyl, isopropyl, cyclopropyl and phenyl;
m is 1, 2 or 3;
R₃ is selected from F, Cl, CN and H; and
X is C, N or CH.

As a preferable scheme, B is phenyl, pyridinyl (further preferably at 3-position, i.e., pyridin-3-yl), halogen substituted pyridinyl (further preferably Cl or F substituted pyridinyl; preferably at 3-position, i.e., Cl or F substituted pyridin-3-yl), cyclohexyl, piperidinyl (further preferably at 4-position (i.e., piperidin-4-yl) and at 1-position (i.e., piperidin-1-yl)), N-acetylpiperidinyl (further preferably at 4-position, i.e., N-acetylpiperidin-4-yl), N-(2-trifluoroethyl) substituted piperidinyl (further preferably at 4-position, i.e., N-(2-trifluoroethyl) substituted piperidin-4-yl), morpholino (further preferably at N-position or C-position), piperazinyl (further preferably at N-position), dimethyl (further preferably dimethyl substituent at C-position) substituted piperazinyl (further preferably at N-position), trifluoromethyl substituted (further preferably trifluoromethyl substituent at C-position) piperazinyl (further preferably at N-position), trifluoroethyl substituted (further preferably trifluoroethyl substituent at N-position) piperazinyl (further preferably at N-position), methyl substituted (further preferably methyl substituent at N-position) piperazinyl (further preferably at N-position), acetyl substituted (further preferably acetyl substituent at N-position) piperazinyl (further preferably at N-position), 3-methoxypropyl and 2-(tetrahydrofuran-2-yl)ethyl.

As preferred, the CK1ε inhibitor is preferably the following specific compounds:

| No. | Structure | No. | Structure | No. | Structure |
|---|---|---|---|---|---|
| II-A-1 | 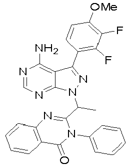 | II-A-1a | 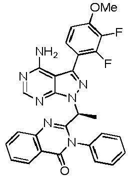 | II-A-1b | 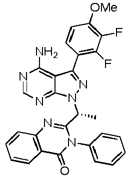 |
| II-A-2 | 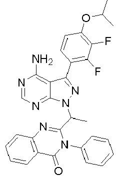 | II-A-2a | 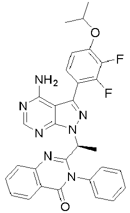 | II-A-2b | 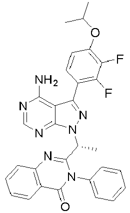 |
| II-A-3 | 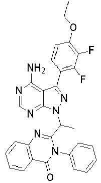 | II-A-4 | 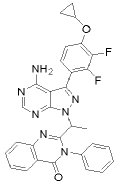 | II-A-5 | 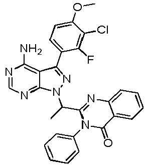 |
| II-A-6 | 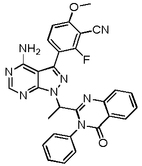 | II-A-7 | 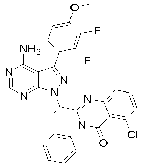 | II-A-7a | 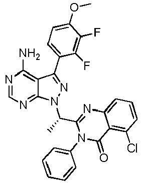 |
| II-A-7b | 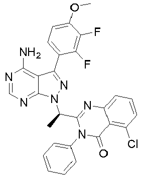 | II-A-8 | 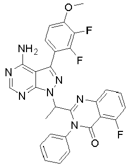 | II-A-8a | 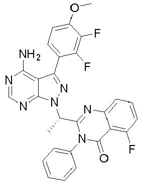 |
| II-A-8b | 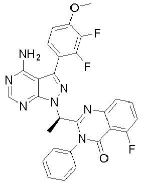 | II-A-9 | 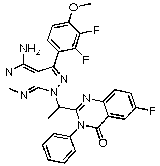 | II-A-10 | 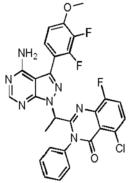 |
| II-A-11 | 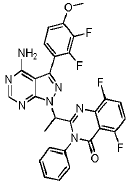 | II-A-12 | 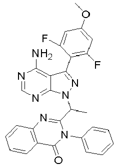 | II-A-13 | 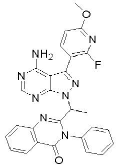 |
| II-A-14 | 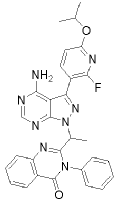 | II-A-15 | 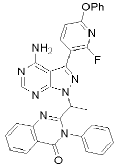 | II-A-16 | 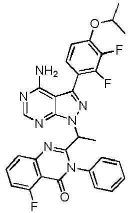 |
| II-B-1 | 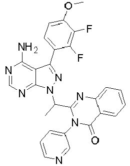 | II-B-2 | 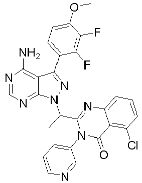 | II-B-2a | 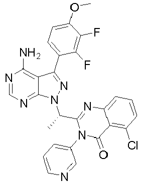 |
| II-B-2b | 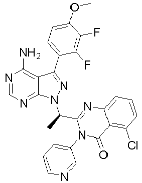 | II-B-3 | 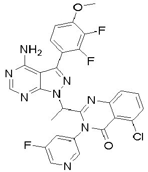 | II-C-1 | 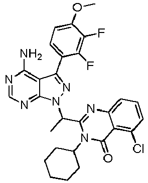 |
| II-C-2 | 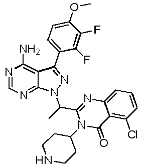 | II-C-3 | 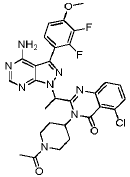 | II-C-4 | 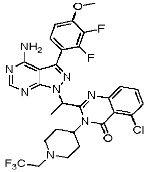 |
| II-C-5 | 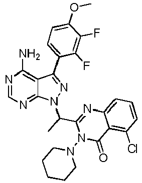 | II-C-6 | 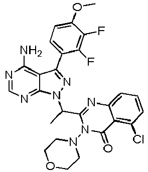 | II-C-7 | 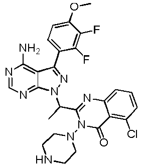 |
| II-C-8 | 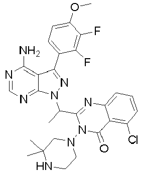 | II-C-9 | 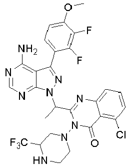 | II-C-10 | 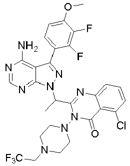 |
| II-C-11 | 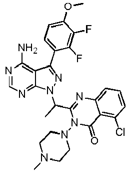 | II-C-12 | 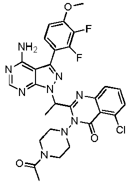 | II-C-13 | 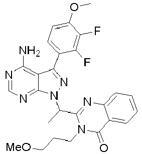 |
| II-C-14 | 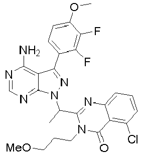 | II-C-15 | 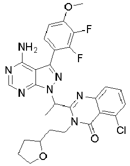 | | |

and stereoisomers of the above formulas, or stereoisomer mixtures thereof, or pharmaceutically acceptable salts or solvates thereof.

As preferred, the CK1ε inhibitor is preferably one of the following compounds:

| | | | | | |
|---|---|---|---|---|---|
| II-D-2 | 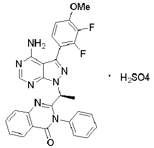 | II-D-3 | 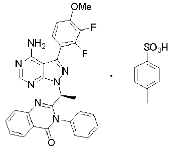 | II-D-4 | 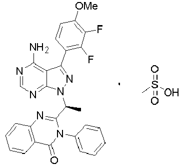 |
| II-D-5 | 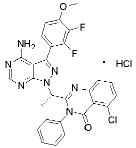 | II-D-6 | 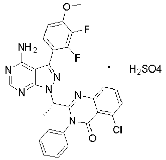 | II-D-7 | 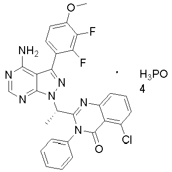 |
| II-D-8 | 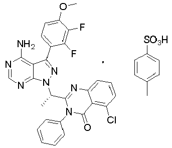 | II-D-9 | 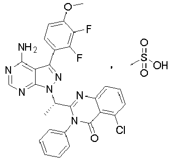 | II-D-10 | 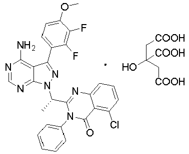 |
| II-D-11 | 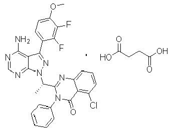 | II-D-12 | 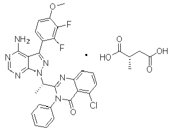 | II-D-13 | 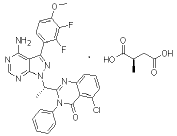 |
| II-D-14 | 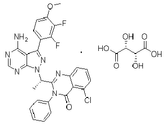 | II-D-15 | 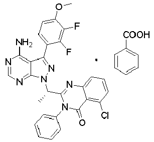 | II-D-16 | 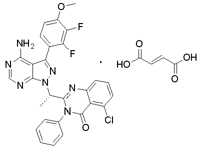 |
| II-D-17 | 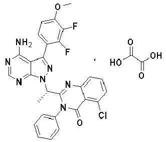 | II-D-1 | 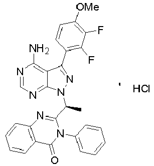 | | |

As preferred, the compound is the following compounds:
2-(1-(4-amino-3-(2,3-difluoro-4-methoxyphenyl)-1H-pyrazolo[3,4-d]pyrimidin-1-yl)eth yl)-3-phenylquinazolin-4(3H)-one (**II-A-1**)
(S)-2-(1(4-amino-3-(2,3-difluoro-4-methoxyphenyl)-1H-pyrazolo[3,4-d]pyrimidin-1-yl )ethyl)-3-phenylquinazolin-4(3H)-one (**II-A-1a**)
(R)-2-(1-(4-amino-3-(2,3-difluoro-4-methoxyphenyl)-1H-pyrazolo[3,4-d]pyrimidin-1-yl )ethyl)-3-phenylquinazolin-4(3H)-one (**II-A-1b**)
2-(1-(4-amino-3-(2,3-difluoro-4-isopropoxyphenyl)-1H-pyrazolo[3,4-d]pyrimidin-1-yl) ethyl)-3-phenylquinazolin-4(3H)-one (**II-A-2**)
(S)-2-(1-(4-amino-3-(2,3-difluoro-4-isopropoxyphenyl)-1H-pyrazolo[3,4-d]pyrimidin-1 -yl)ethyl)-3-phenylquinazolin-4(3H)-one (**II-A-2a**)
(R)-2-(1-(4-amino-3-(2,3-difluoro-4-isopropoxyphenyl)-1H-pyrazolo[3,4-d]pyrimidin-1 -yl)ethyl)-3-phenylquinazolin-4(3H)-one (**II-A-2b**)
2-(1-(4-amino-3-(4-ethoxy-2,3-difluorophenyl)-1H-pyrazolo[3,4-d]pyrimidin-1-yl)ethyl )-3-phenylquinazolin-4(3H)-one (**II-A-3**)
2-(1-(4-amino-3-(4-cyclopropoxy-2,3-difluorophenyl)-1H-pyrazolo[3,4-d]pyrimidin-1-yl)ethyl)-3-phenylquinazolin-4(3H)-one (**II-A-4**)
2-(1-(4-amino-3-(3-chloro-2-fluoro-4-methoxyphenyl)-1H-pyrazolo[3,4-d]pyrimidin-1-yl)ethyl)-3-phenylquinazolin-4(3H)-one (**II-A-5**)
3-(4-amino-1-(1-(4-oxo-3-phenyl-3,4-dihydroquinazolin-2-yl)ethyl)-1H-pyrazolo[3,4-d] pyrimidin-3-yl)-2-fluoro-6-methoxybenzonitrile (**II-A-6**)
2-(1-(4-amino-3-(2,3-difluoro-4-methoxyphenyl)-1H-pyrazolo[3,4-d]pyrimidin-1-yl)eth yl)-5-chloro-3-phenylquinazolin-4(3H)-one (**II-A-7**)
(S)-2-(1(4-amino-3-(2,3-difluoro-4-methoxyphenyl)-1H-pyrazolo[3,4-d]pyrimidin-1-yl )ethyl)-5-chloro-3-phenylquinazolin-4(3H)-one (**II-A-7a**)
(R)-2-(1-(4-amino-3-(2,3-difluoro-4-methoxyphenyl)-1H-pyrazolo[3,4-d]pyrimidin-1-yl )ethyl)-5-chloro-3-phenylquinazolin-4(3H)-one (**II-A-7b**)
2-(1-(4-amino-3-(2,3-difluoro-4-methoxyphenyl)-1H-pyrazolo[3,4-d]pyrimidin-1-yl)eth yl)-5-fluoro-3-phenylquinazolin-4(3H)-one (**II-A-8**)
(S)-2-(1(4-amino-3-(2,3-difluoro-4-methoxyphenyl)-1H-pyrazolo[3,4-d]pyrimidin-1-yl )ethyl)-5-fluoro-3-phenylquinazolin-4(3H)-one (**II-A-8a**)
(R)-2-(1-(4-amino-3-(2,3-difluoro-4-methoxyphenyl)-1H-pyrazolo[3,4-d]pyrimidin-1-yl )ethyl)-5-fluoro-3-phenylquinazolin-4(3H)-one (**II-A-8b**)
2-(1-(4-amino-3-(2,3-difluoro-4-methoxyphenyl)-1H-pyrazolo[3,4-d]pyrimidin-1-yl)eth yl)-6-fluoro-3-phenylquinazolin-4(3H)-one (**II-A-9**)
2-(1-(4-amino-3-(2,3-difluoro-4-methoxyphenyl)-1H-pyrazolo[3,4-d]pyrimidin-1-yl)eth yl)-5-chloro-8-fluoro-3-phenylquinazolin-4(3H)-one (**II-A-10**)
2-(1-(4-amino-3-(2,3-difluoro-4-methoxyphenyl)-1H-pyrazolo[3,4-d]pyrimidin-1-yl)eth yl)-5,8-difluoro-3-phenylquinazolin-4(3H)-one (**II-A-11**)
2-(1-(4-amino-3-(2,6-difluoro-4-methoxyphenyl)-1H-pyrazolo[3,4-d]pyrimidin-1-yl)eth yl)-3-phenylquinazolin-4(3H)-one (**II-A-12**)
2-(1-(4-amino-3-(2-fluoro-6-methoxpyridin-3-yl)-1H-pyrazolo[3,4-d]pyrimidin-1-yl)eth yl)-3-phenylquinazolin-4(3H)-one (**II-A-13**)
2-(1-(4-amino-3 -(2-fluoro-6-isopropoxypyridin-3-yl)-1H-pyrazolo[3,4-d]pyrimidin-1-yl )ethyl)-3-phenylquinazolin-4(3H)-one (**II-A-14**)
2-(1-(4-amino-3-(2-fluoro-6-pheoxypyridin-3-yl)-1H-pyrazolo[3,4-d]pyrimidin-1-yl)eth yl)-3-phenylquinazolin-4(3H)-one (**II-A-15**)
2-(1-(4-amino-3-(2,3-difluoro-4-isopropoxyphenyl)-1H-pyrazolo[3,4-d]pyrimidin-1-yl) ethyl)-5-fluoro-3-phenylquinazolin-4(3H)-one (**II-A-16**)
2-(1-(4-amino-3-(2,3-difluoro-4-methoxyphenyl)-1H-pyrazolo[3,4-d]pyrimidin-1-yl)eth yl)-3-(pyridin-3-yl)quinazolin-4(3H)-one (**II-B-1**)
2-(1-(4-amino-3-(2,3-difluoro-4-methoxyphenyl)-1H-pyrazolo[3,4-d]pyrimidin-1-yl)eth yl)-5-chloro-3-(pyridin-3-yl)quinazolin-4(3H)-one (**II-B-2**)
(S)-2-(1(4-amino-3-(2,3-difluoro-4-methoxyphenyl)-1H-pyrazolo[3,4-d]pyrimidin-1-yl )ethyl)-5-chloro-3-(pyridin-3-yl)quinazolin-4(3H)-one (**II-B-2a**)
(R)-2-(1-(4-amino-3-(2,3-difluoro-4-methoxyphenyl)-1H-pyrazolo[3,4-d]pyrimidin-1-yl )ethyl)-5-chloro-3-(pyridin-3-yl)quinazolin-4(3H)-one (**II-B-2b**)
2-(1-(4-amino-3-(2,3-difluoro-4-methoxyphenyl)-1H-pyrazolo[3,4-d]pyrimidin-1-yl)eth yl)-5-chloro-3-(5-fluoropyridin-3-yl)quinazolin-4(3H)-one (**II-B-3**)
2-(1-(4-amino-3-(2,3-difluoro-4-methoxyphenyl)-1H-pyrazolo[3,4-d]pyrimidin-1-yl)eth yl)-5-chloro-3-cyclohexylquinazolin-4(3H)-one (**II-C-1**)
2-(1-(4-amino-3-(2,3-difluoro-4-methoxyphenyl)-1H-pyrazolo[3,4-d]pyrimidin-1-yl)eth yl)-5-chloro-3-(piperidin-3-yl)quinazolin-4(3H)-one (**II-C-2**)
3-(1-acetylpiperidin-4-yl)-2-(1-(4-amino-3-(2,3-difluoro-4-methoxyphenyl)-1H-pyrazol o[3,4-d]pyrimidin-1-yl)ethyl)-5-chloroquinazolin-4(3H)-one (**II-C-3**)
2-(1-(4-amino-3-(2,3-difluoro-4-methoxyphenyl)-1H-pyrazolo[3,4-d]pyrimidin-1-yl)eth yl)-5-chloro-3-(1-(2,2,2-trifluoroethyl)piperidin-4-yl)quinazolin-4(3H)-one (**II-C-4**)
2-(1-(4-amino-3-(2,3-difluoro-4-methoxyphenyl)-1H-pyrazolo[3,4-d]pyrimidin-1-yl)eth yl)-5-chloro-3-(piperidin-1-yl)quinazolin-4(3H)-one (**II-C-5**)
2-(1-(4-amino-3-(2,3-difluoro-4-methoxyphenyl)-1H-pyrazolo[3,4-d]pyrimidin-1-yl)eth yl)-5-chloro-3-morpholinylquinazolin-4(3H)-one (**II-C-6**)
2-(1-(4-amino-3-(2,3-difluoro-4-methoxyphenyl)-1H-pyrazolo[3,4-d]pyrimidin-1-yl)eth yl)-5-chloro-3-(piperazin-1-yl)quinazolin-4(3H)-one (**II-C-7**)
2-(1-(4-amino-3-(2,3-difluoro-4-methoxyphenyl)-1H-pyrazolo[3,4-d]pyrimidin-1-yl)eth yl)-5-chloro-3-(3,3-dimethylpiperazin-1-yl)quinazolin-4(3H)-one (**II-C-8**)
2-(1-(4-amino-3-(2,3-difluoro-4-methoxyphenyl)-1H-pyrazolo[3,4-d]pyrimidin-1-yl)eth yl)-5-chloro-3-(3-(trifluoromethyl)piperazin-1-yl)quinazolin-4(3H)-one (**II-C-9**)
2-(1-(4-amino-3-(2,3-difluoro-4-methoxyphenyl)-1H-pyrazolo[3,4-d]pyrimidin-1-yl)eth yl)-5-chloro-3-(4-(2,2,2-trifluoroethyl)piperazin-1-yl)quinazolin-4(3H)-one (**II-C-10**)
2-(1-(4-amino-3-(2,3-difluoro-4-methoxyphenyl)-1H-pyrazolo[3,4-d]pyrimidin-1-yl)eth yl)-5-chloro-3-(4-methylpiperazin-1-yl)quinazolin-4(3H)-one (**II-C-11**)
3-(4-acetylpiperazin-1-yl)-2-(1-(4-amino-3-(2,3-difluoro-4-methoxyphenyl)-1H-pyrazol o[3,4-d]pyrimidin-1-yl)ethyl)-5-chloroquinazolin-4(3H)-one (**II-C-12**)
2-(1-(4-amino-3-(2,3-difluoro-4-methoxyphenyl)-1H-pyrazolo[3,4-d]pyrimidin-1-yl)eth yl)-3-(3-methoxypropyl)quinazolin-4(3H)-one (**II-C-13**)
2-(1-(4-amino-3-(2,3-difluoro-4-methoxyphenyl)-1H-pyrazolo[3,4-d]pyrimidin-1-yl)eth yl)-5-chloro-3-(3-methoxypropyl)quinazolin-4(3H)-one (**II-C-14**)
2-(1-(4-amino-3-(2,3-difluoro-4-methoxyphenyl)-1H-pyrazolo[3,4-d]pyrimidin-1-yl)eth yl)-5-chloro-3-(2-(tetrahydrofuran-2-yl)ethyl)quinazolin-4(3H)-one (**II-C-15**)
or a stereoisomer thereof, or a stereoisomer mixture thereof, or a pharmaceutically acceptable salt or solvate thereof.

As preferred, the compound is the following compounds:
(S)-2-(1(4-amino-3-(2,3-difluoro-4-methoxyphenyl)-1H-pyrazolo[3,4-d]pyrimidin-1-yl )ethyl)-3-phenylquinazolin-4(3H)-one hydrochloride (**II-D-1**)
(S)-2-(1(4-amino-3 -(2,3 -difluoro-4-methoxyphenyl)-1H-pyrazolo[3,4-d]pyrimidin-1-yl )ethyl)-3-phenylquinazolin-4(3H)-one sulfate (**II-D-2**)
(S)-2-(1(4-amino-3 -(2,3 -difluoro-4-methoxyphenyl)-1H-pyrazolo[3,4-d]pyrimidin-1-yl )ethyl)-3-phenylquinazolin-4(3H)-one p-toluenesulfonate (**II-D-3**)
(S)-2-(1(4-amino-3 -(2,3 -difluoro-4-methoxyphenyl)-1H-pyrazolo[3,4-d]pyrimidin-1-yl )ethyl)-3-phenylquinazolin-4(3H)-one methanesulfonate (**II-D-4**)
(S)-2-(1(4-amino-3 -(2,3 -difluoro-4-methoxyphenyl)-1H-pyrazolo[3,4-d]pyrimidin-1-yl )ethyl)-5-chloro-3-phenylquinazolin-4(3H)-one hydrochloride (**II-D-5**)
(S)-2-(1(4-amino-3 -(2,3 -difluoro-4-methoxyphenyl)-1H-pyrazolo[3,4-d]pyrimidin-1-yl )ethyl)-5-chloro-3-phenylquinazolin-4(3H)-one sulfate (**II-D-6**)
(S)-2-(1(4-amino-3 -(2,3 -difluoro-4-methoxyphenyl)-1H-pyrazolo[3,4-d]pyrimidin-1-yl )ethyl)-5-chloro-3-phenylquinazolin-4(3H)-one phosphate (**II-D-7**)
(S)-2-(1(4-amino-3-(2,3-difluoro-4-methoxyphenyl)-1H-pyrazolo[3,4-d]pyrimidin-1-yl )ethyl)-5-chloro-3-phenylquinazolin-4(3H)-one p-toluenesulfonate (**II-D-8**)
(S)-2-(1(4-amino-3-(2,3-difluoro-4-methoxyphenyl)-1H-pyrazolo[3,4-d]pyrimidin-1-yl )ethyl)-5-chloro-3-phenylquinazolin-4(3H)-one methanesulfonate (**II-D-9**)
(S)-2-(1(4-amino-3 -(2,3 -difluoro-4-methoxyphenyl)-1H-pyrazolo[3,4-d]pyrimidin-1-yl )ethyl)-5-chloro-3-phenylquinazolin-4(3H)-one citrate (**II-D-10**)
(S)-2-(1(4-amino-3 -(2,3 -difluoro-4-methoxyphenyl)-1H-pyrazolo[3,4-d]pyrimidin-1-yl )ethyl)-5-chloro-3-phenylquinazolin-4(3H)-one succinate **(II-D-11)**
(S)-2-(1(4-amino-3-(2,3-difluoro-4-methoxyphenyl)-1H-pyrazolo[3,4-d]pyrimidin-1-yl )ethyl)-5-chloro-3-phenylquinazolin-4(3H)-one-L-malate **(II-D-12)**
(S)-2-(1(4-amino-3-(2,3-difluoro-4-methoxyphenyl)-1H-pyrazolo[3,4-d]pyrimidin-1-yl )ethyl)-5-chloro-3-phenylquinazolin-4(3H)-one-D-malate **(II-D-13)**
(S)-2-(1(4-amino-3-(2,3-difluoro-4-methoxyphenyl)-1H-pyrazolo[3,4-d]pyrimidin-1-yl )ethyl)-5-chloro-3-phenylquinazolin-4(3H)-one-L-tartrate **(II-D-14)**
(S)-2-(1(4-amino-3-(2,3-difluoro-4-methoxyphenyl)-1H-pyrazolo[3,4-d]pyrimidin-1-yl )ethyl)-5-chloro-3-phenylquinazolin-4(3H)-one benzoate **(II-D-15)**
(S)-2-(1(4-amino-3-(2,3-difluoro-4-methoxyphenyl)-1H-pyrazolo[3,4-d]pyrimidin-1-yl )ethyl)-5-chloro-3-phenylquinazolin-4(3H)-one maleate **(II-D-16)**
(S)-2-(1(4-amino-3-(2,3-difluoro-4-methoxyphenyl)-1H-pyrazolo[3,4-d]pyrimidin-1-yl )ethyl)-5-chloro-3-phenylquinazolin-4(3H)-one oxalate **(II-D-17)**
or a stereoisomer thereof, or a stereoisomer mixture or solvate thereof.

There is further provided in the present invention a preparation method for the compound of general formula I, the method comprising the following 3 methods:

1) Method 1: alkylation of 3-iodo-1H-pyrazolo[3,4-d]pyrimidin-4-amine (compound 5) by compound 4 in the presence of a base yields compound 10, which then undergoes coupling reaction with compound 7 to give compound of general formula I:

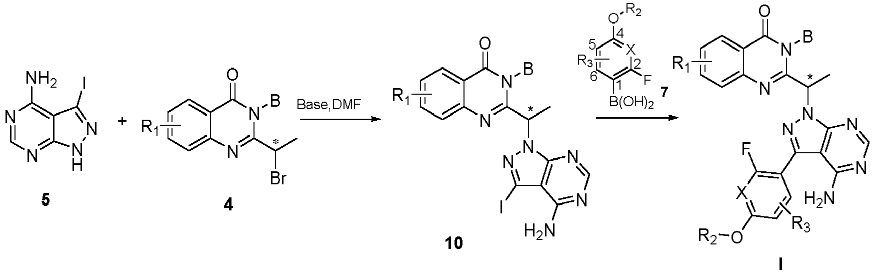

In the reaction of the first step, the reaction solvent can be selected from THF, MeCN, DMSO, etc. in addition to DMF. The mole ratio of compound 4 to compound 5 is about 1:(1~3), further preferably 1:(1-2). The base may be selected from potassium carbonate, caesium carbonate, sodium carbonate, etc.; the mole ratio of the base to compound 5 is about (1~5): 1, further preferably (1.5~2.5): 1. The reaction temperature is between room temperature and 60°C.

In the reaction of the second step, compound 10 reacts with compound 7 in the presence of a base (sodium carbonate or potassium carbonate) and a catalyst (such as a palladium catalyst). The catalyst is preferably tetrakis(triphenylphosphine)palladium, etc. The mole ratio of compound 10 to compound 7 is 1:(1~3), further preferably 1:(1~2). The mole ratio of the base to compound 10 is 1:(1∼4), further preferably 1:(2~3). The amount of the catalyst added is 0.05~1.0% of the moles of compound 10. The reaction solvent is a mixture of an organic solvent, such as dioxane, tetrahydrofuran and N,N-dimethylformamide, with water, etc., in a volume ratio of (2∼10): 1, further preferably (2~5): 1. The reaction temperature is 80~120°C.

When using this method, as preferred, the R₂ is preferably C₁-C₅ alkyl group (preferably methyl, ethyl, isopropyl, propyl, cyclopropyl) or phenyl, the R₃ is preferably H, halogen (preferably F), and X is C-halogen (preferably C-Cl, C-F, etc.), C-CN, CH or N.

2) Method 2: 3-iodo-1H-pyrazolo[3,4-d]pyrimidin-4-amine (compound 5) reacts with triphenylchloromethane in the presence of a base to produce compound 6, which then reacts with compound 7 by coupling to produce compound 8, which alkylates with compound 4 in the presence of a base after deprotection to produce a compound of general formula I:

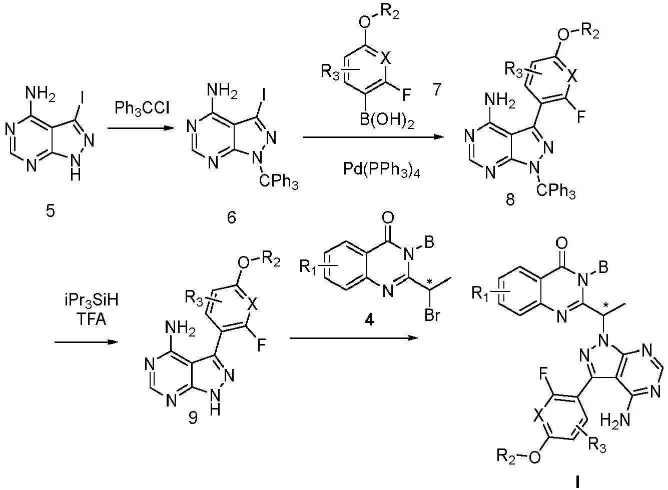

In the first step of this method, the mole ratio of triphenylchloromethane to compound 5 is (1∼5):1, preferably (1.5∼2.5):1; a base (preferably potassium carbonate or caesium carbonate) can be added in this step in a mole ratio of (1~8): 1 between the base and compound 5, preferably (2∼5):1. The reaction temperature is between 60 and 100°C.

In the second step, compound 6 reacts with compound 7 in the presence of a base (sodium carbonate, potassium carbonate) and a catalyst (such as a palladium catalyst). The catalyst is preferably tetrakis(triphenylphosphine)palladium, etc. The mole ratio of compound 6 to compound 7 is 1:(1∼3),further preferably 1:(1∼2). The mole ratio of the base to compound 6 is (1~4): 1, further preferably (2~3): 1. The amount of catalyst added is 0.05~1.0% of the moles of compound 6. The reaction solvent is a mixture of an organic solvent, such as dioxane, tetrahydrofuran and N,N-dimethylformamide, with water, etc., in a volume ratio of (2~10):1, further preferably (2~5): 1. The reaction temperature is between 80 and 120°C.

In the third step, TFA and triisopropylsilane are added during the reaction, and the reaction solvent can be dichloromethane, chloroform, etc. The mole ratio of TFA to compound 8 is (2~8):1, further preferably (3~6):1. The mole ratio of triisopropylsilane to compound 8 is (2∼8):1, further preferably (3~6):1.

In the fourth step, the reaction solvent can be selected from THF, MeCN, DMSO, etc. in addition to DMF. The mole ratio of compound 9 to compound 4 is about 1:(1∼3), further preferably 1:(1~2). A base, selected from potassium carbonate, caesium carbonate, sodium carbonate, etc., can be added during the reaction in a mole ratio of about (1∼5):1 between the base and compound 9, further preferably (1.5∼2.5):1. The reaction temperature is between room temperature and 60°C.

In this method, as preferred, R₁is halogen (preferably one or more of Cl, one or more of F, or a combination thereof); R₂ is C₁∼C₅ (methyl, ethyl, isopropyl etc.); and R₃ is H, halogen, etc. B is preferably phenyl, halogen (Cl, F) substituted or unsubstituted pyridinyl, piperidinyl, methyl substituted piperazinyl, Boc substituted piperidinyl, N-heteropiperidinyl, alkyl substituted piperazinyl, trifluoromethyl substituted piperazinyl, trifluoromethyl substituted alkyl substituted piperazinyl, acetyl substituted piperazinyl, substituted propyl, propyl, substituted ethyl, etc.

### 3) Method 3 (synthesis of a chiral compound):

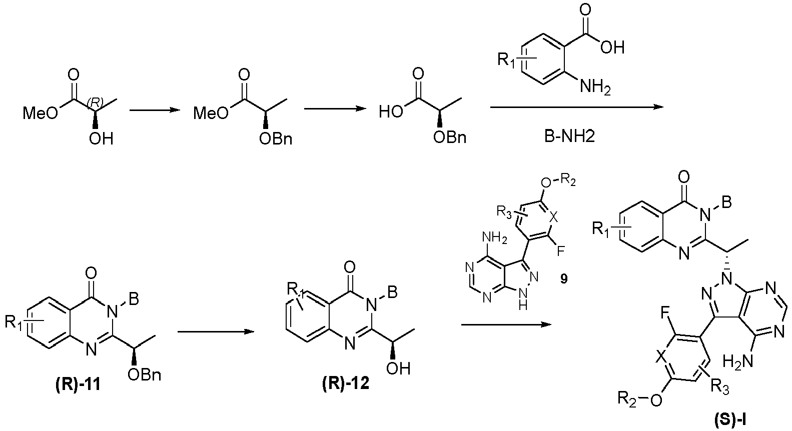

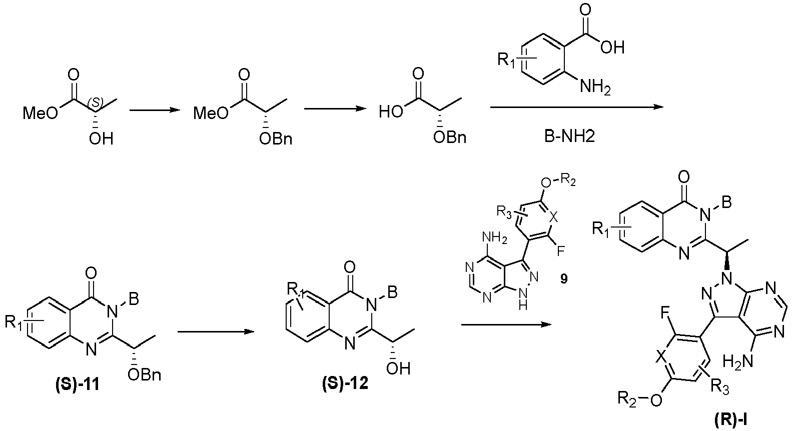

The method first employs chiral methyl D-lactate and methyl L-lactate to produce (R)-12 or (S)-12 by a multi-step reaction, followed by a Mitsunobu reaction with intermediate 9 in the presence of a phosphine ligand and azodicarboxylic acid ester to give (S)-I or (R)-I, respectively.

The reaction solvent of the Mitsunobu reaction may be THF, dichloromethane, toluene, acetonitrile, DMF, etc., in a volume ratio of (2∼10):1, preferably (2∼5):1 to intermediate 9; the phosphine ligand may be selected from triphenylphosphine, tri-tert-butylphosphine, in a molar ratio of (1~4): 1, preferably (1.5~3):1 to intermediate 9; the azodicarboxylic acid ester may be selected from DEAD and DIAD, in a molar ratio of (1~4): 1, preferably (1.5~3):1 to intermediate 9. The reaction temperature is between 15 and 30°C, preferably room temperature.

When using this method, as preferred, the R₂ is preferably C₁-C₅ alkyl (preferably methyl, ethyl, isopropyl, propyl, cyclopropyl) or phenyl, the R₃ is preferably H, halogen (preferably F), and X is C-halogen (preferably C-Cl, C-F, etc.), C-CN, CH or N.

It is another object of the present invention to provide an intermediate as shown by the following compound 9 and an application thereof in preparation of drugs for treating diseases, disorders or conditions that benefit from the inhibition of the activity of the casein kinase 1ε: 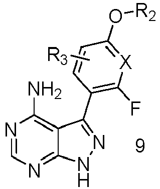 wherein:
R₂ is H, substituted or unsubstituted C₁-C₆ alkyl, substituted or unsubstituted C₃-C₁₀ cycloalkyl, or substituted or unsubstituted C₅-C₁₂ aryl; preferably R₂ is methyl, ethyl, isopropyl or cyclopropyl;
R₃ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, halogen, cyano, amino, nitro or hydroxy; preferably R₃ is F, Cl, CN or H;
X is CR₄ or N;
R₄ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, halogen, cyano, amino or hydroxy; preferably R₄ is F;

As a preferable scheme, the R₃ and R₄ are not H simultaneously.

Preferably, X is CF, N or CH.

As preferred, the intermediate as shown by compound 9 is preferably a compound as shown by the following formula: 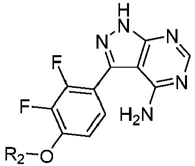 where R₂ is selected from methyl, ethyl, isopropyl and cyclopropyl.

### Description of Terms:

The term "aryl" as used herein is obtained by removing a H from an aromatic ring, the aromatic ring referring to an all-carbon monocyclic or fused polycyclic group of 5 to 12 carbon atoms with a fully conjugated π-electron system. Non-limiting examples of the aromatic ring are: benzene ring, naphthalene ring and anthracene ring. Aromatic rings may be unsubstituted or substituted. A substituent of the aromatic ring is selected from halogen (preferably fluorine, chlorine, bromine or iodine), cyano, nitro, amino, hydroxy, C₁-C₆ alkyl (preferably methyl, ethyl, propyl, isopropyl, butyl, isobutyl, sec-butyl, tert-butyl, pentyl, isopentyl, neopentyl, etc.), C₁-C₆ hydroxyalkyl (preferably hydroxymethyl, hydroxyethyl, hydroxypropyl, hydroxyisopropyl, etc.), C₁-C₆ alkoxy (preferably methoxy, ethoxy, propoxy, isopropoxy, butoxy, isobutoxy, sec-butoxy, tert-butoxy, etc.), C₁-C₆ alkyl halide (preferably halomethyl, haloethyl, halopropyl, haloisopropyl, halobutyl, haloisobutyl, sec-butyl halide, tert-butyl halide, etc.), C₁-C₆ hydroxyalkyl halide (preferably halohydroxymethyl, halohydroxyethyl, halohydroxypropyl, halohydroxyisopropyl, etc.), C₁-C₆ alkoxy halide (preferably halomethoxy, haloethoxy, halopropoxy, haloisopropoxy, halobutoxy, haloisobutoxy, sec-butoxy halide, tert-butoxy halide, etc.), C₃-C₆ cycloalkyl (preferably cyclopropyl, cyclopentyl, cyclohexyl, etc.), C₃-C₆ cycloalkyl halide (preferably halocyclopropyl, halocyclopentyl, halocyclohexyl, etc.), and 3- to 10- membered heterocyclyl (preferably tetrahydrofuranyl, pyrrolidinyl, piperidinyl, piperazinyl, morpholinyl, etc.); substitution of aromatic rings can be monosubstitution (e.g., ortho-, meta- or para-substitution), disubstitution or trisubstitution, etc.

The term "heteroaryl" as used herein refers to an unsaturated ring group of 5 to 12 ring atoms with a fully conjugated π-electron system, equivalent to the above "aryl" with one or more carbons replaced by a heteroatom such as oxygen, nitrogen, sulfur, etc. Heteroaromatic rings can be monocyclic or dicyclic, i.e., formed by fusion of two rings. A particular heterocyclic ring (heteroaryl) can be: pyridinyl, pyrimidinyl, pyrazinyl, isoxazolyl, isothiazolyl, pyrazolyl, thiazolyl, oxazolyl, imidazolyl, etc. Heteroaryl may be unsubstituted or substituted. A substituent of a heteroaryl is selected from halogen, cyano, nitro, amino, hydroxy, C₁-C₆ alkyl, C₁-C₆ hydroxyalkyl, C₁-C₆ alkoxy, C₁-C₆ alkyl halide, C₁-C₆ hydroxyalkyl halide, C₁-C₆ alkoxy halide, C₃-C₆ cycloalkyl, C₃-C₆ cycloalkyl halide and 3- to 10-membered heterocyclyl.

The term "alkyl" as used herein refers to a saturated straight monovalent hydrocarbon chain radical having from one to six carbon atoms, or a saturated branched monovalent hydrocarbon chain radical having from three to six carbon atoms, preferably being methyl, ethyl, propyl, isopropyl, butyl, isobutyl, sec-butyl, tert-butyl, pentyl, iso-pentyl, neopentyl, etc. Alkyl may be unsubstituted or monosubstituted or multi-substituted, and the substituents may be the same or different in a case of multi-substitution; the substituent of alkyl is selected from halogen, nitro, hydroxy, C₁-C₆ alkyl, C₁-C₆ alkoxy, C₃-C₁₀cycloalkyl, alkoxycarbonyl, alkylthio, alkylsulfonyl, alkyamide, hydroxyalkylamide, sulfonamide, 3- to 10-membered heterocyclyl, or amino or mono- or multi-substituted amino, wherein substituents of amino may be the same or different, selected from H, C₁-C₆ alkyl, C₁-C₆ hydroxyalkyl, C₁-C₆ alkoxy, C₃-C₁₀ cycloalkyl, and 3- to 10-membered heterocyclyl.

The term "hydroxyalkyl" used herein refers to -alkyl-OH, wherein the alkyl is as above defined. Embodiments of "hydroxyalkyl" as used herein include, but are not limited to, hydroxymethyl, hydroxyethyl, hydroxypropyl, hydroxyisopropyl, etc. "Hydroxyalkyl" also includes substituted hydroxyalkyl, the substituent thereof may be halogen, amino, hydroxy, C₁-C₆ alkyl, C₁-C₆ hydroxyalkyl, C₁-C₆ alkoxy or C₁-C₆ cycloalkyl.

The term "alkoxy" used herein refers to -O-alkyl group, wherein the alkyl is as above defined. Embodiments of "alkoxy" as used herein include, but are not limited to, methoxy, ethoxy, n-propoxy, isopropoxy, n-butoxy and tert-butoxy. "alkoxy" also includes substituted alkoxy, the substituent thereof may be halogen, amino, hydroxy, C₁-C₆ alkyl, C₁-C₆ hydroxyalkyl, C₁-C₆ alkoxy or C₁-C₆ cycloalkyl.

The term "cycloalkyl" used herein refers to a monocyclic or polycyclic (two monocyclic rings chemically bonded or bridged or spiro or fused), cyclically saturated, monovalent hydrocarbon group having 3 to 10 carbon atoms, preferably cyclopropyl, cyclobutyl, cyclopentyl, cyclohexyl, etc., wherein one or two carbon atoms can be replaced by an oxo group. The cycloalkyl can be unsubstituted or substituted, the substituent thereof being selected from halogen, nitro, hydroxy, C₁-C₆ alkyl, C₁-C₆ hydroxyalkyl, C₁-C₆ alkoxy, C₁-C₆ alkyl halide, C₁-C₆ hydroxyalkyl halide, C₁-C₆ alkoxy halide, C₃-C₆ cycloalkyl, C₃-C₆ cycloalkyl halide, alkoxycarbonyl, alkylthio, alkylsulfonyl, alkyamide, hydroxyalkylamide, sulfonamide, 3- to 10-membered heterocyclyl, or amino or mono- or multi-substituted amino, wherein substituents of amino may be the same or different, selected from H, C₁-C₆ alkyl, C₁-C₆ hydroxyalkyl, C₁-C₆ alkoxy, C₃-C₁₀cycloalkyl, and 3- to 10-membered heterocyclyl.

The term "heterocyclyl" used herein refers to a monocyclic or polycyclic (two monocyclic rings chemically bonded or bridged or spiro or fused) cyclyl having 3 to 10 ring atoms, one or more heteroatoms being selected from N, O and S, including but not limited to 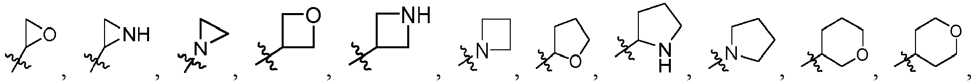 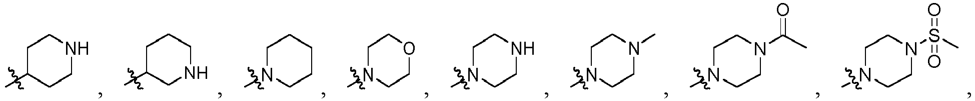 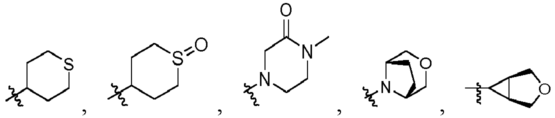 and

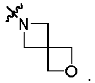

The cycloalkyl can be unsubstituted or substituted, the substituent thereof being selected from halogen, nitro, hydroxy, C₁-C₆ alkyl, C₁-C₆ hydroxyalkyl, C₁-C₆ alkoxy, C₁-C₆ alkyl halide, C₁-C₆ hydroxyalkyl halide, C₁-C₆ alkoxy halide, C₃-C₆ cycloalkyl, C₃-C₆ cycloalkyl halide, alkoxycarbonyl, alkylthio, alkylsulfonyl, alkyamide, hydroxyalkylamide, sulfonamide, 3- to 10- membered heterocyclyl, or amino, or mono- or multi-substituted amino, wherein substituents of amino may be the same or different, selected from H, C₁-C₆ alkyl, C₁-C₆ hydroxyalkyl, C₁-C₆ alkoxy, C₃-C₁₀cycloalkyl and 3- to 10-membered heterocyclyl.

The term "alkenyl" used herein refers to branched or unbranched hydrocarbon chain of 2 to 10 carbon atoms having at least one double bond (i.e., C₂-C₁₀ alkenyl), including but not limited to vinyl, allyl, but-1-enyl, pent-1-enyl, pent-1,4-di-alkenyl, etc. Alkenyl can be substituted by one or more substituents, the substituents are independently alkyl, alkyl halide, alkoxy, alkoxy halide, hydroxyalkyl, hydroxyalkyl halide, cycloalkyl, cycloalkyl halide, heterocycloalkyl, aryl, heteroaryl, hydroxy, halogen, cyano or nitro.

The term "alkinyl" used herein refers to branched or unbranched hydrocarbon chain composed of carbon and hydrogen atoms, containing at least one triple bond and 2 to 10 carbon atoms (i.e., C₂-C₁₀ alkinyl), including but not limited to ethynyl, propargyl, butynyl, pentynyl and hexynyl, etc. Alkinyl can be substituted by one or more substituents, the substituents are independently alkyl, alkyl halide, alkoxy, alkoxy halide, hydroxyalkyl, hydroxyalkyl halide, cycloalkyl, cycloalkyl halide, heterocycloalkyl, aryl, heteroaryl, hydroxy, halogen, cyano or nitro.

The term "halogen" used herein refers to fluorine, chlorine, bromine or iodine, preferably fluorine, chlorine or bromine.

The term "halide" used herein refers to having a same atom or different atoms substituted by halogen, substitution being one time or a plurality of times, such as di-substitution or tri-substitution.

The term "alkyl halide" used herein refers to alkyl group substituted by halogen (preferably fluorine, chlorine, bromine or iodine), wherein the alkyl is as above defined. "Alkyl halide" may be substituted by halogen once or a plurality of times.

The term "hydroxyalkyl halide" used herein refers to hydroxyalkyl group substituted by halogen (preferably fluorine, chlorine, bromine or iodine), wherein the hydroxyalkyl is as above defined. "Hydroxyalkyl halide" may be substituted by halogen once or a plurality of times.

The term "haloalkoxy" used herein refers to alkoxy group substituted by halogen (preferably fluorine, chlorine, bromine or iodine), wherein the alkoxy is as above defined. "haloalkoxy" may be substituted by halogen once or a plurality of times.

The term "cycloalkyl halide" used herein refers to cycloalkyl group substituted by halogen (preferably fluorine, chlorine, bromine or iodine), wherein the cycloalkyl is as above defined. "Cycloalkyl halide" may be substituted by halogen once or a plurality of times.

The term "m" as used herein is preferably 1 or 2; such as it being two same R₁or two different R₁. Take R₁ being F or Cl as an example, when m is 2, it may be Cl at two different substitution positions, or F at two different substitution positions, or a combination of Cl and F at two different substitution positions.

The term "n" as used herein is preferably absent or 1 or 2.

The term "o" as used herein is preferably 1, 2 or 3.

In the compounds of formula I, formulas II-A~II-C, formulas III-A~III-C, formulas IV-A~IV-C and formulas V-A~V-C, C denoted with ^{∗} is chiral and the compound has an R-configuration or S-configuration, or a mixture of R- and S-configurations. Further preferably, the compound has an S-configuration.

The term "solvate" as used herein refers to a variable stoichiometry complex formed by a solute (e.g., a compound of general formulas I to V-A~V-C of the present invention) and a solvent. For the purposes of the present invention, the solvent must not interfere with the biological activity of the solute. Examples of suitable solvents include, but are not limited to, water, methanol, ethanol, and acetic acid. Preferably, the solvent used is a pharmaceutically acceptable solvent. The pharmaceutically acceptable solvent as a suitable solvent includes, but is not limited to, water, ethanol, and acetic acid. Further preferably, the solvent used is water.

In the present invention, a salt of the compound herein may be prepared by using methods known to those skilled in the art. The salt may be an organic acid salt, an inorganic acid salt, etc. The organic acid salt comprisesfumarate, oxalate, malate, lactate, camphorsulfonate, p-toluenesulfonate, methanesulfonate, citrate, succinate, tartarate, maleate, benzoate, etc.; the inorganic acid salt comprises hydrohalate, sulfate, phosphate, nitrate, etc. For example, reaction with a lower alkyl sulfonic acid, such as methanesulfonic acid (methanesulphonic acid), trifluoromethanesulfonic acid, etc., may yield methanesulfonate (methanesulphonate), trifluoromethanesulfonate; reaction with aryl sulfonic acid, such as benzene sulfonic acid or p-toluenesulfonic acid, etc. may yield benzene sulfonate or p-toluenesulfonate; reaction with organic carboxylic acid, such as acetic acid, fumaric acid, tartaric acid, oxalic acid, maleic acid, malic acid, succinic acid or citric acid, etc. may yield a corresponding salt; reaction with amino acids, such as glutamic acid or aspartic acid may yield glutamate or aspartate. Reaction with an inorganic acid such as hydrohalic acid (e.g. hydrofluoric acid, hydrobromic acid, hydroiodic acid, hydrochloric acid), nitric acid, carbonic acid, sulfuric acid or phosphoric acid, may also yield a corresponding salt thereof.

The second purpose of the present invention is to provide a pharmaceutical composition comprising one or more of the compounds of any one above-mentioned technical scheme. The pharmaceutical composition herein may be composed of one or more of the compounds in any one of the above technical schemes with other compounds, or composed of one or more of the compounds in any one of the above technical schemes

In another aspect, provided in the present invention is an application of the compounds disclosed herein of general formulas I to general formulas V-A~V-C, and the stereoisomers thereof or stereoisomer mixtures thereof, or the pharmaceutically acceptable salts or solvates thereof in inhibiting the activity of the casein kinase 1ε (Ck1ε), or treating diseases, disorders or conditions that benefit from the inhibition of the activity of the casein kinase 1ε (CK1ε).

In a further preferable scheme, provided by the present invention is a method for inhibition of the activity of the casein kinase 1ε (CK1ε) of a subject by administration of a composition comprising at lease on compound of a therapeutically effective amount in the subject in need, wherein the compound has a structural formula of compounds of general formulas I to V-A~V-C.

In certain embodiments, the subject in need has cancer, the cancer comprising hematologic neoplasms and solid tumors, such as B-cell lymphoma, multiple myeloma, leukemia, lung cancer, breast cancer, prostate cancer, bladder cancer, ovarian cancer, pancreatic cancer, sarcoma, colon cancer, kidney cancer, melanoma, and brain tumors.

In a further embodiment, the subject in need has B-cell lymphoma, such as diffuse large B-cell lymphoma, follicular lymphoma, chronic lymphocytic lymphoma, chronic lymphocytic leukemia, B-cell prolymphocytic leukemia, lymphoplasmacytic lymphomalWaldenström macroglobulinemia, splenic marginal zone lymphoma, plasma cell myeloma, plasmacytoma, extranodal marginal zone B-cell lymphoma, nodal marginal zone B-cell lymphoma, mantle cell lymphoma, mediastinal (thymus) large B-cell lymphoma, intravascular large B-cell lymphoma, primary effusion lymphoma, Burkitt's lymphoma/leukemia or pulmonary lymphomatoid granulomatosis.

In a further embodiment, the subject in need has an autoimmune disease, such as rheumatoid arthritis, systemic lupus erythematosus, multiple sclerosis, inflammatory bowel disease, Crohn's disease, colitis, autoimmune hemolytic anemia, ankylosing spondylitis, pemphigus, urticaria, asthma, optic neuritis, psoriasis, chronic obstructive airway disease, dermatitis and baldness.

In a further embodiment, the subject in need has T-cell lymphoma, such as peripheral T-cell lymphoma, anaplastic large cell lymphoma, extranodal NK/T-cell lymphoma, cutaneous peripheral T-cell lymphoma, T-cell prolymphocytic leukemia, angioimmunoblastic T-cell lymphoma, adult T-cell leukemia/lymphoma, hepatosplenic T-cell lymphoma, intestinal T-cell lymphoma, breast implant-associated anaplastic large cell lymphoma, T-cell large granular lymphocytic leukemia.

Further provided in the present invention is an application of the compound or the pharmaceutically applicable salt thereof in preparation of a CK1ε inhibitor, particularly an application in preparation of drugs for treating proliferative diseases. The proliferative disease comprises cancer. In other words, further provided in the present invention is an application of the compounds of general formulas I to V-A~V-C, the stereoisomers or stereoisomer mixtures thereof, or the pharmaceutically acceptable salts or solvates thereof in treating proliferative diseases (such as cancers), used alone or in combination with other drugs. The antineoplastic drug which can be used in combination with the compound herein or the pharmaceutically applicable salt thereof includes, but is not limited to, at least one of the following types: mitotic inhibitors (e.g., vinblastine, vindisine and vinorelbine), tubulin proteolysis inhibitors (e.g., taxol), alkylation reagents (e.g., cisplatin, carboplatin, cyclophosphamide, chlorambucil and bendamustine), antimetabolites (e.g., 5-fluorouracil, tegafur, methotrexate, cytarabine and hydroxycarbamide), insertable antibiotics (e.g., adriamycin, mitomycin and bleomycin), enzymes (e.g., aspartate aminotransferase), topoisomerase inhibitors (e.g., etoposide and camptothecin), biological response modifiers (e.g., interferon), immunomodulators (e.g., lenalidomide), BTK inhibitors (e.g., ibrutinib, acalabrutinib), Bcl-2 inhibitors (e.g., Venetoclax), anti-CD20 monoclonal antibodies (e.g., rituximab, ofatumumab and obinutuzumab), mTOR inhibitors (e.g., rapamune and AZD8055), mTORC1 inhibitors (e.g., everolimus and temsirolimus), AKT inhibitors (e.g., MK-2206 and GSD690693), PI3K inhibitors (e.g., Idelalisib and Duvelisib), proteasome inhibitors (e.g., bortezomib, carfilzomib and ixazomib), EGFR inhibitors (e.g., erlotinib, gefitinib and osimertinib), VEGFR inhibitors (sorafenib, cabozantinib, ravatinib, levatinib, apatinib), CDK inhibitors (e.g., paboxinib, reboxinib) and PD-1/PD-L1 inhibitors (pembrolizumab, nivolumab, toripalimab, sintilimab).

The present invention also relates to a method for treating proliferative diseases by using the compound or pharmaceutical composition in combination with radiation treatment Techniques for radiation treatment administration are known in the art and can be used in the combination therapy of the present application.

The compound of the present invention can also be used in combination with one or more steroidal anti-inflammatory drugs, non-steroidal anti-inflammatory drugs or immunoselective anti-inflammatory derivatives, and combinations thereof.

The applicant has reported in prior patent literature (Patent Application No. 201710998664.5) a BTK irreversible inhibitor and optical isomer thereof or pharmaceutically acceptable salt or solvate thereof, of which 17a is a representative compound. Through further studies of the compound of the present application, we have found that the compound herein showed excellent synergistic anti-tumor activities in combination with a BTK inhibitor.

As demonstrated by experiments, the compound of the present invention has a potent inhibitory activity on *in-vitro* CK1ε kinase and *in-vitro* anti-tumor cells, showed good anti-tumor activity in an OCI-LY10 subcutaneous xenogeneic model when used alone, and excellent synergistic anti-tumor activity in combination with a BTK inhibitor, and the compound of the present invention has good pharmacokinetics properties and may be used alone or in combination with other drugs to treat diseases, disorders or conditions that benefit from the inhibition of the activity of the casein kinase 1ε, the diseases, disorders or conditions comprising tumors, autoimmune diseases, etc.

### DESCRIPTION OF THE EMBODIMENTS

The implementability of the present invention is illustrated hereinafter by means of embodiments. It should be understood by those skilled in the art that modifications or replacements of corresponding technical features based on the prior art still fall within the scope of protection claimed by the present invention.

### Embodiment 1: Synthesis of Intermediate 4

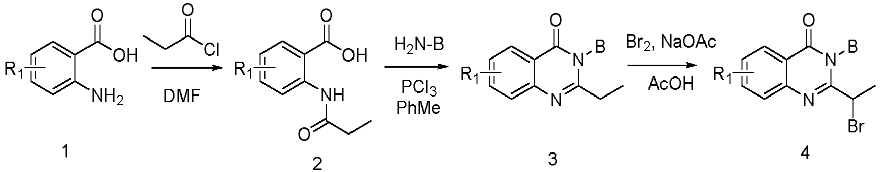

Step 1: Compound **1** (117mmol) and 40ml of DMF were placed in a 250ml reaction flask to dissolve with stirring at 0°C, then propionyl chloride (121mmol) was slowly dripped with a large amount of white solid precipitated after dripping. After 1h of the reaction at the maintained temperature, 200ml of water was added with stirring for 1h, then filtered and dried to obtain a solid product, Intermediate **2.**

Step 2: Intermediate **2** (10mmol), H₂N-B (20mmol) and 20ml of methylbenzene were placed in a 100ml reaction flask. PCl3₃ (10mmol) was dripped with stirring at room temperature, then the temperature was elevated to 110°C for reflux overnight. Once the reaction was completed as detected by the TLC test, ethyl acetate and water were used to extract. After drying and concentration, the crude product was purified by silica gel column chromatography to obtain Intermediate **3.**

Step 3: Intermediate **3** (8mmol), sodium acetate (11.2mmol) and 20ml of glacial acetic acid were placed in a 100ml reaction flask with stirring at room temperature, and then bromine (8mmol) was slowly dripped. The reaction continued after dripping for about 3h at room temperature till basically completed as detected by the TLC test; sodium thiosulfate aqueous solution was added with stirring for 15min, then ethyl acetate and water were used to extract. After drying and concentration, the crude product was purified by silica gel column chromatography to obtain Intermediate **4.**

The following compounds were prepared using the synthetic route and the method for the above Intermediate **4:**

For ease of description, Compound 1 is defined as: R₁-substituted 2-aminobenzoic acid; 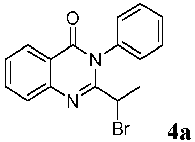 (R₁ in Compound **1** is H, B in H₂N-B is phenyl)

2-(1-bromoethyl)-3-phenylquinazolin-4(3H)-one; 3-step reaction with 55% overall yield; LC-MS (ESI-MS): 329[M+H]⁺; ¹H NMR (400 MHz, CDCl₃) δ 8.34 (d, *J* = 8.0 Hz, 1H), 7.91-7.76 (m, 2H), 7.71-7.49 (m, 5H), 7.22 (dt, *J=* 7.3, 2.9 Hz, 1H), 4.62 (q, *J=* 6.7 Hz, 1H), 2.10 (d, *J* = 6.7 Hz, 3H). 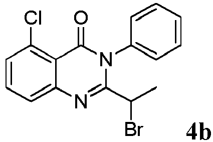 (R₁ in Compound **1** is substituted Cl at 6-position; B in H₂N-B is phenyl)

2-(1-bromoethyl)-5-chloro-3-phenylquinazolin-4(3H)-one, 3-step reaction with 49% overall yield; LC-MS (ESI-MS): 363[M+H]⁺; ¹H NMR (500 MHz, CDCl₃) δ 7.71 (dd, J = 8.2, 1.2 Hz, 1H), 7.64 (dd, J = 14.4, 6.3 Hz, 1H), 7.62 - 7.48 (m, 5H), 7.20 - 7.13 (m, 1H), 4.52 (q, J = 6.7 Hz, 1H), 2.03 (t, J = 7.2 Hz, 3H). 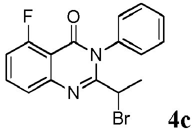 (R₁ in Compound **1** is F substituted at 6-position; B in H₂N-B is phenyl)

2-(1-bromoethyl)-5-fluoro-3-phenylquinazolin-4(3H)-one; 3-step reaction with 28% overall yield; LC-MS (ESI-MS): 347[M+H]⁺; ¹H NMR (500 MHz, CDCl₃) δ 7.73 (dd, J = 13.5, 8.1 Hz, 1H), 7.57 (dd, J = 22.5, 6.9 Hz, 5H), 7.16 (t, J = 9.0 Hz, 2H), 4.52 (q, J = 6.6 Hz, 1H), 2.02 (d, J = 6.7 Hz, 3H). 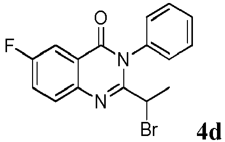 (R₁ in Compound 1 is F substituted at 5-position; B in H₂N-B is phenyl)

2-(1-bromoethyl)-6-fluoro-3-phenylquinazolin-4(3H)-one; 3-step reaction with 39% overall yield; LC-MS (ESI-MS): 347 [M+H]⁺. 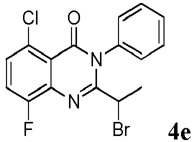 (R₁ in Compound 1 is F substituted at 3-position and Cl substituted at 6-position; B in H₂N-B is phenyl)

2-(1-bromoethyl)-5-chloro-8-fluoro-3-phenylquinazolin-4(3H)-one, 3-step reaction with 26% overall yield; LC-MS (ESI-MS): 381 [M+H]⁺. 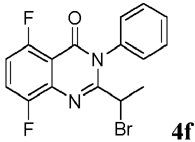 (R₁ in Compound 1 is F substituted at 3-position and F substituted at 6-position; B in H₂N-B is phenyl)

2-(1-bromoethyl)-5,8-difluoro-3-phenylquinazolin-4(3H)-one, 3-step reaction with 20% overall yield; LC-MS (ESI-MS): 365 [M+H]⁺. 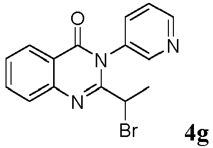 (R₁ in Compound 1 is H; B in H₂N-B is pyridin-3-yl)

2-(1-bromoethyl)-3-(pyridin-3-yl)quinazolin-4(3H)-one, 3-step reaction with 36% overall yield; LC-MS (ESI-MS): 330 [M+H]⁺. 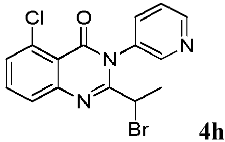 (R₁ in Compound 1 is Cl substituted at 6-position; B in H₂N-B is pyridin-3-yl)

2-(1-bromoethyl)-5-chloro-3-(pyridin-3-yl)quinazolin-4(3H)-one, 3-step reaction with 33% overall yield; LC-MS (ESI-MS): 364 [M+H]⁺. 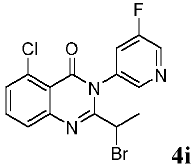 (R₁ in Compound **1** is Cl substituted at 6-position; B in H₂N-B is 5 -fluoropyridin-3 -yl)

2-(1-bromoethyl)-5-chloro-3-(5-fluoropyridin-3-yl)quinazoline-4(3H)-one, 3-step reaction with 35% overall yield; LC-MS (ESI-MS): 382 [M+H]⁺. 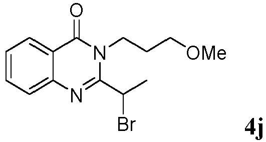 (R₁ in Compound 1 is H; B in H₂N-B is 3-methoxypropyl)

2-(1-bromoethyl)-3-(3-methoxypropyl)quinazolin-4(3H)-one, 3-step reaction with 45% overall yield; LC-MS (ESI-MS): 325 [M+H]⁺. 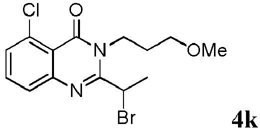 (R₁ in Compound **1** is Cl substituted at 6-position; B in H₂N-B is 3-methoxypropyl)

2-(1-bromoethyl)-5-chloro-3-(3-methoxypropyl)quinazolin-4(3H)-one, 3-step reaction with 46% overall yield; LC-MS (ESI-MS): 359 [M+H]⁺. 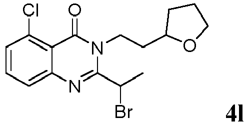 (R₁ in Compound 1 is Cl substituted at 6-position; B in H₂N-B is 2-(tetrahydrofuran-2-yl)ethyl)

2-(1-bromoethyl)-5-chloro-3-(2-(tetrahydrofuran-2-yl)ethyl)quinazolin-4(3H)-one, 3-step reaction with 30% overall yield; LC-MS (ESI-MS): 385 [M+H]⁺. 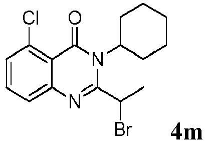 (R₁ in Compound **1** is Cl substituted at 6-position; B in H₂N-B is cyclohexyl)

2-(1-bromoethyl)-5-chloro-3-cyclohexylquinazolin-4(3H)-one, 3-step reaction with 18% overall yield; LC-MS (ESI-MS): 369 [M+H]⁺. 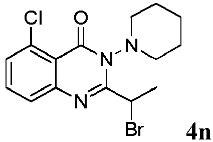 (R₁ in Compound 1 is Cl substituted at 6-position; B in H₂N-B is pyridin-1-yl)

2-(1-bromoethyl)-5-chloro-3-(pyridin-1-yl)quinazolin-4(3H)-one, 3-step reaction with 36% overall yield; LC-MS (ESI-MS): 370 [M+H]⁺. 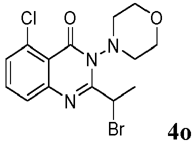 (R₁ in Compound **1** is Cl substituted at 6-position; B in H₂N-B is morpholin-4-yl)

2-(1-bromoethyl)-5-chloro-3-morpholinquinazolin-4(3H)-one, 3-step reaction with 40% overall yield; LC-MS (ESI-MS): 372 [M+H]⁺. 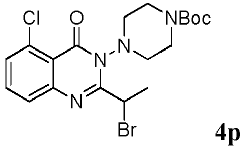 (R₁ in Compound **1** is Cl substituted at 6-position; B in H₂N-B is 4-boc-piperazinyl)

4-(2-(1-bromoethyl)-5-chloro-4-oxoquinazolin-3(4H)-yl)piperazine-1-carboxylic acid tert-butyl ester, 3-step reaction with 28% overall yield; LC-MS (ESI-MS): 471 [M+H]⁺. 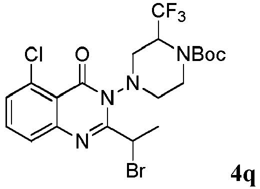 (R₁ in Compound **1** is Cl substituted at 6-position; B in H₂N-B is 3-trifluoromethyl-4-boc-piperazinyl)

4-(2-(1-bromoethyl)-5-chloro-4-oxoquinazolin-3(4H)-yl)-2-(trifluoromethyl)piperazine-1-carboxylic acid tert-butyl ester, 3-step reaction with 36% overall yield; LC-MS (ESI-MS): 539 [M+H]⁺. 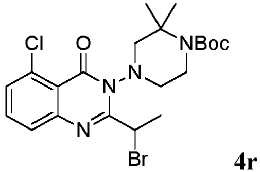 (R₁ in Compound **1** is Cl substituted at 6-position; B in H₂N-B is 3,3-dimethyl-4-boc-piperazinyl)

4-(2-(1-bromoethyl)-5-chloro-4-oxoquinazolin-3(4H)-yl)-2,2-dimethylpiperazine-1-carb oxylic acid tert-butyl ester, 3-step reaction with 31% overall yield; LC-MS (ESI-MS): 499 [M+H]⁺. 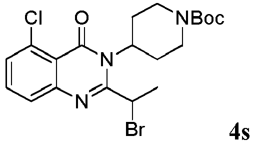 (R₁ in Compound 1 is Cl substituted at 6-position; B in H₂N-B is N-Boc-pyridin-4-yl)

4-(2-(1-bromoethyl)-5-chloro-4-oxoquinazolin-3(4H)-yl)pyridin-1-carboxylic acid tert-butyl ester, 3-step reaction with 15% overall yield; LC-MS (ESI-MS): 470 [M+H]⁺.

### Embodiment 2: Synthesis of Intermediate 9

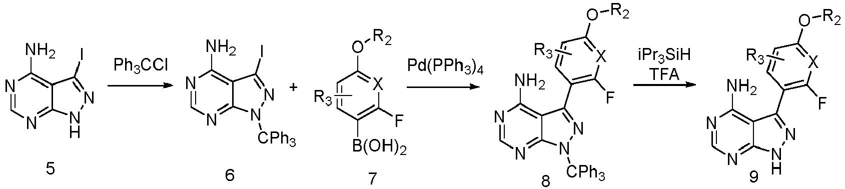

Step 1: Compound 5 (5g, 19mmol), triphenylmethyl chloride (10.7g, 38mmol), potassium carbonate (10.6g, 76mmol) and 50ml of DMF were placed separately in a 100ml reaction flask and reacted for about 24h at 80°C. Once the reaction was completed as detected by the TLC test, the temperature was lowered to room temperature, and ethyl acetate and water were added. Insoluble substances were removed by filtration, and the ethyl acetate layer was separated. The crude product concentrated under reduced pressure was purified by silica gel column chromatography to obtain 6.75g of the product, Intermediate **6,** with a yield of 73%.

Step 2: Intermediate **6** (18mmol), compound 7, potassium carbonate (54mmol), tetrakis(triphenylphosphine)palladium(0) (0.9mmol), 135ml of dioxane and 45ml of water were placed separately in a 500ml reaction flask. The temperature was elevated to 100°C with stirring to react for 14h and brought to room temperature when the reaction was completed as detected by the TLC test. The reaction liquid was filtered with diatomite, and the filter cake was rinsed with ethyl acetate. The recovered filtrate was extracted with 100ml of water and 300ml of ethyl acetate. The ethyl acetate layer was separated, dried over anhydrous sodium sulfate and concentrated under reduced pressure. The crude product was purified by silica gel column chromatography to obtain the product, Intermediate **8.**

Step 3: Intermediate **8** (3.85mmol) and 30ml of dichloromethane were placed separately in a 100ml reaction flask, then mixed with 1.15ml of TFA (15.4mmol) and 3.1ml of triisopropylsilane (15.4mmol) to react for 18h at room temperature. When the reaction was completed as detected by the TLC test, the solvent was directly evaporated with a rotary evaporator under reduced pressure till almost driness, then triturated with 20ml of MTBE (methyl tert-butyl ether) for 2h at room temperature and filtered to obtain the target product, Intermediate **9.**

The following compounds were prepared using the synthetic route and the method of Intermediate **9:**

The positions of carbon atoms on the conjugate ring in Compound 7 are defined as follows. 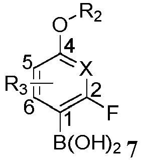 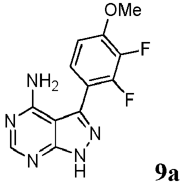 (in Compound 7: R₂ is methyl, X is CF; and R₃ is H)

3-(2,3-difluoro-4-methoxyphenyl)-1H-pyrazolo[3,4-d]pyrimidin-4-amine, 3-step reaction with 55% overall yield; LC-MS (ESI-MS): 278 [M+H]⁺. 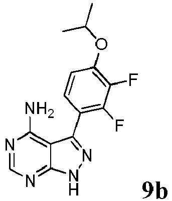 (in Compound 7: R₂ is isopropyl, X is CF, and R₃ is H)

3-(2,3-difluoro-4-isopropoxyphenyl)-1H-pyrazolo[3,4-d]pyrimidin-4-amine, 3-step reaction with 42% overall yield; LC-MS (ESI-MS): 306 [M+H]⁺. 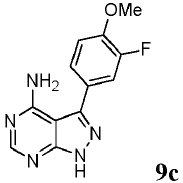 (Compound **7** was replaced with 3-fluoro-4-methoxyphenylboronic acid)

3-(3-fluoro-4-methoxyphenyl)-1H-pyrazolo[3,4-d]pyrimidin-4-amine, 3-step reaction with 58% overall yield; LC-MS (ESI-MS): 260 [M+H]⁺. 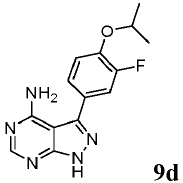 (Compound 7 was replaced with 3-fluoro-4-isopropoxyphenylboronic acid)

3-(3-fluoro-4-isopropoxyphenyl)-1H-pyrazolo[3,4-d]pyrimidin-4-amine, 3-step reaction with 55% overall yield; LC-MS (ESI-MS): 288 [M+H]⁺.

### Embodiment 4: Synthesis of Intermediate 10

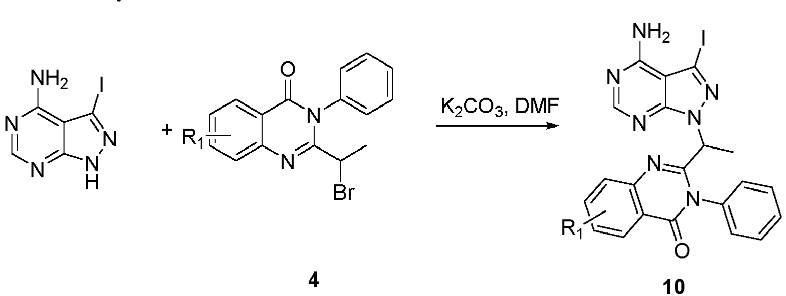

3-iodo-1H-pyrazolo[3,4-d]pyrimidin-4-amine (5.2g, 20mmol), potassium carbonate (4.2g, 30mmol) and 100ml of DMF were placed separately in a 500ml reaction flask, and then mixed with Compound **4** (8.6g, 26mmol) with stirring at room temperature to react for about 12h at room temperate. When the reaction was completed as detected by the TLC test, ethyl acetate and water were added to extract and then water was used to wash for 3 times. The ethyl acetate layer was separated, dried over anhydrous sodium sulfate and concentrated under reduced pressure. The crude product was then purified by silica gel column chromatography to obtain the white solid product, Intermediate **10.** 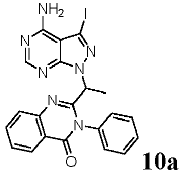 (Compound 4 was 2-(1-bromoethyl)-3-phenylquinazolin-4(3H)-one, i.e., R₁=H)

2-(1-(4-amino-3-iodo-1H-pyrazolo[3,4-d]pyrimidin-1-yl)ethyl)-3-phenylquinazolin-4(3 H)-one; 83% yield. LC-MS (ESI-MS): 510 [M+H]⁺.

### Embodiment 5: Synthesis of Intermediates (R)-12a~(R)-12d

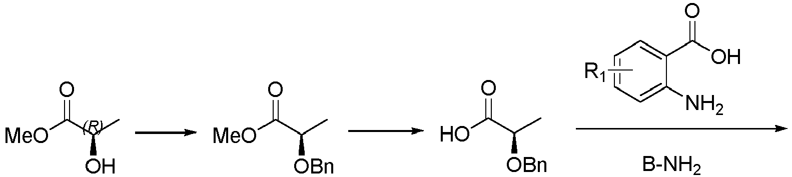

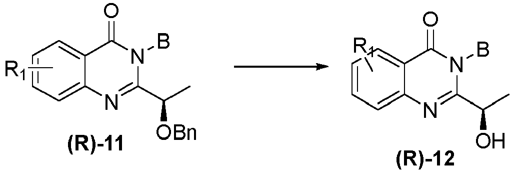

Step 1: Methyl (R)-(+)-lactate (20g, 192mmol) and 400ml of methyl tert-butyl ether (MTBE) were placed in a 1000ml reaction flask, and then mixed with benzyl bromide (36g, 211mmol) and silver oxide (44g, 192mmol) with stirring at room temperature to react for about 20h at room temperature till the reaction was completed as detected by the TLC test. The reaction liquid was filtered with diatomite and the filter cake with rinsed with MTBE. The filtrate was then concentrated under reduced pressure and purified by silica gel column chromatography to obtain 29.8g of a colorless oily product methyl (R) 2-(benzyloxy)propanoate with a yield of 80%. Chiral assay ee%: 99.0%, chiral-HPLC being performed using a normal-phase CHIRALCEL OD-H column with a retention time of 10.12min (retention time for the enantiomer was 8.07min). ¹H NMR (500 MHz, CDCl₃) δ 7.44-7.27 (m, 5H), 4.69 (d, *J* = 11.7 Hz, 1H), 4.46 (d, *J* = 11.7 Hz, 1H), 4.07 (q, *J* = 6.8 Hz, 1H), 3.76 (s, 3H), 1.44 (d, *J* = 6.9 Hz, 3H).

Step 2: Methyl (R)-2-(benzyloxy)propanoate (11g, 57mmol) and 100ml of methanol were placed in a reaction flask, and then mixed with lithium hydroxide (2.1g, 85mmol) with stirring to react for about 5h at room temperature till the reaction was completed as detected by the TLC test. Then 0.1N aqueous hydrochloric acid was added to neutralized to neutrality. Most of the methanol was removed by concentration under reduced pressure, then ethyl acetate and water were added to extract. The ethyl acetate layer was separated, dried over anhydrous sodium sulfate and concentrated under reduced pressure. The resulting residue was purified by silica gel column chromatography to obtain 8.0g of a colorless oily product (R)-2-(benzyloxy)propionic acid with a yield of 78%. ¹H NMR (500 MHz, CDCl₃) δ 7.45-7.28 (m, 5H), 4.72 (d, *J=* 11.6 Hz, 1H), 4.53 (d, *J* = 11.6 Hz, 1H), 4.12 (q, *J* = 6.9 Hz, 1H), 1.50 (d, *J* = 6.9 Hz, 3H).

Step 3: Substituted 2-amino-benzoic acid (19mmol), (R)-2-(benzyloxy)propionic acid (4.0g, 22mmol), triphenyl phosphite (23g, 76mmol) and 50ml of pyridine were placed in a reaction flask, then the reaction mixture was heated to 60°C and reacted for 2h with stirring. The B-NH2 (38mmol) was added to the reaction system, which was then heated to 70°C and reacted for about 12h with stirring till the reaction was completed as detected by the TLC test, and then brought to room temperature and concentrated under reduced pressure to remove most of the pyridine. Ethyl acetate was added, and washed with 2M aqueous potassium bisulfate for 3 times. The ethyl acetate layer was separated, dried over anhydrous sodium sulfate and concentrated under reduced pressure. The residue was purified by silica gel column chromatography to obtain a white solid product, Intermediate **(R)-11.**

Step 4: **(R)-11** (5.4 mmol) and 50ml of dichloromethane were added in a reaction flask, cooled to -10°C, and 28ml of 2M boron tribromide in dichloromethane solution was dripped. The mixture was then heated to room temperature after dripping to react for about 2h till the reaction was completed as detected by the TLC test. The reaction flask was placed in a ice bath and water was added to quench the reaction, and then dichloromethane was added to extract. The dichloromethane layer was separated, dried over anhydrous sodium sulfate and concentrated under reduced pressure. The residue was purified by silica gel column chromatography to obtain a white solid product, **(R)-12.**

The following compounds were prepared by using the synthetic route and the method of Intermediate **(R)-12:** 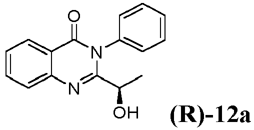 (R₁ in substituted 2-amino-benzoic acid is H; B in B-NH2 is phenyl)

(R)-2-(1-hydroxyethyl)-3-phenylquinazolin-4(3H)-one, 1.4g with 97% yield in the last step. LC-MS (ESI-MS): 267 [M+H]⁺. 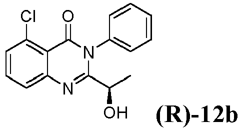 (R₁ in substituted 2-amino-benzoic acid is Cl substituted at 6-position; B in B-NH2 is phenyl)

(R)-5-chloro-2-(1-hydroxyethyl)-3-phenylquinazolin-4(3H)-one, 1.6g with 98% yield in the last step. Chiral assay ee%: 98.5%, chiral-HPLC being performed using a reversed-phase CHIRALPAK IG-3 column with a retention time of 52.7min. ¹H NMR (500 MHz, CDCl₃) δ 7.70-7.62 (m, 2H), 7.60-7.52 (m, 3H), 7.52-7.49 (m, 1H), 7.32 (dd, J = 7.5, 2.2 Hz, 1H), 7.27-7.23 (m, 1H), 4.45 (q, J = 6.4 Hz, 1H), 1.24 (d, J = 6.4 Hz, 3H). LC-MS (ESI-MS): 301 [M+H]⁺. 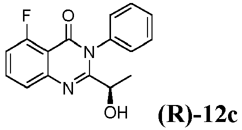 (R₁ in substituted 2-amino-benzoic acid is F substituted at 6-position; B in B-NH2 is phenyl)

(R)-5-fluoro-2-(1-hydroxyethyl)-3-phenylquinazolin-4(3H)-one, 1.5g with 98% yield in the last step. LC-MS (ESI-MS): 285 [M+H]⁺. 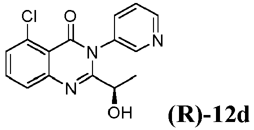 (R₁ in substituted 2-amino-benzoic acid is Cl substituted at 6-position; B in B-NH2 is pyridin-3-yl)

(R)-5-chloro-2-(1-hydroxyethyl)-3-(pyridin-3-yl)quinazolin-4(3H)-one, 1.5g with 92% yield in the last step. LC-MS (ESI-MS): 302 [M+H]⁺.

### Embodiment 6: Synthesis of Intermediates (S)-12a~(S)-12d

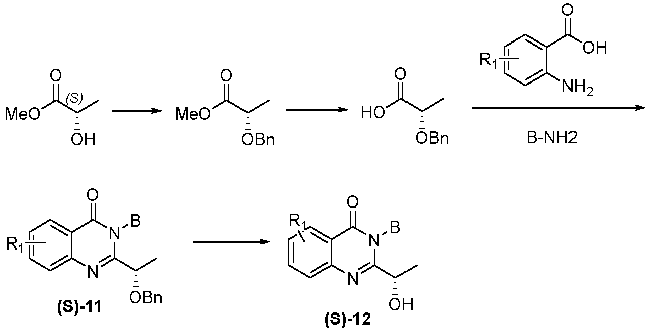

By referring to the preparation method of **Intermediate (R)-12 of Embodiment 5,** the following compounds were prepared using methyl (S)-(-)-lactate as the starting material: 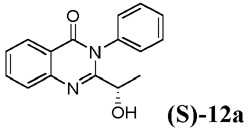 (R₁ in substituted 2-amino-benzoic acid is H; B in B-NH2 is phenyl)

(S)-2-(1-hydroxyethyl)-3-phenylquinazolin-4(3H)-one, 1.4g with 97% yield in the last step. LC-MS (ESI-MS): 267 [M+H]⁺. 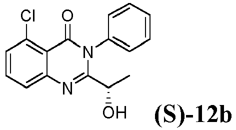 (R₁ in substituted 2-amino-benzoic acid is Cl substituted at 6-position; B in B-NH2 is phenyl)

(S)-5-chloro-2-(1-hydroxyethyl)-3-phenylquinazolin-4(3H)-one, 1.5g with 93% yield in the last step. Chiral assay ee%: 98.5%, chiral-HPLC being performed using a reversed-phase CHIRALPAK IG-3 column with a retention time of 43.7min. LC-MS (ESI-MS): 301 [M+H]⁺. 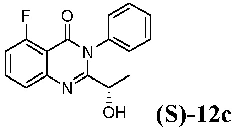 (R₁ in substituted 2-amino-benzoic acid is F substituted at 6-position; B in B-NH2 is phenyl)

(S)-5-fluoro-2-(1-hydroxyethyl)-3-phenylquinazolin-4(3H)-one, 1.4g with 91% yield in the last step. LC-MS (ESI-MS): 285 [M+H]⁺. 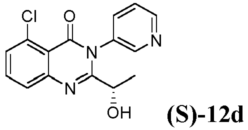 (R₁ in substituted 2-amino-benzoic acid is Cl substituted at 6-position; B in B-NH2 is pyridin-3-yl)

(S)-5-chloro-2-(1-hydroxyethyl)-3-(pyridin-3-yl)quinazolin-4(3H)-one, 1.5g with 92% yield in the last step. LC-MS (ESI-MS): 302 [M+H]⁺.

### Embodiment 7: Synthesis of Target Compounds II-A-1∼II-A-6 and II-A-12∼II-A-15

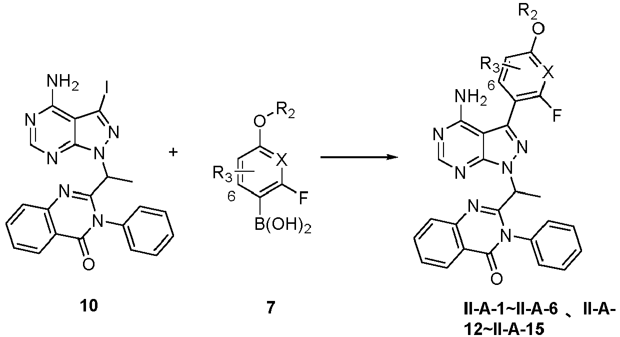

Intermediate **10** (2.0mmol), intermediate **7** (3.0mmol), potassium carbonate (4.0mmol), tetrakis(triphenylphosphine)palladium(0) (0.02mmol) were mixed with the solvent, 9ml of dioxane and 3ml of water in a 50ml reaction flask, heated to 100°C with stirring and reacted for about 14h till the reaction was completed as detected by the TLC test, then brought to room temperature. The reaction liquid was filtered with diatomite and rinsed with ethyl acetate. The filtrate was extracted with 20ml of water and 50ml of ethyl acetate, then the ethyl acetate layer was separated, dried over anhydrous sodium sulfate and concentrated under reduced pressure. The crude product was purified by silica gel column chromatography to obtain a white solid product.

**Target compound II-A-1 (R₂=CH₃, R₃=H, X=CF):** white solid product, 0.76g, 72% yield. ¹H NMR (500 MHz, CDCl₃) δ 8.27 (d, *J* = 7.9 Hz, 1H), 8.04 (s, 1H), 7.89-7.72 (m, 2H), 7.61-7.40 (m, 2H), 7.31 (d, *J* = 7.7 Hz, 1H), 7.21 (t, *J* = 7.5 Hz, 1H), 7.16 (t, *J* = 7.4 Hz, 1H), 6.90 (t, *J* = 7.7 Hz, 1H), 6.85 (t, *J* = 7.9 Hz, 1H), 6.54 (d, *J* = 7.7 Hz, 1H), 6.03 (q, *J* = 6.5 Hz, 1H), 5.45 (brs, 2H), 3.94 (s, 3H), 1.93 (d, *J* = 6.7 Hz, 3H). LC-MS (ESI-MS): 526 [M+H]⁺.

**Target compound II-A-2 (R₂=CH(CH₃)₂, R₃=H, X=CF):** white solid product, 0.78g, 70% yield. LC-MS (ESI-MS): 554 [M+H]⁺.

**Target compound II-A-3 (R₂=CH₂CH₃, R₃=H, X=CF):** white solid product, 0.68g, 63% yield. ¹H NMR (500 MHz, CDCl₃) δ 8.27 (d, *J* = 7.8 Hz, 1H), 8.04 (s, 1H), 7.92 - 7.73 (m, 2H), 7.49 (dt, *J* = 18.2, 7.7 Hz, 2H), 7.31 (d, *J* = 7.8 Hz, 1H), 7.17 (dt, *J* = 14.7, 7.3 Hz, 2H), 6.89 (t, *J* = 7.6 Hz, 1H), 6.83 (t, *J* = 7.6 Hz, 1H), 6.53 (d, *J* = 7.9 Hz, 1H), 6.03 (q, *J* = 6.7 Hz, 1H), 5.38 (brs, 2H), 4.16 (q, *J* = 6.9 Hz, 2H), 1.92 (d, *J* = 6.7 Hz, 3H), 1.48 (t, *J* = 7.0 Hz, 3H). LC-MS (ESI-MS): 540 [M+H]⁺.

**Target compound II-A-4 (R₂=cyclopropyl, R₃=H, X=CF):** white solid product, 0.41g, 37% yield. ¹H NMR (500 MHz, CDC13) δ 8.27 (d, *J* = 7.9 Hz, 1H), 8.03 (s, 1H), 7.80 (ddd, *J* = 11.6, 9.6, 4.3 Hz, 2H), 7.55 - 7.45 (m, 2H), 7.31 (d, *J* = 7.3 Hz, 1H), 7.24 - 7.13 (m, 2H), 6.89 (t, *J* = 7.7 Hz, 1H), 6.53 (d, *J* = 7.9 Hz, 1H), 6.03 (q, *J* = 6.7 Hz, 1H), 5.49 (brs, 2H), 3.87 (ddd, *J* = 9.0, 5.9, 3.1 Hz, 1H), 1.93 (t, *J* = 6.0 Hz, 3H), 0.89 - 0.82 (m, 4H). LC-MS (ESI-MS): 552 [M+H]⁺.

**Target compound II-A-5 (R₂=CH₃, R₃=H, X=CCl):** white solid product, 0.44g, 41% yield. LC-MS (ESI-MS): 542 [M+H]⁺.

**Target compound II-A-6 (R₂=CH₃, R₃=H, X=CCN):** white solid product, 0.42g, 39% yield. LC-MS (ESI-MS): 533 [M+H]⁺.

**Target compound II-A-12 (R₂=CH₃, R₃=6-F, X=CH):** white solid product, 0.36g, 34% yield. ¹H NMR (500 MHz, CDCl₃) δ 8.27 (d, *J* = 8.0 Hz, 1H), 8.03 (s, 1H), 7.91 - 7.71 (m, 2H), 7.63-7.41 (m, 2H), 7.31 (d, *J* = 7.6 Hz, 1H), 7.24-7.18 (m, 1H), 7.15 (dd, *J* = 14.5, 7.0 Hz, 1H), 6.89 (dd, *J* = 11.1, 4.4 Hz, 1H), 6.85 (t, *J* = 7.4 Hz, 1H), 6.54 (d, *J* = 8.1 Hz, 1H), 6.03 (q, *J* = 6.7 Hz, 1H), 5.49 (brs, 2H), 3.94 (s, 3H), 1.92 (d, *J* = 6.7 Hz, 3H). LC-MS (ESI-MS): 526 [M+H]⁺.

**Target compound II-A-13 (R₂=CH₃, R₃=H, X=N):** white solid product, 0.75g, 74% yield. LC-MS (ESI-MS): 509 [M+H]⁺.

**Target compound II-A-14 (R₂=CH(CH₃)₂, R₃=H, X=N):** white solid product, 0.72g, 67% yield. ¹H NMR (500 MHz, CDCl₃) δ 8.27 (dd, *J* = 7.9, 0.9 Hz, 1H), 8.03 (s, 1H), 7.89 - 7.75 (m, 2H), 7.54-7.49 (m, 1H), 7.47 (td, *J* = 7.7, 1.2 Hz, 1H), 7.31 (d, *J* = 7.9 Hz, 1H), 7.16 (t, *J* = 7.5 Hz, 1H), 6.96-6.87 (m, 1H), 6.67 (d, *J* = 8.2 Hz, 1H), 6.51 (d, *J* = 8.0 Hz, 1H), 6.02 (q, *J* = 6.7 Hz, 1H), 5.60 (brs, 2H), 5.33-5.22 (m, 1H), 4.06 (t, *J* = 6.7 Hz, 1H), 1.92 (d, *J* = 6.8 Hz, 3H), 1.36 (dd, *J* = 6.2, 3.0 Hz, 6H). LC-MS (ESI-MS): 537 [M+H]⁺.

**Target compound II-A-15 (R₂=Ph, R₃=H, X=N):** white solid product, 0.82g, 72% yield. ¹H NMR (500 MHz, CDCl₃) δ 8.27 (d, *J* = 7.7 Hz, 1H), 8.04 (s, 1H), 7.96 (t, *J* = 8.8 Hz, 1H), 7.81 (dt, *J* = 15.0, 7.4 Hz, 2H), 7.51 (t, *J* = 6.9 Hz, 1H), 7.47 (t, *J* = 7.9 Hz, 1H), 7.43 (t, *J* = 7.9 Hz, 2H), 7.36 - 7.23 (m, 2H), 7.16 (*d, J* = 7.6 Hz, 3H), 6.90 (t, *J=* 7.7 Hz, 1H), 6.86 (d, *J* = 8.1 Hz, 1H), 6.50 (d, *J=* 7.8 Hz, 1H), 6.03 (q, *J=* 6.6 Hz, 1H), 5.46 (brs, 2H), 1.92 (d, *J=* 6.7 Hz, 3H). LC-MS (ESI-MS): 571 [M+H]⁺.

### Embodiment 8: Synthesis of Target Compounds II-A-7∼II-A-11

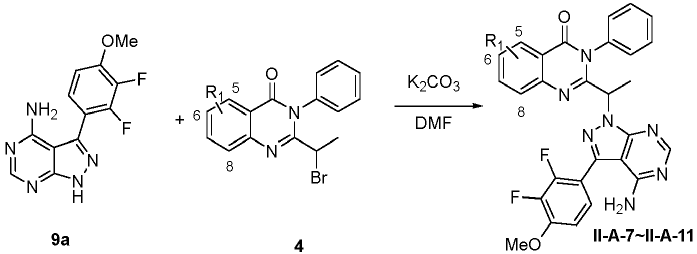

3-(2,3-difluoro-4-methoxyphenyl)-1H-pyrazolo[3,4-d]pyrimidin-4-amine (1.0mmol) and 5ml of DMF were placed separately in a 20ml reaction flask, and stirred to dissolve at room temperature, then mixed in turn with Intermediate **4** (1.1mmol) and potassium carbonate (2.0mmol), and heated to 40°C to react for about 15h. When the reaction was completed as detected by the TLC test, ethyl acetate was added to extract, and then washed with water. The ethyl acetate layer was separated, dried over anhydrous sodium sulphate and concentrated under reduced pressure. The resulting residue was purified by silica gel column chromatography to obtain the target product.

**Target compound II-A-7 (R₁=5-chloro):** white solid product, 0.48g, 85% yield. ¹H NMR (500 MHz, CDCl₃) δ 8.05 (s, 1H), 7.73 (d, *J* = 8.1 Hz, 1H), 7.64 (t, *J* = 8.0 Hz, 1H), 7.51 (d, *J* = 7.8 Hz, 1H), 7.47 (t, *J* = 7.6 Hz, 1H), 7.29 (d, *J* = 7.9 Hz, 1H), 7.22 (dd, *J* = 11.8, 4.4 Hz, 1H), 7.16 (t, *J* = 7.4 Hz, 1H), 6.93 - 6.84 (m, 2H), 6.53 (d, *J* = 8.0 Hz, 1H), 6.00 (q, *J* = 6.7 Hz, 1H), 5.33 (brs, 2H), 3.96 (s, 3H), 1.90 (d, *J=* 6.7 Hz, 3H). LC-MS (ESI-MS): 560 [M+H]⁺.

**Target compound II-A-8 (R₁=5-fluoro):** white solid product, 0.36g, 66% yield. ¹H NMR (500 MHz, CDCl₃) δ 8.04 (s, 1H), 7.71 (dd, *J* = 13.5, 8.1 Hz, 1H), 7.61 (d, *J* = 8.2 Hz, 1H), 7.47 (t, *J* = 7.6 Hz, 1H), 7.29 (d, *J* = 8.1 Hz, 1H), 7.21 (t, *J* = 7.9 Hz, 1H), 7.15 (t, *J* = 9.3 Hz, 2H), 6.87 (dd, *J* = 17.1, 8.6 Hz, 2H), 6.52 (d, J= 8.0 Hz, 1H), 6.00 (q, *J* = 6.6 Hz, 1H), 5.31 (brs, 2H), 3.95 (s, 3H), 1.90 (d, *J=* 6.7 Hz, 3H). LC-MS (ESI-MS): 544 [M+H]⁺.

**Target compound II-A-9 (R₁=6-fluoro):** white solid product, 0.38g, 70% yield. LC-MS (ESI-MS): 544 [M+H]⁺.

**Target compound II-A-10 (R₁=5-chloro-8-fluoro):** white solid product, 0.34g, 58% yield. LC-MS (ESI-MS): 578 [M+H]⁺.

**Target compound II-A-11 (R₁=5,8-difluoro):** white solid product, 0.37g, 65% yield. LC-MS (ESI-MS): 562 [M+H]⁺.

### Embodiment 9: Synthesis of Target Compound II-A-16

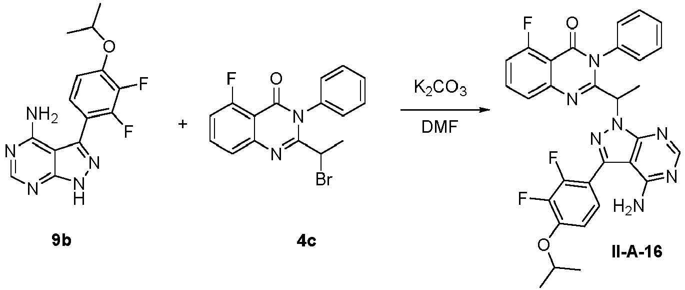

3-(2,3-difluoro-4-isopropoxyphenyl)-1H-pyrazolo[3,4-d]pyrimidin-4-amine (**9b**, 1.0mmol) and 5ml of DMF were placed separately in a 20ml reaction flask and stirred to dissolve at room temperature, then mixed in turn with Intermediate **4c** (1.1mmol) and potassium carbonate (2.0mmol), and heated to 40°C to react for about 15h. When the reaction was completed as detected by the TLC test, ethyl acetate was added to extract and washed with water. The ethyl acetate layer was separated, dried over anhydrous sodium sulphate and concentrated under reduced pressure. The resulting residue was purified by silica gel column chromatography to obtain 0.36g of the target product **II-A-16** with a yield of 63%. ¹H NMR (500 MHz, DMSO) δ 7.87 (brs, 2H), 7.60 (brs, 2H), 7.47-7.28 (m, 2H), 7.24-7.02 (m, 3H), 6.79 (brs, 1H), 6.31 (brs, 1H), 5.90 (brs, 1H), 4.71 (brs, 1H), 1.71 (s, 3H), 1.31 (s, 6H). LC-MS (ESI-MS): 572 [M+H]⁺.

### Example 10: Synthesis of Target Compounds II-B-1∼II-B-3

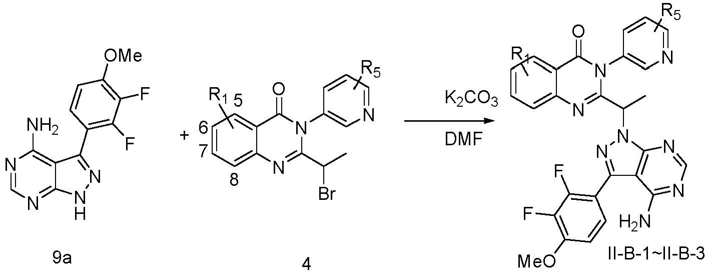

3-(2,3-difluoro-4-methoxyphenyl)-1H-pyrazolo[3,4-d]pyrimidin-4-amine (**9a**, 1.0mmol) was placed in a 20ml reaction flask with 5ml of DMF, and stirred to dissolve at room temperature, then mixed in turn with Intermediate **4** (1.1mmol) and potassium carbonate (2.0mmol), and heated to 40°C to react for about 15h. When the reaction was completed as detected by the TLC test, ethyl acetate was added to extracted, and washed with water. The ethyl acetate layer was separated, dried over anhydrous sodium sulphate and concentrated under reduced pressure. The resulting residue was purified by silica gel column chromatography to obtain the target product.

**Target product II-B-1 (R₁=H, R₅=H):** white solid product, 0.37g, 71% yield. LC-MS (ESI-MS): 527 [M+H]⁺.

**Target product II-B-2 (R₁=5-chloro, R₅=H):** white solid product, 0.36g, 65% yield. ¹H NMR (500 MHz, CDCl₃) δ 8.58 (d, *J* = 2.3 Hz, 0.5H), 8.39 (d, *J* = 3.6 Hz, 0.5H), 8.30 (d, *J* = 4.3 Hz, 0.5H), 8.08 (d, *J* = 11.6 Hz, 1H), 7.81 - 7.72 (m, 1.5H), 7.67 (td, *J=* 8.1, 3.9 Hz, 1.5H), 7.58 - 7.50 (m, 1H), 7.38 (dd, *J* = 8.0, 4.9 Hz, 0.5H), 7.29 - 7.23 (m, 0.5H), 7.20 (t, *J* = 7.3 Hz, 0.5H), 6.92-6.79 (m, 2H), 5.99 (q, *J* = 6.7 Hz, 0.5H), 5.90 (q, *J* = 6.8 Hz, 0.5H), 5.39 (brs, 2H), 3.95 (d, *J* = 2.0 Hz, 3H), 1.91 (t, *J* = 6.1 Hz, 3H). LC-MS (ESI-MS): 561 [M+H]⁺.

**Target product II-B-3 (R₁=5-chloro, R₅=5-fluoro):** white solid product, 0.35g, 60% yield. ¹H NMR (500 MHz, CDCl₃) δ 8.43 (s, 0.6H), 8.26 (d, *J* = 2.6 Hz, 0.6H), 8.16 (d, *J* = 2.5 Hz, 0.6H), 8.12 (s, 1H), 7.77 (d, *J* = 8.1 Hz, 1H), 7.69 (td, *J* = 8.0, 1.3 Hz, 1H), 7.63-7.52 (m, 1.4H), 7.46-7.41 (m, 0.4H), 7.25-7.18 (m, 1H), 6.88 (dd, *J* = 14.2, 7.0 Hz, 1H), 6.69-6.57 (m, 0.7H), 5.97 (dq, *J* = 24.5, 6.7 Hz, 1.3H), 5.33 (d, *J* = 24.5 Hz, 2H), 3.96 (d, *J* = 3.3 Hz, 3H), 2.11-1.76 (m, 3H). LC-MS (ESI-MS): 579 [M+H]⁺.

### Embodiment 11: Synthesis of Target Compounds II-C-1, II-C-5 and II-C-6

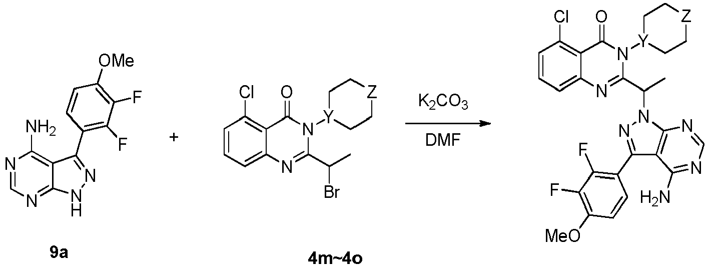

3-(2,3-difluoro-4-methoxyphenyl)-1H-pyrazolo[3,4-d]pyrimidin-4-amine (1.0mmol) was placed in a 20ml reaction flask with 5ml of DMF, and stirred to dissolve at room temperature, then mixed in turn with Intermediate **4** (1.1mmol) and potassium carbonate (2.0mmol), and heated to 40°C to react for about 15h. When the reaction was completed as detected by the TLC test, ethyl acetate was added to extract, and washed with water. The ethyl acetate layer was separated, dried with anhydrous sodium sulphate and concentrated under reduced pressure. The resulting residue was purified by silica gel column chromatography to obtain the target product.

**Target product II-C-1 (Y=CH, Z=CH₂):** white solid product, 0.41g, 72% yield. ¹H NMR (500 MHz, CDCl₃) δ 8.46 (s, 1H), 7.65 (d, *J* = 8.0 Hz, 1H), 7.55 (t, *J* = 8.0 Hz, 1H), 7.45 (d, *J* = 7.7 Hz, 1H), 7.18 (t, *J* = 7.4 Hz, 1H), 6.84 (t, *J* = 7.9 Hz, 1H), 6.36 (q, *J* = 6.5 Hz, 1H), 5.66 (brs, 2H), 4.00-3.86 (m, 1H), 3.93 (s, 3H), 2.73 (qd, *J* = 12.5, 3.4 Hz, 1H), 2.47 (qd, *J* = 12.7, 3.4 Hz, 1H), 1.99 (d, *J* = 6.5 Hz, 3H), 1.86-1.77 (m, 1H), 1.66 (d, *J* = 12.0 Hz, 1H), 1.44 (d, *J* = 12.8 Hz, 1H), 1.37 (d, *J* = 13.0 Hz, 1H), 1.29 (dd, *J* = 16.7, 6.7 Hz, 1H), 1.16-1.05 (m, 1H), 0.43 (d, *J* = 11.6 Hz, 1H), 0.11 (dd, *J* = 26.0, 13.0 Hz, 1H). LC-MS (ESI-MS): 566 [M+H]⁺.

**Target product II-C-5 (Y=N, Z=CH₂):** white solid product, 0.40g, 71% yield. ¹H NMR (500 MHz, CDCl₃) δ 8.43 (s, 1H), 7.59 (dt, *J* = 6.7, 3.3 Hz, 1H), 7.54 (t, *J* = 7.9 Hz, 1H), 7.43 (dd, *J* = 7.7, 1.1 Hz, 1H), 7.25-7.17 (m, 1H), 6.84 (dd, *J* = 11.8, 4.3 Hz, 1H), 6.53 (q, *J* = 6.9 Hz, 1H), 5.54 (brs, 2H), 4.02 - 3.90 (m, 1H), 3.94 (s, 3H), 3.88 - 3.72 (m, 1H), 2.98 (d, *J* = 10.3 Hz, 1H), 2.26 (d, *J* = 9.9 Hz, 1H), 1.95 (d, *J* = 6.9 Hz, 3H), 1.78-1.68 (m, 3H), 1.58 - 1.45 (m, 1H), 1.29 (d, *J* = 14.7 Hz, 1H), 1.22-1.06 (m, 1H), 0.45 (dtd, *J* = 12.9, 9.0, 4.0 Hz, 1H). LC-MS (ESI-MS): 567 [M+H]⁺.

**Target product II-C-6 (Y=N, Z=O):** white solid product, 0.31g, 55% yield. ¹H NMR (500 MHz, CDCl₃) δ 8.39 (s, 1H), 7.57 (d, *J* = 8.1 Hz, 1H), 7.51 (t, *J* = 8.0 Hz, 1H), 7.39 (d, *J* = 6.9 Hz, 1H), 7.13 (dd, *J* = 11.7, 4.7 Hz, 1H), 6.78 (t, *J* = 7.4 Hz, 1H), 6.48 (q, *J* = 6.9 Hz, 1H), 5.36 (brs, 2H), 4.18 (td, *J* = 11.2, 3.1 Hz, 1H), 4.05 (dd, *J* = 14.4, 7.3 Hz, 1H), 4.03 - 3.96 (m, 1H), 3.87 (s, 3H), 3.80 (d, *J* = 10.3 Hz, 1H), 3.65 (dd, *J* = 11.4, 9.2 Hz, 1H), 3.37 (d, *J* = 10.8 Hz, 1H), 2.77 (d, *J* = 10.6 Hz, 1H), 2.40 (dd, *J* = 11.4, 9.2 Hz, 1H), 1.87 (d, *J* = 6.9 Hz, 3H). LC-MS (ESI-MS): 569 [M+H]⁺.

### Embodiment 12: Synthesis of Target Compound II-C-2

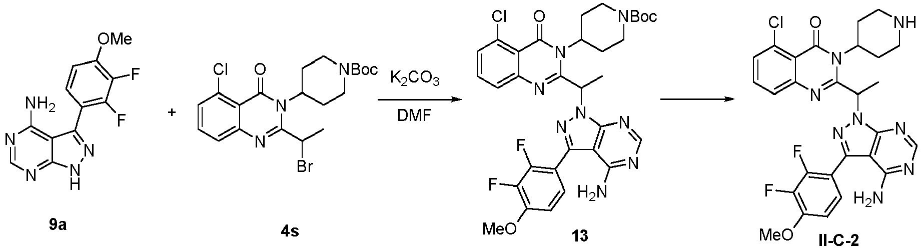

Step 1: 3-(2,3-difluoro-4-methoxyphenyl)-1H-pyrazolo[3,4-d]pyrimidin-4-amine (1.0mmol) was placed in a 20ml reaction flask with with 5ml of DMF, and stirred to dissolve at room temperature, then mixed in turn with 4-(2-bromoethyl)-5-chloro-4-oxoquinazolin-3(4H)-yl)piperidine-1-carboxylic acid, tert-butyl ester (1.1mmol) and potassium carbonate (2.0mmol), and heated to 40°C to react for about 15h. When the reaction was completed as detected by the TLC test, ethyl acetate was added to extract, and washed with water. The ethyl acetate layer was separated, dried over anhydrous sodium sulphate and concentrated under reduced pressure. The resulting residue was purified by silica gel column chromatography to obtain 0.46 g of **Intermediate 13** with a yield of 69%.

Step 2: **Intermediate 13** (0.6mmol) was placed in a 20ml reaction flask with 6ml of dichloromethane, then mixed with 1ml of trifluoroacetic acid to react for about 12h at room temperature. When the reaction was basically completed as detected by the TLC test, water and dichloromethane were added to extract. The organic phase was washed with saturated aqueous sodium bicarbonate solution. The dichloromethane layer was separated, dried over anhydrous sodium sulfate and concentrated under reduced pressure. The resulting residue was purified by silica gel column chromatography to obtain 0.27g of the target product, Compound **II-C-2**, with a yield of 80%. LC-MS (ESI-MS): 567 [M+H]⁺.

### Embodiment 13: Synthesis of Target Compound II-C-3

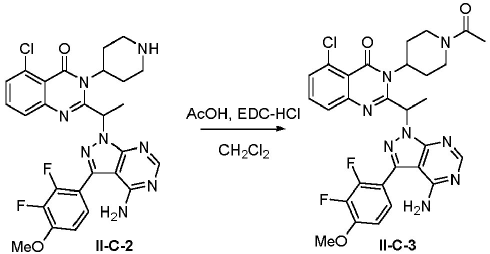

Compound **II-C-2** (0.3mmol) was placed in a 20ml reaction flask with 6ml of dichloromethane, and then mixed with acetic acid (0.3mmol) and 1-ethyl-(3-dimethylaminopropyl)carbodiimide hydrochloride (0.6mmol) to react for about 3h at room temperature. When the reaction was completed as detected by the TLC test, water and dichloromethane were added to extract. The organic phase was separated, dried over anhydrous sodium sulfate and concentrated under reduced pressure. The resulting residue was purified by silica gel column chromatography to obtain 151mg of a white product, compound **II-C-3**, with a yield of 83%. LC-MS (ESI-MS): 609 [M+H]⁺.

### Embodiment 14: Synthesis of Target Compound II-C-4

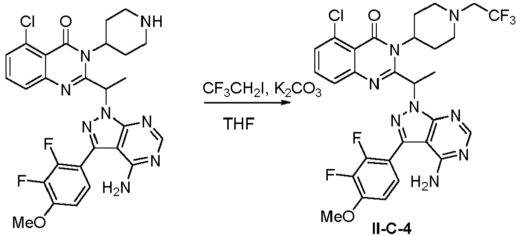

Compound **II-C-2** (0.3mmol) was placed in a 20ml reaction flask with 4ml of tetrahydrofuran, and then mixed with trifluoromethyl iodoethane (0.3mmol) and potassium carbonate (0.6mmol) to react for about 2h at room temperature as the reaction was completed as detected by the TLC test, then quenched with water and extracted with ethyl acetate. The organic phase was dried with anhydrous sodium sulfate and concentrated under reduced pressure. The resulting residue was purified by silica gel column chromatography to obtain 101mg of an off-white solid product, Compound **II-C-4**, with a yield of 52%. LC-MS (ESI-MS): 649 [M+H]⁺.

### Embodiment 15: Synthesis of Target Compound II-C-7

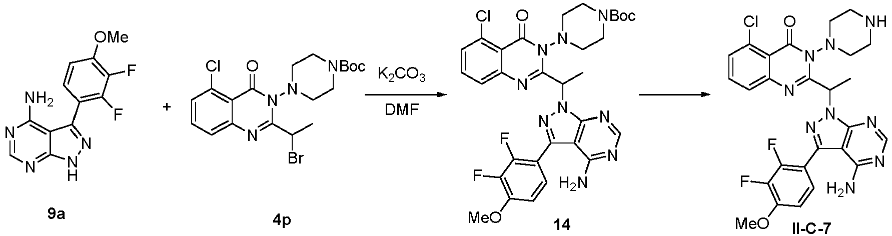

Step 1: 3-(2,3-difluoro-4-methoxyphenyl)-1H-pyrazolo[3,4-d]pyrimidin-4-amine **(9a,** 1.0mmol) was placed in a 20ml reaction flask with 5ml of DMF, and stirred to dissolve at room temperature, then mixed in turn with 4-(2-bromoethyl)-5-chloro-4-oxoquinazolin-3(4H)-yl)piperazine-1-carboxylic acid, tert-butyl ester **(4p,** 1.1mmol) and potassium carbonate (2.0mmol), and heated to 40°C to react for about 15h. When the reaction was completed as detected by the TLC test, ethyl acetate was added to extract, and washed with water. The ethyl acetate layer was separated, dried over anhydrous sodium sulphate and concentrated under reduced pressure. The resulting residue was purified by silica gel column chromatography to obtain 0.42 g of **Intermediate 14** with a yield of 63%.

Step 2: **Intermediate 14** (0.6mmol) was placed in a 20ml reaction flask with 6ml of dichloromethane, then mixed with 1ml of trifluoroacetic acid to react for about 12h at room temperature. When the reaction was basically completed as detected by the TLC test, water and dichloromethane were added to extract. The organic phase was washed with saturated aqueous sodium bicarbonate solution. The dichloromethane layer was separated, dried over anhydrous sodium sulfate and concentrated under reduced pressure. The resulting residue was purified by silica gel column chromatography to obtain 0.28g of the target product, Compound **II**-C-7, with a yield of 82%. ¹H NMR (500 MHz, DMSO) δ 9.11 (s, 3H), 8.32 (s, 1H), 7.78 (t, *J* = 7.9 Hz, 1H), 7.67 (d, *J* = 8.1 Hz, 1H), 7.59 (d, *J* = 8.0 Hz, 1H), 7.20 (t, *J* = 7.9 Hz, 1H), 7.05 (t, *J* = 8.1 Hz, 1H), 6.44 (dd, *J* = 13.3, 6.8 Hz, 1H), 4.04-3.89 (m, 2H), 3.88 (s, 3H), 3.45 (d, *J* = 11.7 Hz, 2H), 3.02 (t, *J* = 11.5 Hz, 1H), 2.85 (d, *J* = 11.3 Hz, 1H), 1.89 (d, *J* = 11.1 Hz, 1H), 1.72 (d, *J* = 6.5 Hz, 3H), 1.42 (t, *J=* 10.8 Hz, 1H). LC-MS (ESI-MS): 567 [M+H]⁺.

### Embodiment 16: Synthesis of Target Compound II-C-8

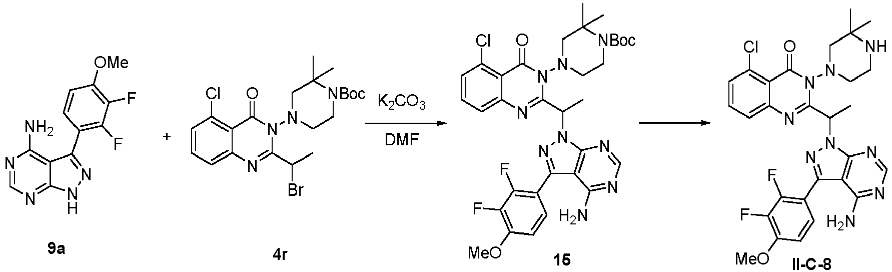

Step 1: 3-(2,3-difluoro-4-methoxyphenyl)-1H-pyrazolo[3,4-d]pyrimidin-4-amine **(9a,** 1.0mmol) was placed in a 20ml reaction flask with 5ml of DMF, and stirred to dissolve at room temperature, then mixed in turn with 4-(2-bromoethyl)-5-chloro-4-oxoquinazolin-3(4H)-yl)-2,2-dimethylpiperazine-1-carboxylic acid, tert-butyl ester **(4r,** 1.1mmol) and potassium carbonate (2.0mmol), and heated to 40°C to react for about 15h. When the reaction was completed as detected by the TLC test, ethyl acetate was added to extract, and washed with water. The ethyl acetate layer was separated, dried over anhydrous sodium sulphate and concentrated under reduced pressure. The resulting residue was purified by silica gel column chromatography to obtain 0.38 g of **Intermediate 15** with a yield of 55%.

Step 2: **Intermediate 15** (0.6mmol) was placed in a 20ml reaction flask with 6ml of dichloromethane, and then mixed with 1ml of trifluoroacetic acid to react for about 12h at room temperature. When the reaction was basically completed as detected by the TLC test, water and dichloromethane were added to extract, and the organic phase was washed with saturated aqueous sodium bicarbonate solution. The dichloromethane layer was separated, dried over anhydrous sodium sulfate and concentrated under reduced pressure. The resulting residue was purified by silica gel column chromatography to obtain 0.28g of the target product, Compound **II-C-8,** with a yield of 79%. LC-MS (ESI-MS): 596 [M+H]⁺.

### Embodiment 17: Synthesis of Target Compound II-C-9

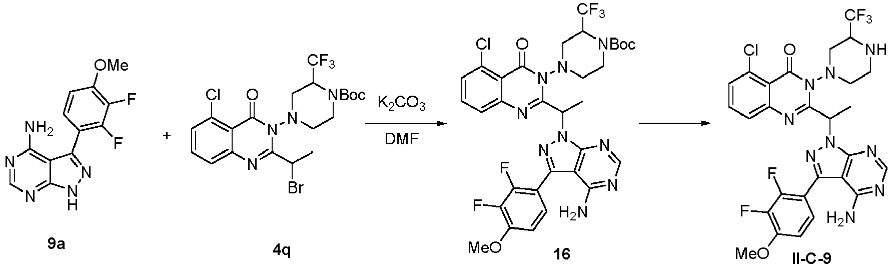

Step 1: 3-(2,3-difluoro-4-methoxyphenyl)-1H-pyrazolo[3,4-d]pyrimidin-4-amine (9a, 1.0mmol) was placed in a 20ml reaction flask with 5ml of DMF, and stirred to dissolve at room temperature, then mixed in turn with 4-(2-bromoethyl)-5-chloro-4-oxoquinazolin-3(4H)-yl)-2-(trifluoromethyl)piperazine-1-carboxyli c acid, tert-butyl ester **(4q,** 1.1mmol) and potassium carbonate (2.0mmol), and heated to 40°C to react for about 15h. When the reaction was complete as detected by the TLC test, ethyl acetate was added to extracted, and washed with water. The ethyl acetate layer was separated, dried with anhydrous sodium sulphate and concentrated under reduced pressure. The resulting residue was purified by silica gel column chromatography to obtain 0.36 g of **Intermediate 16,** with a yield of 49%.

Ste 2: **Intermediate 16** (0.6mmol) was placed in a 20ml reaction flask with 6ml of dichloromethane, and then mixed with 1ml of trifluoroacetic acid to react for about 12h at room temperature. When the reaction was basically completed as detected by the TLC test, water and dichloromethane were added to extract. The organic phase was washed with saturated aqueous sodium bicarbonate solution. The dichloromethane layer was separated, dried with anhydrous sodium sulfate and concentrated under reduced pressure. The resulting residue was purified by silica gel column chromatography to obtain 0.28g of the target product, Compound **II-C-9,** with a yield of 75%. LC-MS (ESI-MS): 636 [M+H]⁺.

### Embodiment 18: Synthesis of Target Compound II-C-10

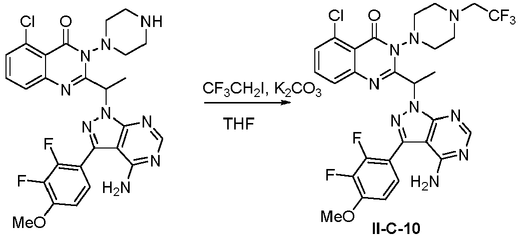

Compound **II-C-7** (0.5mmol) was placed in a 20ml reaction flask with 5ml of tetrahydrofuran, and then mixed with trifluoromethyl iodoethane (0.5mmol) and potassium carbonate (1.0mmol) to react for about 2h at room temperature and the reaction was completed as detected by the TLC test, then quench with water and extract with ethyl acetate The organic phase was dried over anhydrous sodium sulfate and concentrated under reduced pressure. The resulting residue was purified by silica gel column chromatography to obtain 172mg of off-white solid, compound **II-C-10**, with a yield of 53%. LC-MS (ESI-MS): 650 [M+H]⁺.

### Embodiment 19: synthesis of target compound II-C-11

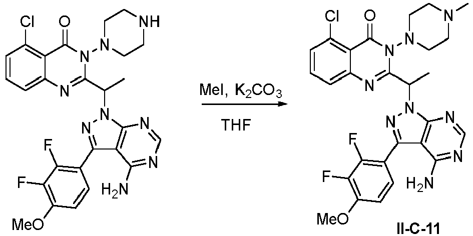

Place compound **II-C-7** (0.5mmol) and 5ml of tetrahydrofuran, mix with iodomethane (0.45mmol) and potassium carbonate (1.0mmol) in a 20ml reaction flask, react for about 2h at room temperature, quench with water and extract with ethyl acetate, dry the organic phase with anhydrous sodium sulfate and concentrate under reduced pressure, purify the resulting residue by silica gel column chromatography to obtain 125mg of white-like solid, compound **II-C-11** with a yield of 43%. ¹H NMR (500 MHz, CDCl₃) δ 8.44 (s, 1H), 7.60 (d, *J* = 7.9 Hz, 1H), 7.55 (t, *J* = 7.8 Hz, 1H), 7.43 (d, *J=* 7.5 Hz, 1H), 7.19 (t, *J=* 7.6 Hz, 1H), 6.84 (t, *J=* 7.8 Hz, 1H), 6.50 (dd, *J=* 13.2, 6.6 Hz, 1H), 5.51 (brs, 2H), 4.23 (t, *J=* 10.4 Hz, 1H), 4.06 (t, *J=* 10.1 Hz, 1H), 3.94 (s, 3H), 2.84 (dd, *J=* 33.7, 10.2 Hz, 2H), 2.40 (d, *J* = 11.5 Hz, 2H), 2.17 (s, 3H), 2.08 (d, *J=* 9.6 Hz, 2H), 1.94 (d, *J=* 6.6 Hz, 3H). LC-MS (ESI-MS): 582 [M+H]⁺.

### Embodiment 20: synthesis of target compound II-C-12

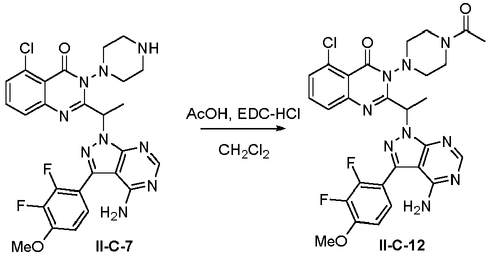

Place compound **II-C-7** (0.3mmol) and 6ml of dichloromethane in a 20ml reaction flask, mix with acetic acid (0.3mmol) and 1-ethyl-(3-dimethylaminopropyl)carbodiimide hydrochloride (0.6mmol), react for about 3h at room temperature, complete the TLC test, extract with water and dichloromethane, separate the organic phase to dry with anhydrous sodium sulfate and concentrate under reduced pressure, purify the resulting residue by silica gel column chromatography to obtain 155mg of white product, compound **II-C-12** with a yield of 85%. LC-MS (ESI-MS): 610 [M+H]⁺.

### Embodiment 21: synthesis of compound II-C-13

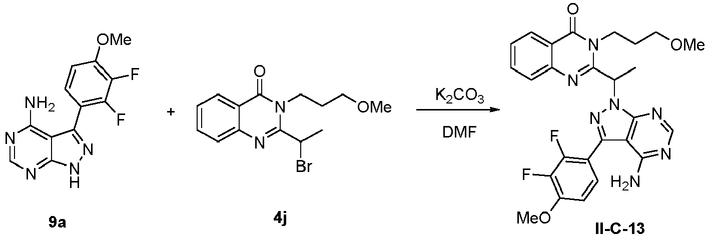

place 3-(2,3-difluoro-4-methoxyphenyl)-1H-pyrazolo[3,4-d]pyrimidin-4-amine (9a, 1.0mmol) and 5ml of DMF in a 20ml reaction flask, stir to dissolve at room temperature, then add in turn 2-(1-bromoethyl)-3-(3-methoxypropyl)quinazolin-4(3H)-one (4j, 1.1mmol) and potassium carbonate (2.0mmol), heat to 40°C to react for about 15h, complete TLC test, extract with ethyl acetate, wash with water, separate the ethyl acetate layer, dry with anhydrous sodium sulphate and concentrate under reduced pressure, purify the resulting residue through silica gel column chromatography to obtain 0.35g of solid product, compound **II-C-13** with a yield of 67%. LC-MS (ESI-MS): 522 [M+H]⁺.

### Embodiment 22: synthesis of compound II-C-14

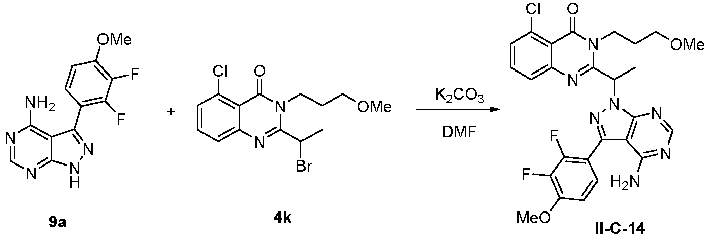

place 3-(2,3-difluoro-4-methoxyphenyl)-1H-pyrazolo[3,4-d]pyrimidin-4-amine (9a, 1.0mmol) and 5ml of DMF in a 20ml reaction flask, stir to dissolve at room temperature, then add in turn 2-(1-bromoethyl)-5-chloro-3-(3-methoxypropyl)quinazolin-4(3H)-one (4k, 1.1mmol) and potassium carbonate (2.0mmol), heat to 40°C to react for about 15h, complete TLC test, extract with ethyl acetate, wash with water, separate the ethyl acetate layer, dry with anhydrous sodium sulphate and concentrate under reduced pressure, purify the resulting residue through silica gel column chromatography to obtain 0.36g of solid product, compound **II-C-14** with a yield of 65%. ¹H NMR (500 MHz, DMSO) δ 8.30 (s, 1H), 7.72 (t, *J=* 7.5 Hz, 1H), 7.57 (dd, *J* = 28.8, 7.5 Hz, 2H), 7.18 (s, 1H), 7.08 (d, *J* = 7.2 Hz, 1H), 6.40 (d, *J* = 5.7 Hz, 1H), 4.06 - 3.75 (m, 2H), 3.84 (s, 3H), 3.05 (s, 3H), 2.96 (brs, 2H), 1.85 - 1.76 (m, 5H). LC-MS (ESI-MS): 556 [M+H]⁺.

### Embodiment 23: synthesis of compound II-C-15

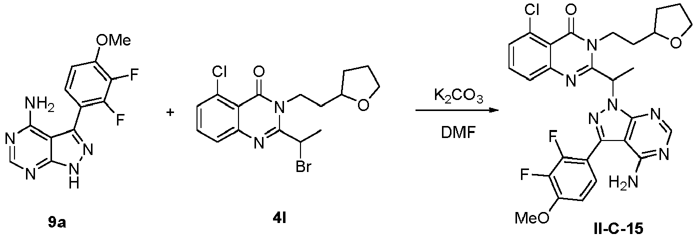

place 3-(2,3-difluoro-4-methoxyphenyl)-1H-pyrazolo[3,4-d]pyrimidin-4-amine (9a, 1.0mmol) and 5ml of DMF in a 20ml reaction flask, stir to dissolve at room temperature, then add in turn the 2-(1-bromoethyl)-5-chlorine-3-(2-(tetrahydrofuran-2-yl)ethyl)quinazolin-4(3H)-one (41, 1.1mmol) and potassium carbonate (2.0mmol), heat to 40°C to react for about 15h, complete TLC test, extract with ethyl acetate, wash with water, separate the ethyl acetate layer, dry with anhydrous sodium sulphate and concentrate under reduced pressure, purify the resulting residue through silica gel column chromatography to obtain 0.30g of solid product, compound **II-C-15** with a yield of 52%. LC-MS (ESI-MS): 582 [M+H]⁺.

### Embodiment 24: Synthesis of Target Compounds II-A-1a and II-A-1b

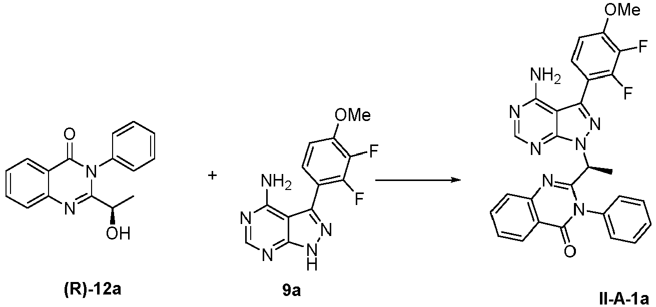

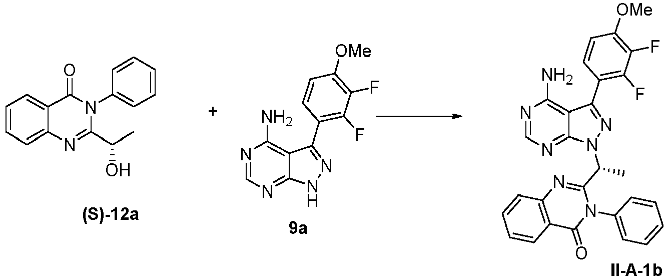

Place **intermediate 9a** (10mmol), **(R)-12a** or **(S)-12a** (20mmol), triphenylphosphine (5.3g, 20mmol) and tetrahydrofuran 50ml in a reaction flask, cool to 0°C, drip diisopropyl azodicarboxylate (3.9ml, 20mmol); heat to room temperature after dripping to react for about 6h so TLC test is completed, concentrate under reduced pressure to remove most of the tetrahydrofuran, and purify the residue by silica gel column chromatography to obtain a white solid product.

**Target compound II-A-1a:** 3.1g, 58% yield; Chiral assay ee%: 97.8%, chiral-HPLC being performed using a normal-phase CHIRALCEL OD-H column with a retention time of 13.86min. LC-MS (ESI-MS): 526 [M+H]⁺.

**Target compound II-A-1b:** 2.9g, 55% yield; Chiral assay ee%: 98.2%, chiral-HPLC being performed using a normal-phase CHIRALCEL OD-H column with a retention time of 17.24min. LC-MS (ESI-MS): 526 [M+H]⁺.

### Embodiment 25: synthesis of target compounds II-A-2a and II-A-2b

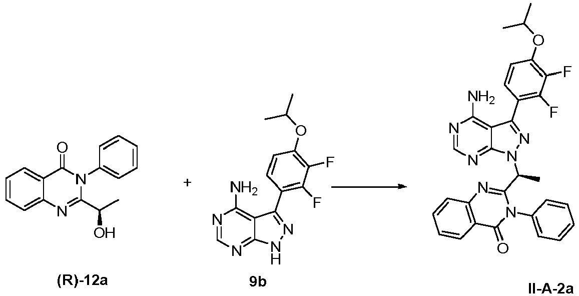

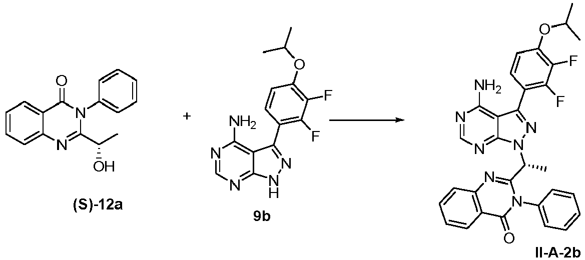

Place **intermediate 9b** (10mmol), **(R)-12a** or **(S)-12a** (20mmol), triphenylphosphine (5.3g, 20mmol) and tetrahydrofuran 50ml in a reaction flask, cool to 0°C, drip diisopropyl azodicarboxylate (3.9ml, 20mmol); heat to room temperature after dripping to react for about 6h so TLC test is completed, concentrate under reduced pressure to remove most of the tetrahydrofuran, and purify the residue by silica gel column chromatography to obtain a white solid product.

**Target compound II-A-2a:** 3.1g, 56% yield; Chiral assay ee%: 98.2%, chiral-HPLC being performed using a normal-phase CHIRALCEL OD-H column with a retention time of 14.10min. LC-MS (ESI-MS): 554 [M+H]⁺.

**Target compound II-A-2b:** 3.0g, 54% yield; Chiral assay ee%: 97.8%, chiral-HPLC being performed using a normal-phase CHIRALCEL OD-H column with a retention time of 16.90min. LC-MS (ESI-MS): 554 [M+H]⁺.

### Embodiment 26: Synthesis of Target Compounds II-A-7a and II-A-7b

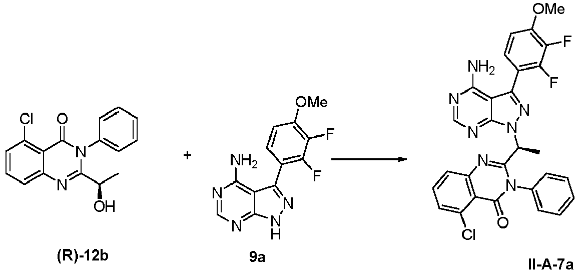

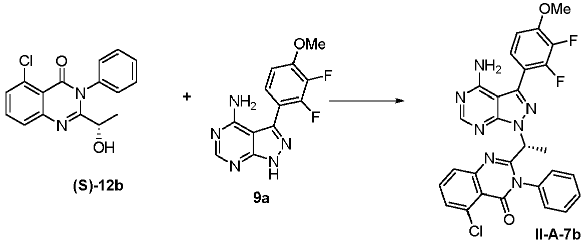

Place **intermediate 9a** (10mmol), **(R)-12b** or **(S)-12b** (20mmol), triphenylphosphine (5.3g, 20mmol) and tetrahydrofuran 50ml in a reaction flask, cool to 0°C, drip diisopropyl azodicarboxylate (3.9ml, 20mmol); heat to room temperature after dripping to react for about 6h so TLC test is completed, concentrate under reduced pressure to remove most of the tetrahydrofuran, and purify the residue by silica gel column chromatography to obtain a white solid product.

**Target compound II-A-7a:** 3.1g, 55% yield; Chiral assay ee%: 98.6%, chiral-HPLC being performed using a normal-phase CHIRALCEL OD-H column with a retention time of 12.09min. LC-MS (ESI-MS): 560 [M+H]⁺.

**Target compound II-A-7b:** 3.0g, 54% yield; Chiral assay ee%: 98.1%, chiral-HPLC being performed using a normal-phase CHIRALCEL OD-H column with a retention time of 19.90min. LC-MS (ESI-MS): 560 [M+H]⁺.

### Embodiment 27: Synthesis of Target Compounds II-A-8a and II-A-8b

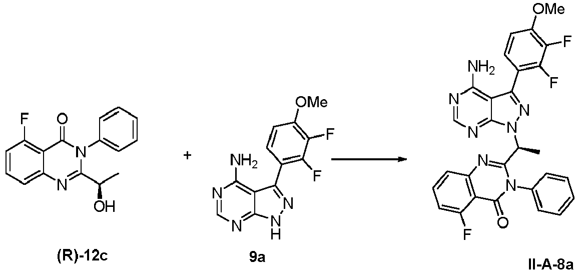

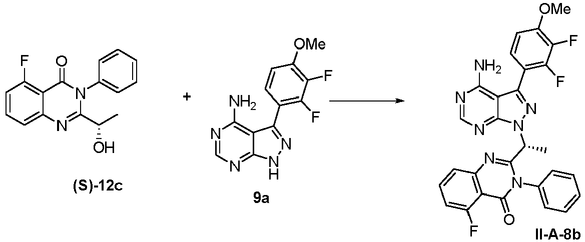

**Intermediate 9a** (10mmol), **(R)-12c** or **(S)-12c** (20mmol) and triphenylphosphine (5.3g, 20mmol) were placed in a reaction flask with 50ml of tetrahydrofuran, cooled to 0°C, dripped with diisopropyl azodicarboxylate (3.9ml, 20mmol) and then heated to room temperature after dripping to react for about 6h till the reaction was completed as detected by the TLC test. Most of the tetrahydrofuran was then removed by concentration under reduced pressure, and the residue was purified by silica gel column chromatography to obtain a white solid product.

**Target compound II-A-8a:** 3.1g, 57% yield; chiral assay ee%: 98.7%, chiral-HPLC being performed using a normal-phase CHIRALCEL OD-H column with a retention time of 12.75min. LC-MS (ESI-MS): 544 [M+H]⁺.

**Target compound II-A-8b:** 3.0g, 55% yield; chiral assay ee%: 98.2%, chiral-HPLC being performed using a normal-phase CHIRALCEL OD-H column with a retention time of 18.99min. LC-MS (ESI-MS): 544 [M+H]⁺.

### Embodiment 28: Synthesis of Target Compounds II-B-2a and II-B-2b

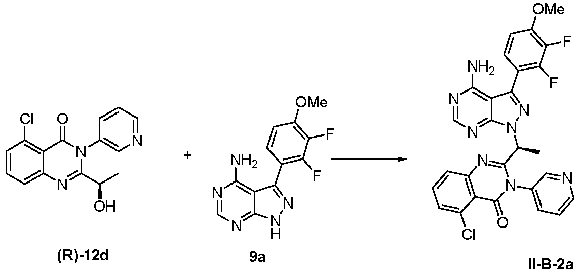

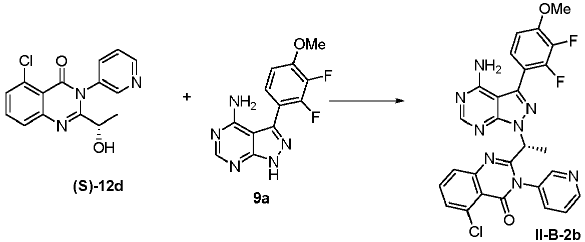

**Intermediate 9a** (10mmol), **(R)-12d** or **(S)-12d** (20mmol) and triphenylphosphine (5.3g, 20mmol) were placed in a reaction flask with 50ml of tetrahydrofuran, cooled to 0°C, drippd with diisopropyl azodicarboxylate (3.9ml, 20mmol) and then heated to room temperature after dripping to react for about 6h till the reaction was completed as detected by the TLC test. Most of the tetrahydrofuran was removed by concentration under reduced pressure, and the residue was purified by silica gel column chromatography to obtain a white solid product.

**Target compound II-B-2a:** 2.7g, 48% yield; chiral assay ee%: 98.0%, chiral-HPLC being performed using a normal-phase CHIRALCEL OD-H column with a retention time of 13.42min. LC-MS (ESI-MS): 561 [M+H]⁺.

**Target compound II-B-2b:** 2.6g, 46% yield; chiral assay ee%: 97.8%, chiral-HPLC being performed using a normal-phase CHIRALCEL OD-H column with a retention time of 23.15min. LC-MS (ESI-MS): 561 [M+H]⁺.

### Embodiment 29: Synthesis of Compounds II-D-1~II-D-4

Compound **II-A-1a** (231mg, 0.4 mmol) was placed in a flask, mixed with 6ml of isopropanol, stirred, mixed with an inorganic or organic acid (0.44mmol), heated to reflux, reacted for 1h with stirring, slowly cooled to room temperature, desolvated and filtered. The solid was collected, dried in vacuum at room temperature to obtain the solid product.

**Compound II-D-1:** the acid added was hydrochloric acid (0.44mmol). The final product **II-D-1** was a white solid, 241mg with 98.1% yield. LC-MS (ESI-MS): 526 [M+H]⁺.

**Compound II-D-2:** the acid added was sulfuric acid (0.44mmol). The final product **II-D-2** was a white solid, 250mg with 99.5% yield. LC-MS (ESI-MS): 526 [M+H]⁺.

**Compound II-D-3:** the acid added was toluenesulfonic acid monohydrate (84mg, 0.44mmol). The final product **II-D-3** was a white solid, 273mg with 91% yield. ¹H NMR (400 MHz, DMSO) δ 8.17 (s, 1H), 7.84 (t, *J* = 8.0 Hz, 1H), 7.76 (dd, *J* = 8.2, 1.1 Hz, 1H), 7.65 (dd, *J* = 12.9, 5.1 Hz, 2H), 7.50-7.43 (m, 3H), 7.37-7.25 (m, 1H), 7.22 - 7.04 (m, 4H), 6.93 (dd, *J* = 11.1, 4.3 Hz, 1H), 6.44 (d, *J=* 8.2 Hz, 1H), 5.99 (q, *J=* 6.6 Hz, 1H), 3.93 (s, 3H), 2.29 (s, 3H), 1.78 (d, *J=* 6.7 Hz, 3H)_{∘} LC-MS (ESI-MS): 526[M+H]⁺.

**Compound II-D-4:** the acid added was methanesulfonic acid (29µl, 0.44mmol). The final product **II-D-4** was a white solid, 260mg with 96.5% yield. LC-MS (ESI-MS): 526 [M+H]⁺.

### Embodiment 30: Synthesis of Compounds II-D-5~II-D-17

Compound **II-A-7a** (0.4 mmol) was placed in a flask, mixed 6ml of isopropanol, stirred, mixed with an inorganic or organic acid (0.44mmol), heated to reflux, reacted for 1h with stirring, slowly cooled to room temperature, desolvated and filtered. The solid was collected and dried in vacuum at room temperature to obtain the solid product.

**Compound II-D-5:** the acid added was hydrochloric acid (0.44mmol). The final product **II-D-5** was a white solid, 233mg with 99.5% yield. ¹H NMR (500 MHz, DMSO) δ 8.04 (s, 1H), 7.84 (t, *J* = 8.0 Hz, 1H), 7.75 (d, *J* = 8.2 Hz, 1H), 7.64 (d, *J* = 7.9 Hz, 2H), 7.46 (t, *J* = 7.7 Hz, 1H), 7.29 (t, *J=* 7.7 Hz, 1H), 7.20 - 7.05 (m, 2H), 6.87 (t, *J=* 7.7 Hz, 1H), 6.37 (d, *J* = 8.0 Hz, 1H), 5.95 (q, *J=* 6.6 Hz, 1H), 3.92 (s, 3H), 1.75 (d, *J=* 6.6 Hz, 3H). LC-MS (ESI-MS): 560 [M+H]⁺.

**Compound II-D-6:** the acid added was sulfuric acid (0.44mmol). The final product **II-D-6** was an off-white solid, 244mg with 99.5% yield. ¹H NMR (500 MHz, DMSO) δ 8.06 (s, 1H), 7.84 (t, *J* = 8.0 Hz, 1H), 7.75 (d, *J* = 8.1 Hz, 1H), 7.64 (d, *J* = 7.8 Hz, 2H), 7.46 (t, *J* = 7.7 Hz, 1H), 7.30 (t, *J=* 7.5 Hz, 1H), 7.22-7.06 (m, 2H), 6.88 (t, *J=* 7.7 Hz, 1H), 6.38 (d, *J=* 8.1 Hz, 1H), 5.96 (q, *J* = 6.6 Hz, 1H), 3.92 (s, 3H), 1.75 (d, *J* = 6.6 Hz, 3H). LC-MS (ESI-MS): 560 [M+H]⁺.

**Compound II-D-7:** the acid added was phosphoric acid (0.44mmol). The final product **II-D-7** was an off-white solid, 207mg with 78.6% yield. ¹H NMR (500 MHz, DMSO) δ 7.88 (s, 1H), 7.83 (t, *J=* 8.0 Hz, 1H), 7.75 (d, *J=* 8.1 Hz, 1H), 7.61 (dd, *J=* 13.8, 7.9 Hz, 2H), 7.43 (t, *J* = 7.6 Hz, 1H), 7.24 (t, *J=* 7.6 Hz, 1H), 7.10 (dd, *J* = 12.8, 7.5 Hz, 2H), 6.81 (t, *J=* 7.7 Hz, 1H), 6.31 (d, *J=* 7.8 Hz, 1H), 5.90 (q, *J=* 6.6 Hz, 1H), 3.91 (s, 3H), 1.72 (d, *J=* 6.6 Hz, 3H). LC-MS (ESI-MS): 560 [M+H]⁺.

**Compound II-D-8:** the acid added was toluenesulfonic acid monohydrate (84mg, 0.44mmol). The final product **II-D-8** was an off-white solid, 268mg with 91.5% yield. ¹H NMR (400 MHz, DMSO) δ 8.20 - 8.05 (m, 2H), 7.97-7.87 (m, 1H), 7.82 (d, *J=* 7.9 Hz, 1H), 7.68 (d, *J* = 8.0 Hz, 1H), 7.65-7.56 (m, 1H), 7.54-7.39 (m, 3H), 7.34-7.24 (m, 1H), 7.20-7.01 (m, 3H), 6.93 (dd, *J* = 11.1, 4.3 Hz, 1H), 6.42 (d, *J* = 8.0 Hz, 1H), 6.05 (q, *J* = 6.6 Hz, 1H), 3.92 (s, 3H), 2.29 (s, 3H), 1.80 (d, *J=* 6.6 Hz, 3H). LC-MS (ESI-MS): 560 [M+H]⁺.

**Compound II-D-9:** the acid added was methanesulfonic acid (29µl, 0.44mmol). The final product **II-D-9** was an off-white solid, 262mg with 99.5% yield. ¹H NMR (500 MHz, DMSO) δ 8.14 (s, 1H), 7.84 (t, *J=* 8.0 Hz, 1H), 7.75 (d, *J=* 8.1 Hz, 1H), 7.65 (t, *J=* 7.7 Hz, 2H), 7.47 (t, *J* = 7.6 Hz, 1H), 7.31 (t, *J* = 8.1 Hz, 1H), 7.15 (dt, *J* = 16.3, 7.8 Hz, 2H), 6.92 (t, *J* = 7.5 Hz, 1H), 6.41 (d, *J* = 7.8 Hz, 1H), 5.97 (t, *J* = 6.6 Hz, 1H), 3.93 (s, 3H), 2.36 (s, 3H), 1.77 (d, *J* = 6.6 Hz, 3H). LC-MS (ESI-MS): 560 [M+H]⁺.

**Compound II-D-10:** the acid added was citric acid (85mg, 0.44mmol). The final product **II-D-10** was an off-white solid, 206mg with 68.5% yield. LC-MS (ESI-MS): 560 [M+H]⁺.

**Compound II-D-11:** the acid added was succinic acid (52mg, 0.44mmol). The final product **II-D-11** was an off-white solid, 198mg with 73.1% yield. LC-MS (ESI-MS): 560 [M+H]⁺.

**Compound II-D-12:** the acid added was L-malic acid (66mg, 0.44mmol). The final product **II-D-12** was an off-white solid, 213mg with 76.7% yield. LC-MS (ESI-MS): 560 [M+H]⁺.

**Compound II-D-13:** the acid added was D-malic acid (59mg, 0.44mmol). The final product **II-D-13** was an off-white solid, 201mg with 72.4% yield. LC-MS (ESI-MS): 560 [M+H]⁺.

**Compound II-D-14:** the acid added was L-tartaric acid (66mg, 0.44mmol). The final product **II-D-14** was an off-white solid, 209mg with 73.6% yield. LC-MS (ESI-MS): 560 [M+H]⁺.

**Compound II-D-15:** the acid added was benzoic acid (54mg, 0.44mmol). The final product **II-D-15** was an off-white solid, 202mg with 74% yield. LC-MS (ESI-MS): 560[M+H]⁺.

**Compound II-D-16:** the acid added was maleic acid (51mg, 0.44mmol). The final product **II-D-16** was an off-white solid, 220mg with 81.5% yield. LC-MS (ESI-MS): 560[M+H]⁺.

**Compound II-D-17:** the acid added was oxalic acid (55mg, 0.44mmol). The final product **II-D-17** was an off-white solid, 216mg with 83.1% yield. LC-MS (ESI-MS): 560[M+H]⁺.

### Embodiment 31: Synthesis of Compounds A and B

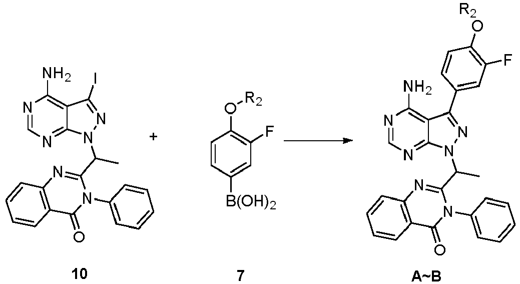

Compound **10** (2.0mmol), compound **7** (3.0mmol), potassium carbonate (4.0mmol), tetrakis(triphenylphosphine)palladium(0) (0.02mmol) were placed separately in a 50ml reaction flask with the solvent, 9ml of dioxane and 3ml of water, heated to 100°C with stirring to react for about 14h, and brought to room temperature when the reaction was completed as detected by the TLC test. The reaction liquid was filtered with diatomite and rinsed with ethyl acetate. The filtrate was extracted with 20ml of water and 50ml of ethyl acetate. The ethyl acetate layer was separated, dried over anhydrous sodium sulfate and concentrated under reduced pressure. The crude product was purified by silica gel column chromatography to obtain a white solid product.

**Compound A (R₂=CH(CH₃)₂):** 0.67g, 63% yield. ¹H NMR (500 MHz, CDCl₃) δ 8.27 (dt, *J* = 11.9, 5.8 Hz, 1H), 8.05 (s, 1H), 7.80 (ddd, *J* = 11.3, 9.6, 4.4 Hz, 2H), 7.55 - 7.49 (m, 1H), 7.47 (td, *J* = 7.7, 1.1 Hz, 1H), 7.37 (dd, *J* = 11.5, 2.0 Hz, 1H), 7.31 (d, *J* = 7.6 Hz, 2H), 7.16 (t, *J* = 7.5 Hz, 1H), 7.07 (t, *J* = 8.4 Hz, 1H), 6.89 (td, *J* = 7.8, 1.1 Hz, 1H), 6.50 (d, *J* = 8.3 Hz, 1H), 6.00 (q, *J* = 6.7 Hz, 1H), 5.55 (brs, 2H), 4.60 (dq, *J* = 12.1, 6.1 Hz, 1H), 1.91 (t, *J* = 8.9 Hz, 3H), 1.38 (dt, *J* = 7.0, 3.5 Hz, 6H). LC-MS (ESI-MS): 536 [M+H]⁺.

**Compound B (R₂=cyclopropyl):** 0.49g, 46% yield. ¹H NMR (500 MHz, CDCl₃) δ 8.28 (d, *J* = 7.9 Hz, 1H), 8.06 (s, 1H), 7.88 - 7.76 (m, 2H), 7.50 (dt, *J* = 15.1, 7.2 Hz, 2H), 7.36 (ddd, *J* = 25.8, 18.5, 8.0 Hz, 4H), 7.17 (t, *J* = 7.4 Hz, 1H), 6.89 (t, *J* = 7.6 Hz, 1H), 6.50 (d, *J* = 7.8 Hz, 1H), 6.00 (q, *J* = 6.7 Hz, 1H), 5.51 (s, 2H), 3.91 - 3.81 (m, 1H), 1.92 (d, *J* = 6.7 Hz, 3H), 0.92 - 0.78 (m, 4H). LC-MS (ESI-MS): 534 [M+H]⁺.

### Embodiment 32: Synthesis of Compound C

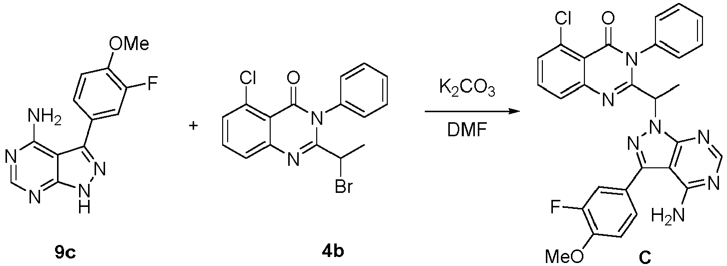

**Intermediate 9c** (1.0mmol) was placed in a 20ml reaction flask with 5ml of DMF, stirred to dissolve at room temperature, then mixed in turn with **intermediate 4b** (1.1mmol) and potassium carbonate (2.0mmol), heated to 40°C to react for about 15h. When the reaction was completed as detected by the TLC test, ethyl acetate was added to extract, and washed with water. The ethyl acetate layer was separated, dried over anhydrous sodium sulphate and concentrated under reduced pressure. The resulting residue was purified by silica gel column chromatography to obtain 0.42g of solid product C with a yield of 78%. ¹H NMR (500 MHz, DMSO) δ 7.92 (brs, 1H), 7.89 - 7.80 (m, 1H), 7.77 (d, *J* = 7.6 Hz, 1H), 7.62 (dd, *J* = 16.6, 7.6 Hz, 2H), 7.48 - 7.40 (m, 2H), 7.37 (d, *J* = 8.1 Hz, 1H), 7.30 (d, *J* = 8.5 Hz, 1H), 7.11 (d, *J* = 6.9 Hz, 1H), 6.86 (d, *J* = 7.2 Hz, 1H), 6.34 (d, *J* = 6.8 Hz, 1H), 5.91 (d, *J* = 6.3 Hz, 1H), 3.89 (s, 3H), 1.74 (d, *J* = 6.1 Hz, 3H). LC-MS (ESI-MS): 544 [M+H]⁺.

### Embodiment 33: Synthesis of Compound D

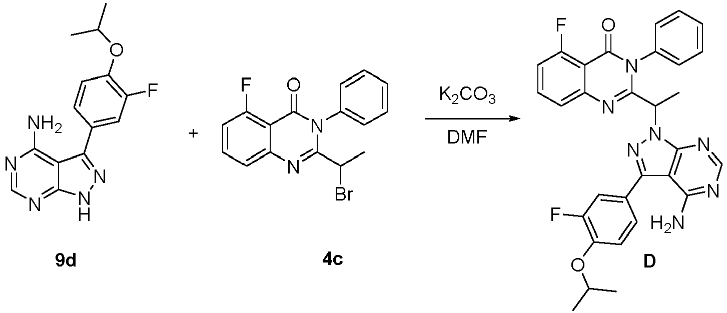

**Intermediate 9d** (1.0mmol) was placed in a 20ml reaction flask with 5ml of DMF, stirred to dissolve at room temperature, then mixed in turn with **Intermediate 4c** (1.1mmol) and potassium carbonate (2.0mmol), heated to 40°C to react for about 15h. When the reaction was completed as detected by the TLC test, ethyl acetate was added, and washed with water. The ethyl acetate layer was separated, dried over anhydrous sodium sulphate and concentrated under reduced pressure. The resulting residue was purified by silica gel column chromatography to obtain 0.32g of solid product D with a yield of 58%. ¹H NMR (500 MHz, CDCl₃) δ 8.04 (s, 1H), 7.71 (td, J = 8.2, 5.4 Hz, 1H), 7.61 (d, J = 8.2 Hz, 1H), 7.46 (t, J = 7.7 Hz, 1H), 7.37 (dd, J = 11.4, 1.9 Hz, 1H), 7.30 (dd, J = 13.9, 8.5 Hz, 2H), 7.15 (dd, J = 15.6, 8.0 Hz, 2H), 7.07 (dd, J = 17.1, 8.8 Hz, 1H), 6.88 (t, J = 7.7 Hz, 1H), 6.49 (d, J = 7.9 Hz, 1H), 5.96 (q, J = 6.7 Hz, 1H), 5.56 (brs, 2H), 4.60 (dq, J = 12.0, 6.0 Hz, 1H), 1.89 (d, J = 6.8 Hz, 3H), 1.39 (dd, J = 6.0, 2.5 Hz, 6H). LC-MS (ESI-MS): 554 [M+H]⁺.

### Embodiment 34: CK1ε kinase activity assay

The *in-vitro* assay of inhibitory activity of the compound herein against **CK1ε** kinase was:
(1) preparation of 1 × kinase basic buffer and stop buffer:
   - 1 × kinase basic buffer: 50 mM HEPES, pH 7.5; and 0.0015% Brij-35;
   - stop buffer: 100 mM HEPES, pH 7.5; 0.015% Brij-35; 0.2% Coating Reagent #3; and 50 mM EDTA.
(2) preparation of the test compound:
   - a 50× ("n×" represents dilution factor, same hereafter) stock solution of the compound with DMSO (10mM, DMSO) to obtain stock solution I for use; 100µl of stock solution I was placed in a 96-well plate, each compound sample was diluted by 5-fold serial dilution till 10 concentrations was obtained while ensuring 10µl of drug solution in each well; meanwhile, 100µl of DMSO was used as a blank control group and a negative control group without enzyme substrate;
   - a separate 96-well plate was prepared, and to 10µl of the above compound,
      90µl of 1× kinase foundation buffer was added, mixed well for 10min to obtain a mixture.
(3) preparation of the test plate:
   - 5µl of the above mixture prepared in the 96-well plate was placed in a 384-well plate, each compound in duplicate;
   - a 2.5× kinase solution was prepared with corresponding 1 × kinase basic buffer added;
   - a 2.5 × polypeptide solution was prepared, and 1× kinase basic buffer was added with FAM-labelled polypeptide and ATP;
   - 10µl of 2.5× kinase solution was placed in the test 384-well plate and allowed to stand for 10min at room temperature, mixed with 10µl of 2.5 × polypeptide solution, and reacted for 1h at 28°C and then mixed with 25µl of stop buffer.
   - the plate was read with Caliper program and IC₅₀ values were obtained for corresponding compounds in kinase inhibition based on the obtained data.

**Table 1 Inhibitory activities of partial compounds against CK1ε kinase**

| **Compound** | **Structure** | **CK1ε (IC₅₀, nM)** | **Compo und** | **Structure** | **CK1ε (IC₅₀, nM)** |
|---|---|---|---|---|---|
| **CUX-031723** | 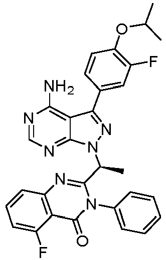 | 9362^{∗} | **II-A-8** | 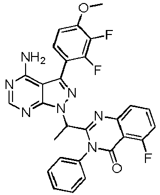 | 118 |
| **CUX-03166** | 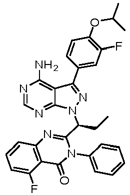 | 81180^{∗} | **II-A-8a** | 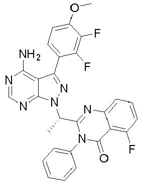 | 60 |
| **II-A-1** | 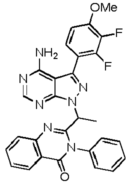 | 164 | **II-A-9** | 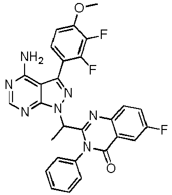 | 100 |
| **II-A-1a** | 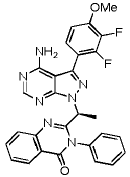 | 108 | **II-A-12** | 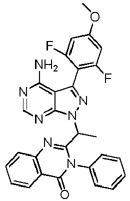 | 202 |
| **II-A-1b** | 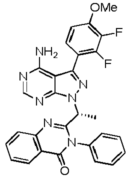 | 484 | **II-A-16** | 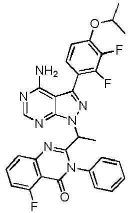 | 100 |
| **II-A-2** | 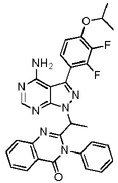 | 145 | **II-B-2** | 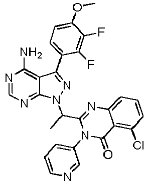 | 372 |
| **II-A-2a** | 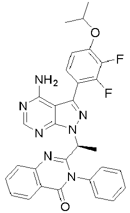 | 88 | **II-B-2a** | 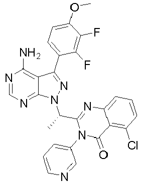 | 202 |
| **II-A-2b** | 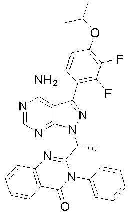 | 450 | **II-B-3** | 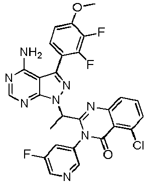 | 89 |
| **II-A-3** | 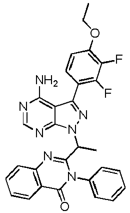 | 232 | **II-C-5** | 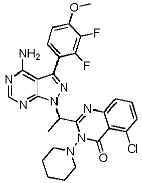 | 299 |
| **II-A-4** | 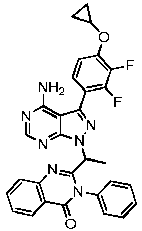 | 234 | **A** | 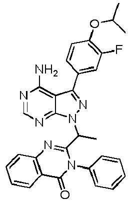 | 1732 |
| **II-A-5** | 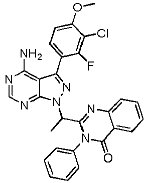 | 386 | **B** | 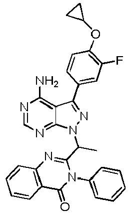 | 2714 |
| **II-A-7** | 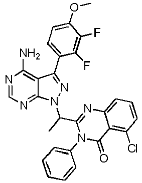 | 97 | **C** | 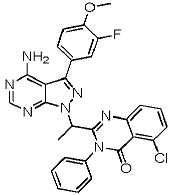 | 701 |
| **II-A-7a** | 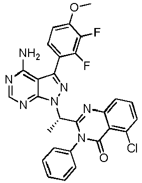 | 45 | **D** | 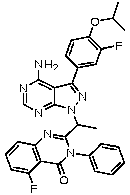 | 1290 |

| | | | | | |
|---|---|---|---|---|---|
| ^{∗}: Data from literature values in: WO2017079558A1. | | | | | |

Data in Table 1 demonstrates that all compounds obtained in the present invention have shown remarkable inhibitory activities against **CK1ε** kinase, which are all significantly better than that of compound **CUX-031723** (International Publication No.: WO2017079558A1, IC₅₀ of **CK1ε** is 9.362µM) and compounds **A~D** (4-amino-disubstituted phenyl-1H-pyrazolo[3,4-d]pyrimidin-1-yl analogues). Wherein, the IC₅₀ values for **CK1ε** inhibition by chiral compounds **II-A-1a**, **II-A-2a, II-A-7a, II-A-8a,** and **II-B-2a** are as high as 108, 88, 45, 60, and 202nM, respectively. The inhibitory activities against **CK1ε** of **compounds II-A-2**, **II-A-4**, **and II-A-7 showed a 12-, 12-, and 7.2-fold increase, respectively, compared to compounds A, B, and C, respectively,** and the inhibitory activity against **CK1ε** of **II-A-16** shows a 12.9- and 93.6-fold increase, respectively, compared to compounds **D and CUX-031773,** respectively, indicating that the 2-position fluorine atom on the substituent benzene ring in the pyrazolo[3,4-d]pyrimidin-1-yl portion of the compounds of the present invention is very important in improving the **CK1ε** inhibition activity. It is demonstrated that the compounds of the present invention have further application prospects.

### Embodiment 35: Determination of in-vitro anti-tumor activity

The *in-vitro* anti-tumor activities of the synthesized compounds were determined using a diffuse large B-cell lymphoma (OCI-LY10) cell strain:

Drug preparation method: The drug was dissolved in DMSO to make a stock solution of 10mM and diluted in a certain ratio to obtain 7 different concentrations (test concentration: 100×).

*In vitro* culture of tumor cells: The selected OCI-LY10 cells were incubated with a medium of IMDM + fetal bovine serum in a 5% CO₂ cell culture incubator at 37°C, and were passaged when the cell density grew to 70-90% (adherent cells were passaged after digestion with Duck's EDTA) for later experiments as needed. Four thousand cells/200µl/well were seeded in a 96-well plate and incubated overnight in a 5% CO₂ cell incubator at 37°C. Two µl of the compounds were dispensed in each well at final concentrations of 10µM, 2.5µM, 0.625µM, 0.15625µM, 0.039063µM, 0.0097656µM and 0.0024414µM, and incubation was performed together for 72h in a 5% CO₂ cell incubator at 37°C with DMSO (2%) as control. After 72h, 20µl of CCK-8 solution was added and the plate was placed in a 5% CO₂ cell culture incubator at 37°C for 4h. Wells with the corresponding amounts of cell culture medium and CCK-8 solution but without cells were used as blank controls. The absorbance (OD values) was measured at 450nm using an enzyme marker, and the obtained data were used to calculate the IC₅₀. See Table 2 for the test results.

The cell inhibition rate was calculated by the formula: % cell inhibition = [(OD of control group - OD of blank group) - (OD of dosing group - OD of blank group) ]/ (OD of control group- OD of blank group) × 100%, and the half-maximal inhibitory concentration (IC₅₀) was calculated using the CalcuSyn software.

**Table 2 Inhibitory activities of partial compounds against OCI-LY10 kinase**

| **Compound** | **Structure** | **IC₅₀, µM** | **Compound** | **Structure** | **IC₅₀, µM** |
|---|---|---|---|---|---|
| **II-A-1** | 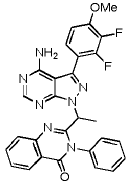 | 0.1420 | **II-C-5** | 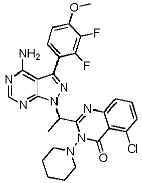 | 0.3071 |
| **II-A-2** | 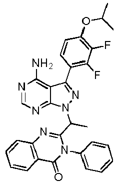 | 0.2342 | **C** | 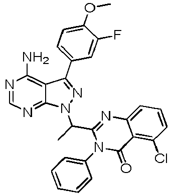 | 2.6970 |
| **II-A-7** | 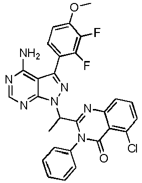 | 0.0220 | **D** | 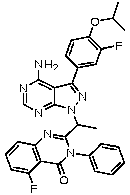 | 0.7456 |
| **II-B-2** | 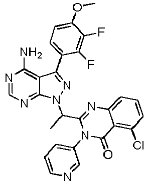 | 0.1930 | **II-A-7a** | 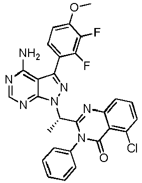 | 0.0102 |
| **II-A-1a** | 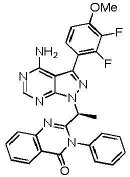 | 0.1125 | | | |

Results show that at the cellular level, the tested compounds exhibited significant tumor cell proliferation inhibitory activities (IC₅₀<0.31µM) against the OCI-LY10 cell stain, all significantly better than that of compound **D,** wherein, the IC₅₀ value of compound **II-A-7** reached 22nM, showing a 123-fold increase compared to compound **C.** This further indicates that the 2-position fluorine atom on the substituent benzene ring in the 4-aminopyrazolo[3,4-d]pyrimidin-1-yl portion of the compounds of the present invention is very important in improving the **CK1ε** inhibition activity. And it is further demonstrated that the compounds of the present invention have broad prospects in anti-tumor applications.

### Embodiment 36: Determination of in-vitro anti-tumor cells activity

*In-vitro* anti-tumor activities of the synthesized compounds were determined using Human Non-Hodgkin's Ki-positive Large Cell Lymphoma (Karpas299) cell strain:

Drug preparation method: The drugs were dissolved in DMSO to make stock solutions of 10mM and diluted in a certain ratio to obtain 7 different concentrations (test concentration: 100×).

*In vitro* culture of tumor cells: The selected Karpas299 cells were incubated in a 5% CO₂ cell culture incubator at 37°C using RPMI-1640 + fetal bovine serum medium. Four thousand cells/100µl/well were seeded in a 96-well plate and incubated overnight in a 5% CO₂ cell incubator at 37°C. Five µl of the compounds were dispensed in each well at final concentrations of 10µM, 2.5µM, 0.625µM, 0.15625µM, 0.039063µM, 0.0097656µM and 0.0024414µM, and incubation was performed together for 72h in a 5% CO₂ cell incubator at 37°C with DMSO (0.1%) as controls. After 72h, the CellTiter-Glo buffer and reaction substrate were taken out of the fridge and equilibrated to room temperature. The buffer was then poured into a brown bottle containing the substrate. The bottle was turned up and down so that the substrate powder was fully dissolved. The cells were observed under the microscope and the cell culture plate was allowed to equilibrate at room temperature for 30min. The prepared CellTiter-Glo was dispensed in a 96-well plate at 100µl per well. The plate was shaken on a plate shaker for 10min to mix well, and then allowed to stand at room temperature for 10min. A white sealing film was attached to the bottom of the well plate, and the chemiluminescence signal of each well was detected with an Enspire microplate reader. The obtained data were used to calculate the IC₅₀ values. The test results are shown in Table 3.

The cell inhibition rate was calculated by the formula: % cell inhibition = [(OD of control group - OD of blank group) - (OD of dosing group - OD of blank group) ]/ (OD of control group- OD of blank group) × 100%, and the half-maximal inhibitory concentration (IC₅₀) was calculated using CalcuSyn software.

**Table 3 Inhibitory activities of partial compounds against Karpas299**

| **Compound** | **Structure** | **IC₅₀, µM** | **Compound** | **Structure** | **IC₅₀, µM** |
|---|---|---|---|---|---|
| **II-A-1a** | 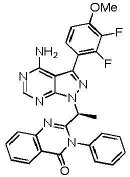 | 0.9878 | **II-A-7a** | 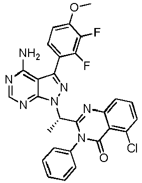 | 0.8546 |

Results show that at the cellular level, the tested compounds exhibited significant tumor cell proliferation inhibitory activities (IC₅₀<1µM) against the Karpas299 cell stain, further demonstrating that the compounds of the present invention have broad prospects in anti-tumor applications.

### Embodiment 37: In vivo pharmacodynamic study of ectopic inoculation of OCI-LY10 in SCID mouse metastatic model

### (1) Methods

OCI-LY10 cells were cultured in an IMDM medium containing 20% FBS and maintained in a 5% CO₂ incubator at 37°C with saturated humidity. OCI-LY10 cells of logarithmic phase were collected and resuspended in an IMDM basic medium containing 50% Matrigel, and the cell concentration was adjusted to 5×10⁷/ml. Subcutaneous inoculation of 0.1ml of the cell suspension was performed aseptically in the right dorsum of mice at a concentration of 5×10⁶/0.1ml/mouse. Once the tumor volume reached about 150mm³, the animals were randomly grouped according to tumor volume so that the difference in tumor volume between the groups was less than 10% of the mean value. The date of grouping was recorded as Day 0, and drug administration was started based on the body weight of the animals. During drug administration, body weight of certain animals decreased by more than 15% compared to Day 0 (BWL≥15%), drug administration was discontinued for these animals until their body weight recovered (BWL<15%). The body weight and tumor volume of the animals were measured twice a week during the experiment, and clinical signs were observed and recorded daily.

### (2) Evaluation indicators

The efficacy in terms of tumor volume was evaluated by the relative tumor proliferation rate T/C ratio, and T/C (%) was calculated as T_{RTV}/C_{RTV}×100%, where T_{RTV} is the relative tumor volume (RTV) of the treatment group and C_{RTV} is the RTV of the negative control group. The RTV was calculated as Vt/Vo, where Vo is the tumor volume at the time of grouping and Vt is the tumor volume at each measurement. The drug is considered effective with T/C≤40% according to NMPA guidelines.

The efficacy in terms of tumor weight was evaluated by TGI (tumor weight inhibition) values, TGI %=( TW_{C}-TW_{T})/TW_{C} × 100%, where TWc is tumor weight of control group, and TW_{T} is tumor weight of treatment group. The drug is considered effective with TGI≥58% according to NIH guidelines .

### (3) Results

The mice performed well in terms of body weight during drug administration, and no drug-related animal death or other significant drug-related toxic or side effects were observed during the experiment. At the end of the last administration, the tumor volume was measured, and the tumors were taken and weighed. See Tables 4 and 5 for the results.

**Table 4 Effect of test materials on tumor volume and tumor weight in the human diffuse large B-cell lymphoma OCI-LY10 xenograft tumor model (N=5)**

| **Groups** | **Administration scheme** | **RTV Day 39** | **T/C (%) Day 39** | **Tumor weight (g)** | **TGI (%)** |
|---|---|---|---|---|---|
| **Negative control group** | 0.5% MC PO QD×39 | 6.92±0.85 | / | 0.798±0.107 | / |
| **II-A-1** | 100mg/kg PO QD Day0-38 | 2.45±0.50 | 35.40 | 0.288±0.064 | 63.91 |
| **II-A-7** | 100mg/kg PO QD Day0-38 | 2.23±0.15 | 32.35 | 0.269±0.028 | 66.29 |
| **17a + II-A-1** | **17a:** 6mg/kg PO QD Day0-38 + **II-A-1**: 100mg/kg PO QD Day0-38 | 1.21±0.08 | 17.49 | 0.128±0.006 | 83.96 |
| **17a + II-A-7** | **17a:** 6mg/kg PO QD Day0-38 + **II-A-7**: 100mg/kg PO QD Day0-38 | 1.15±0.06 | 16.59 | 0.109±0.009 | 86.34 |

**Table 5 Effect of test materials on tumor volume and tumor weight in the human diffuse large B-cell lymphoma OCI-LY10 xenograft tumor model (N=5)**

| **Groups** | **Administration scheme** | **RTV Day 39** | **T/C (%) Day 39** | **Tumor weight (g)** | **TGI (%)** |
|---|---|---|---|---|---|
| **Negative control group** | 0.5% MC PO QD×39 | 7.31±0.62 | / | 1.198±0.106 | / |
| **II-A-1a** | 50mg/kg PO QD Day0-38 | 1.69±0.27 | 23.12 | 0.270±0.043 | 77.46 |
| **II-A-7a** | 50mg/kg PO QD Day0-38 | 2.11±0.60 | 28.86 | 0.327±0.048 | 72.70 |
| **17a + II-A-1a** | **17a:** 6mg/kg PO QD Day0-38 + **II-A-1a:** 50mg/kg PO QD Day0-38 | 0.76±0.15 | 10.40 | 0.143±0.020 | 88.06 |
| **17a + II-A-7a** | **17a:** 6mg/kg PO QD Day0-38 + **II-A-7a:** 50mg/kg PO QD Day0-38 | 0.55±0.06 | 7.52 | 0.110±0.008 | 90.82 |

According to the results in Tables 4 and 5, the tested compounds showed excellent tumor suppressive effects with T/C less than 40% and TGI greater than 58% in both single and combination groups. In particular, the tumor suppressive effect of the treatment group was remarkable where BTK inhibitor **17a** (Patent Application No. 201710998664.5) was administered in combination: 1) **II-A-1+17a:** T/C of 17.49% and TGI of 83.96%; 2) **II-A-7+17a:** T/C of 16.59% and TGI of 86.34%; 3) **II-A-1a +17a:** T/C of 10.40% and TGI of 88.06%; 4) **II-A-7a+17a:** T/C of 7.52% and TGI of 90.82%. Therefore, the compounds of the present invention have broad prospects in anti-tumor applications.

### Embodiment 38: Bioavailability study in rats

### Methods:

SD rats were used as the experimental animals, to which drugs of 10mg/kg were administered by gavage and 1mg/kg by tail intravenous injection. Blood samples were taken at the following time points for administration by gavage: 0.25, 0.5, 1, 2, 4, 6, 8, and 24h before and after drug administration; time points for blood sampling for intravenous administration were: 0.0833, 0.25, 0.5, 1, 2, 4, 6, 8, and 24h before and after drug administration. Whole blood (0.3ml) was collected, 0.1ml of plasma was separated by centrifugation at 2000g for 10min, and the samples were analyzed by LC-MS/MS.

Absolute bioavailability was calculated as: F =(AUC_{PO}/Dose_{PO}) / (AUC_{IV}/Dose_{IV}) × 100%

**Table 6 Summary of primary pharmacokinetic parameters after oral administration to rats**

| Parameter (Mean, N=3) | **II-A-7a** | | **II-D-6** |
|---|---|---|---|
| Route of administration | IV | PO | PO |
| Administration dosage (mg/kg) | 1 | 10 | 10 |
| Tₘₐₓ (h) | / | 6.00 | 1.67 |
| Cₘₐₓ (ng/ml) | 1518.3 | 456.3 | 2126.6 |
| AUCₗₐₛₜ (h^{∗}ng/ml) | 7200.4 | 5778.9 | 21474.6 |
| F (%) | 8.02 | | 29.82 |

The pharmacokinetic properties of the compound in the free state **(II-A-7a)** and the pharmaceutically acceptable salt thereof **(II-D-6)** were investigated in rats, and the results in Table 6 show that the compound of the present invention can be absorbed orally; and the pharmaceutically acceptable salt can improve the oral absorption of the drug by increasing the in *vivo* exposure of the compound in the free state, with a significant increase in bioavailability (3.7-fold). Therefore, the compounds of the present invention can be administered by oral absorption for treatment of diseases.

## Claims

1. A compound, wherein the compound having a structure of general formula I: 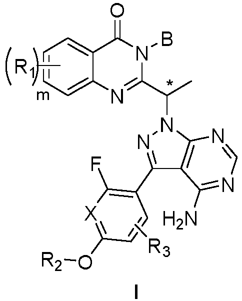 or a stereoisomer thereof, or a stereoisomer mixture thereof, or a pharmaceutically acceptable salt or solvate thereof;
wherein:
R₁ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, substituted or unsubstituted C₂-C₁₀ alkenyl, substituted or unsubstituted C₂-C₁₀ alkinyl, halogen, cyano, amino, nitro or hydroxy;
m is an integer between 1 and 4; and when m is greater than 1, a plurality of R₁ may be the same or different, independently of each other;
R₂ is H, substituted or unsubstituted C₁-C₆ alkyl, substituted or unsubstituted C₃-C₁₀ cycloalkyl, substituted or unsubstituted C₅-C₁₂ aryl;
R₃ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, halogen, cyano, amino, nitro or hydroxy;
X is CR₄ or N;
R₄ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, halogen, cyano, amino or hydroxy, and R₃ and R₄ are not H simultaneously; and
B is selected from substituted or unsubstituted C₁-C₆ alkyl, substituted or unsubstituted C₃-C₁₀ cycloalkyl, substituted or unsubstituted 3- to 10-membered heterocycloalkyl, substituted or unsubstituted C₅-C₁₂ aryl, and substituted or unsubstituted 5- to 12-membered heteroaryl.

2. The compound according to claim 1, wherein the compound having a structure of general formula II-A, II-B or II-C: 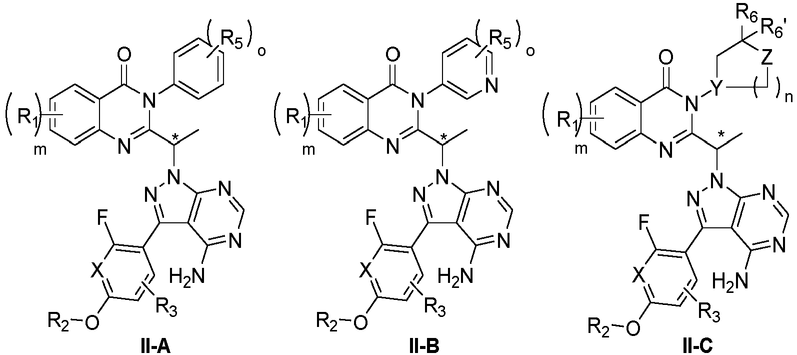 or a stereoisomer thereof, or a stereoisomer mixture thereof, or a pharmaceutically acceptable salt or solvate thereof;
wherein:
R₅ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, substituted or unsubstituted C₁-C₆ hydroxyalkyl, substituted or unsubstituted C₁-C₆ halohydroxyalkyl, halogen, cyano, amino, nitro or hydroxy;
o is an integer between 1 and 5;
R₆ and R₆' are independently selected from H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, halogen, cyano, amino or hydroxy, or, R₆ and R₆' are joined to form a 3- to 6-membered cycloalkyl or heterocycloalkyl;
n is absent, or 0, 1 or 2; wherein:
when n is absent, i.e., when Y and Z are not joined to form a ring, Y is CR₇R₇' or NR₈; and Z is CHR₇R₇', OH or NHR₈; and
when n is 0, 1 or 2, Y is CH or N; and Z is CR₇R₇', O or NR₈;
R₇ and R₇' are independently selected from H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, substituted or unsubstituted C₃-C₁₀ cycloalkyl, substituted or unsubstituted 3- to 10-membered heterocycloalkyl, halogen, cyano, amino or hydroxy; or, R₇ and R₇' are joined to form a 3- to 6-membered cycloalkyl or heterocycloalkyl;
R₈ is H, substituted or unsubstituted C₁-C₆ alkyl, substituted or unsubstituted C₃-C₁₀ cycloalkyl, substituted or unsubstituted 3- to 10-membered heterocycloalkyl, -C(O)-R₉ or -S(O)₂-R₁₀;
R₉ is substituted or unsubstituted C₁-C₆ alkyl, or substituted or unsubstituted C₃-C₁₀ cycloalkyl; and
R₁₀ is substituted or unsubstituted C₁-C₆ alkyl, or substituted or unsubstituted C₃-C₁₀ cycloalkyl;

3. The compound according to claim 2, **characterized by** having a structure of general formula III-A, III-B or III-C: 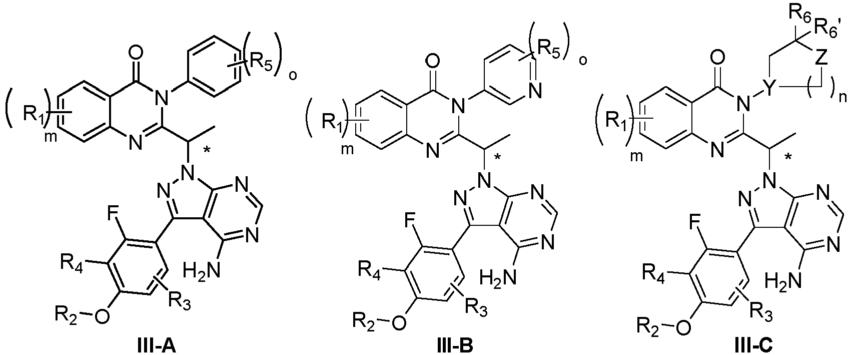 or a stereoisomer thereof, or a stereoisomer mixture thereof, or a pharmaceutically acceptable salt or solvate thereof.

4. The compound according to claim 3, wherein the compound having a structure of general formula IV-A, IV-B or IV-C: 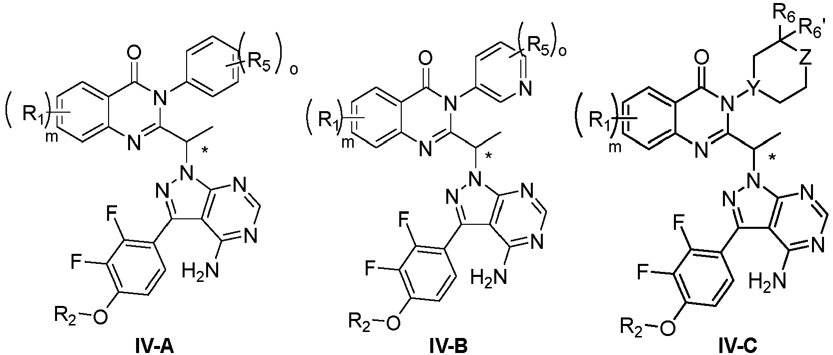 or a stereoisomer thereof, or a stereoisomer mixture thereof, or a pharmaceutically acceptable salt or solvate thereof;
wherein:
R₁ is selected from H, substituted or unsubstituted C₁-C₆ alkyl, and halogen;
m is selected from integers of 1 to 4;
R₂ is selected from methyl, ethyl, isopropyl and cyclopropyl;
R₅ is selected from H, substituted or unsubstituted C₁-C₆ alkyl, and halogen; o is selected from integers of 1 to 5;
R₆ and R₆' are selected from H, substituted or unsubstituted C₁-C₆ alkyl, and halogen;
Y is selected from N and CH;
Z is selected from CH₂, O and NR₈;
R₈ is selected from H, substituted or unsubstituted C₁-C₆ alkyl, and -C(O)-R₉; and
R₉ is selected from substituted or unsubstituted C₁-C₆ alkyl.

5. The compound according to claim 4, wherein the compound having a structure of general formula V-A, V-B or V-C: 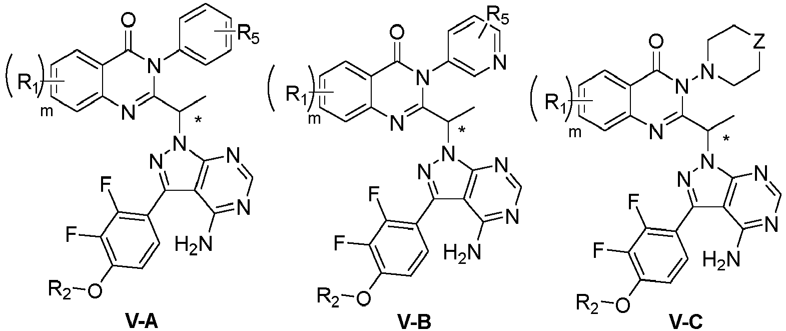 or a stereoisomer thereof, or a stereoisomer mixture thereof, or a pharmaceutically acceptable salt or solvate thereof; wherein: the definitions of R₁, m, R₅ and Z are the same as defined in general formulas IV-A, IV-B and IV-C; and R₂ is methyl or isopropyl.

6. The compound according to claim 1, wherein C denoted by ^{∗} in Formula I has a R configuration or an S configuration.

7. The compound according to claim 1, wherein the compound having one of the following structures:
| No. | Structure | No. | Structure | No. | Structure |
|---|---|---|---|---|---|
| II-A-1 | 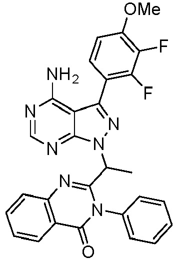 | II-A-1a | 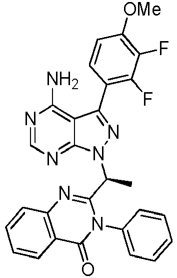 | II-A-1 b | 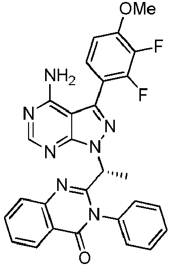 |
| II-A-2 | 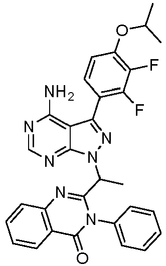 | II-A-2a | 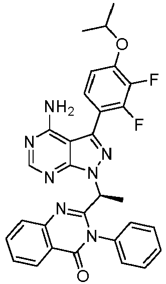 | II-A-2 b | 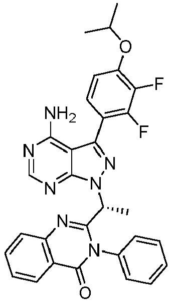 |
| II-A-3 | 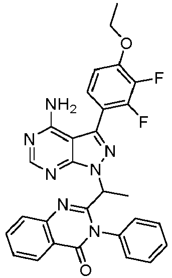 | II-A-4 | 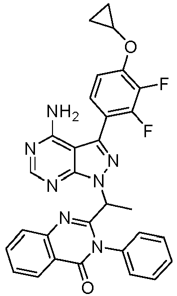 | II-A-5 | 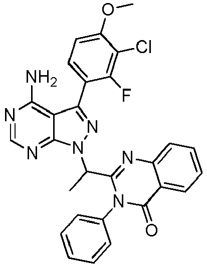 |
| II-A-6 | 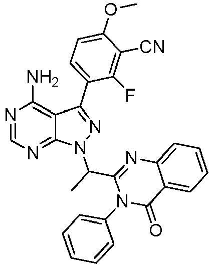 | II-A-7 | 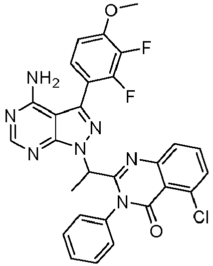 | II-A-7a | 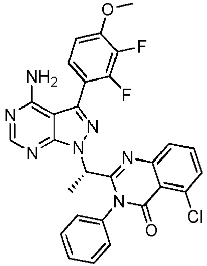 |
| II-A-7 b | 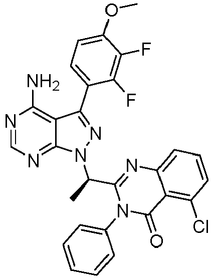 | II-A-8 | 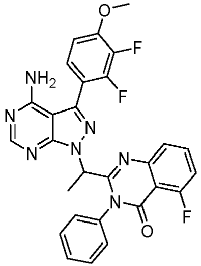 | II-A-8a | 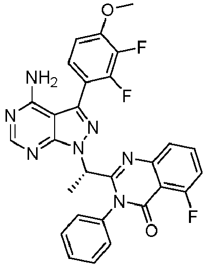 |
| II-A-8 b | 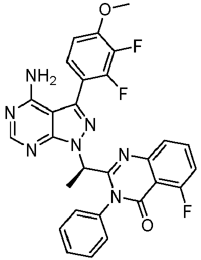 | II-A-9 | 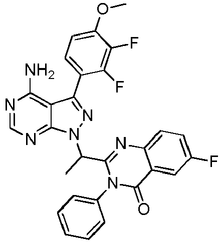 | II-A-1 0 | 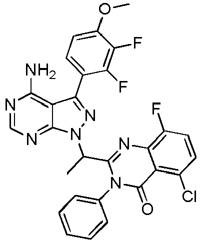 |
| II-A-11 | 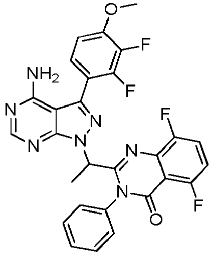 | II-A-1 2 | 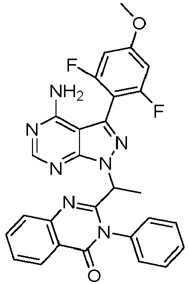 | II-A-1 3 | 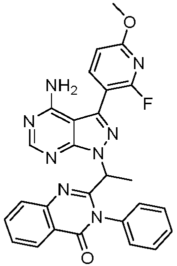 |
| II-A-1 4 | 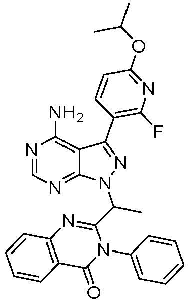 | II-A-1 5 | 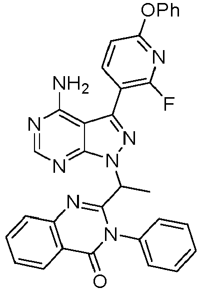 | II-A-1 6 | 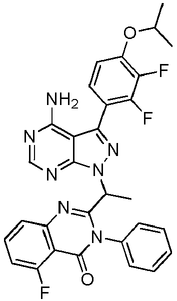 |
| II-B-1 | 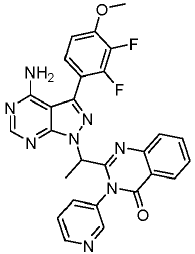 | II-B-2 | 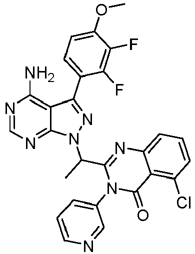 | II-B-2a | 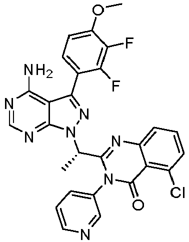 |
| II-B-2b | 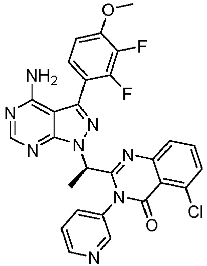 | II-B-3 | 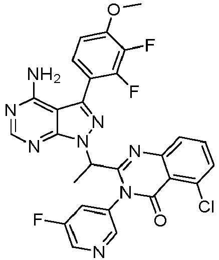 | II-C-1 | 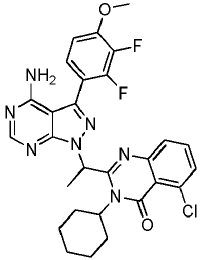 |
| II-C-2 | 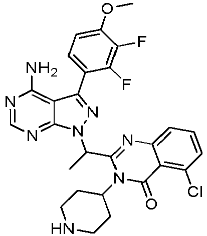 | II-C-3 | 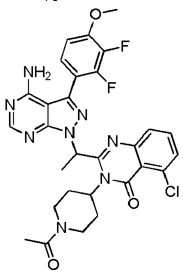 | II-C-4 | 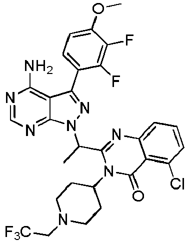 |
| II-C-5 | 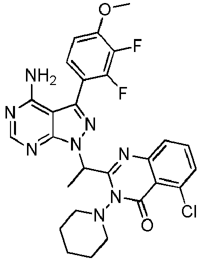 | II-C-6 | 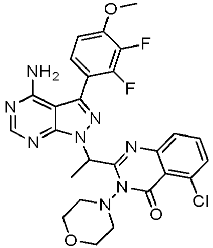 | II-C-7 | 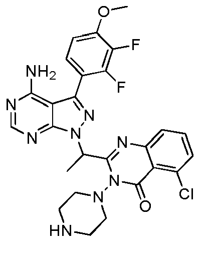 |
| II-C-8 | 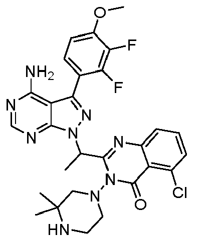 | II-C-9 | 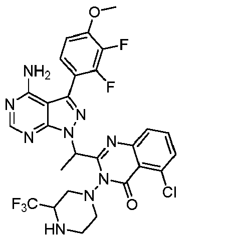 | II-C-10 | 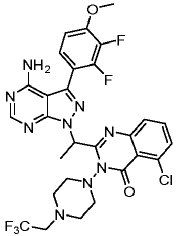 |
| II-C-11 | 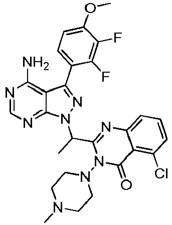 | II-C-12 | 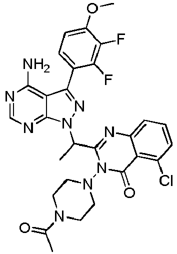 | II-C-13 | 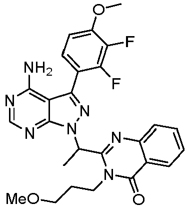 |
| II-C-14 | 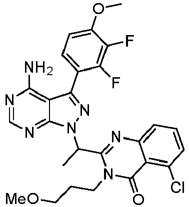 | II-C-15 | 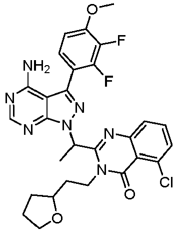 | | |
or a stereoisomer thereof, or a stereoisomer mixture thereof, or a pharmaceutically acceptable salt or solvate thereof.

8. The compound according to claim 1, wherein the compound having one of the following structures:
| | | | | | |
|---|---|---|---|---|---|
| II-D-1 | 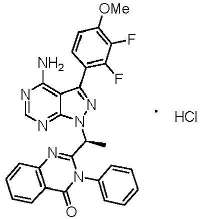 | II-D-2 | 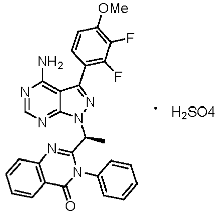 | II-D -3 | 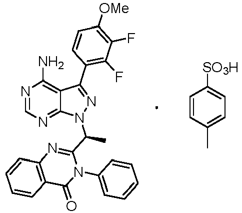 |
| II-D-4 | 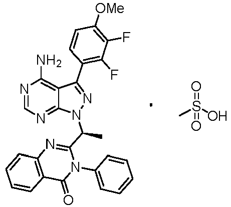 | II-D-5 | 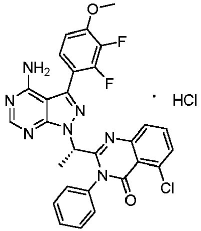 | II-D -6 | 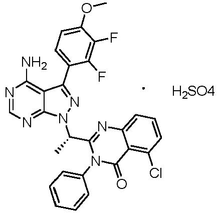 |
| II-D-7 | 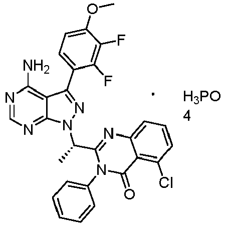 | II-D-8 | 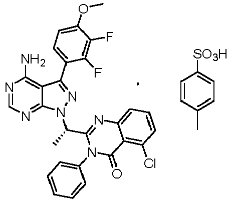 | II-D -9 | 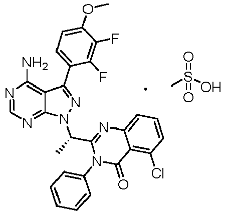 |
| II-D-10 | 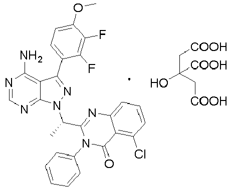 | II-D-11 | 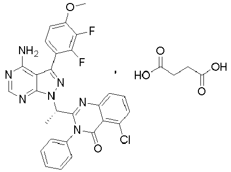 | II-D -12 | 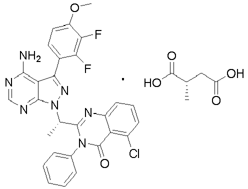 |
| II-D-13 | 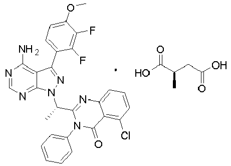 | II-D-11 4 | 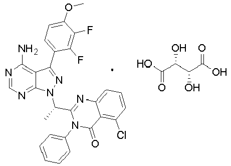 | II-D -15 | 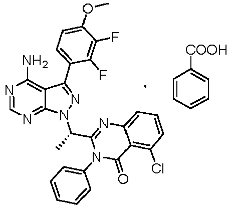 |
| II-D-16 | 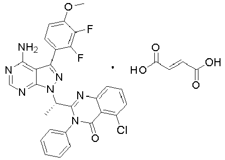 | II-D-11 7 | 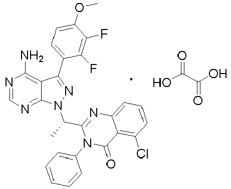 | | |
or a stereoisomer thereof, or a stereoisomer mixture or solvate thereof.

9. The compound according to any one of claims 1-8, wherein C denoted by ^{∗} has an S configuration.

10. A pharmaceutical composition, comprising one or more of the compounds according to any one of claims 1-9.

11. An application of the compound according to any one of claims 1-9 in preparation of a drug which can be used alone or in combination with other drugs for treating a disease, a disorder or a condition that benefits from an inhibition of activity of a casein kinase 1ε.

12. The application according to claim 11, the other drugs being one or more selected from the following drugs: an antineoplastic drug, an anti-inflammatory agent, an immunosuppressive agent, a steroid and a non-steroidal anti-inflammatory agent.

13. The application according to claim 11, wherein the disease is selected from B-cell lymphoma, multiple myeloma, leukemia, lung cancer, breast cancer, prostate cancer, bladder cancer, ovarian cancer, pancreatic cancer, sarcoma, colon cancer, kidney cancer, liver cancer, melanoma, brain tumor, rheumatoid arthritis, systemic lupus erythematosus, multiple sclerosis, inflammatory bowel disease, Crohn's disease, colitis, autoimmune hemolytic anemia, ankylosing spondylitis, pemphigus, urticaria, asthma, optic neuritis, psoriasis, chronic obstructive airway disease, dermatitis and baldness.

14. The application according to claim 13, wherein the B-cell lymphoma comprises diffuse large B-cell lymphoma, follicular lymphoma, chronic lymphocytic lymphoma, chronic lymphocytic leukemia, B-cell prolymphocytic leukemia, lymphoplasmacytic lymphoma, splenic marginal zone lymphoma, plasma cell myeloma, plasmacytoma, extranodal marginal zone B-cell lymphoma, nodal marginal zone B-cell lymphoma, mantle cell lymphoma, mediastinal large B-cell lymphoma, intravascular large B-cell lymphoma, primary effusion lymphoma, Burkitt's lymphoma/leukemia or pulmonary lymphomatoid granulomatosis.

15. The application according to claim 11, wherein the disease is selected from T-cell lymphoma.

16. The application according to claim 15, wherein the T-cell lymphoma comprises peripheral T-cell lymphoma, anaplastic large cell lymphoma, extranodal NK/T-cell lymphoma, cutaneous peripheral T-cell lymphoma, T-cell prolymphocytic leukemia, angioimmunoblastic T-cell lymphoma, adult T-cell leukemia/lymphoma, hepatosplenic T-cell lymphoma, intestinal T-cell lymphoma, breast implant-associated anaplastic large cell lymphoma, T-cell large granular lymphocytic leukemia.

17. The application according to claim 12, the antineoplastic drug comprising one or more of the following drugs: a mitotic inhibitor, a tubulin proteolysis inhibitor, an alkylation reagent, an antimetabolite, an insertable antibiotic, an enzyme, a topoisomerase inhibitor, a biological response modifier, an immunomodulator, a BTK inhibitor, a Bcl-2 inhibitor, an anti-CD20 monoclonal antibody, an mTOR inhibitor, an mTORC1 inhibitor, an AKT inhibitor, a PI3K inhibitor, a proteasome inhibitor, an EGFR inhibitor, a VEGFR inhibitor, a CDK inhibitor and a PD-1/PD-L1 inhibitor.

18. The application according to claim 12, the antineoplastic drug being a BTK inhibitor.

19. An intermediate, wherein the intermediate having a structure as shown in a following formula; 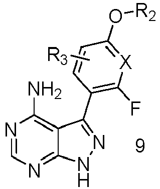 wherein:
R₂ is H, substituted or unsubstituted C₁-C₆ alkyl, substituted or unsubstituted C₃-C₁₀ cycloalkyl, or substituted or unsubstituted C₅-C₁₂ aryl;
R₃ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, halogen, cyano, amino, nitro or hydroxy; and
X is CR₄ or N;
R₄ is H, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ haloalkoxy, halogen, cyano, amino or hydroxy.
